# EUROPEAN PATENT APPLICATION

(11) **EP 4 098 653 A1**
(43) Date of publication of application: **07.12.2022**
(21) Application number: 20916984.6
(22) Date of filing: 26.03.2020
(51) Int. Cl.: C07D 405/12, A61K 31/437

(54) **PHARMACEUTICAL USE OF ALDEHYDE-BASED COMPOUND**

(30) Priority: 29.01.2020 CN 202010077463
(71) Applicant: Shanghai Institute of Materia Medica, Chinese Academy of Sciences, Pudong, Shanghai 201203 (CN); Frontier Biotechnologies Inc., Jiangning, Nanjing 211122 (CN)
(72) Inventor: LIU, Hong, Shanghai 201203 (CN); LI, Jian, Shanghai 201203 (CN); DAI, Wenhao, Shanghai 201203 (CN); PENG, Jingjing, Shanghai 201203 (CN); XIE, Xiong, Shanghai 201203 (CN); HU, Shulei, Shanghai 201203 (CN); LI, Chunpu, Shanghai 201203 (CN); XU, Yechun, Shanghai 201203 (CN); YANG, Haitao, Shanghai 201203 (CN); ZHANG, Leike, Shanghai 201203 (CN); SU, Haixia, Shanghai 201203 (CN); JIANG, Hualiang, Shanghai 201203 (CN); JIN, Zhenming, Shanghai 201203 (CN); XIAO, Gengfu, Shanghai 201203 (CN); CHEN, Kaixian, Shanghai 201203 (CN)
(74) Representative: Finnegan Europe LLP
(86) International application number: PCT/CN2020/081481
(87) International publication number: WO 2021/151265

(57) **Abstract**

Use of an aldehyde-based compound as represented by general formula I, a pharmaceutical composition, a pharmaceutical salt, an enantiomer, a diastereomer and a racemic compound thereof as a novel coronavims 2019 (2019-nCov) 3CL protease inhibitor in preparation of a medicament for treating and/or preventing and relieving respiratory tract infections, pneumonia and other related diseases caused by the novel coronavims infection 2019.

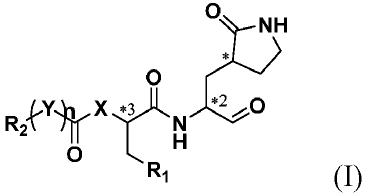

## Description

### FIELD OF THE INVENTION

The invention relates to the field of medicine, in particular to a medical use of aldehyde compounds.

### BACKGROUND OF THE INVENTION

Most of acute infectious diseases are viral infectious diseases. The viral infectious diseases are of high incidence rate and high mortality rate. Due to limited detection and diagnosis methods, new epidemic outbreaks caused by new viruses are often sudden, random and unpredictable. Once it outbroke, if there is no effective prevention and control method, it can easily cause large-scale epidemic and seriously threaten public health and safety. At the end of 2019, there was an outbreak of new coronavirus (2019-nCoV, also known as SARS-CoV-2) infection, which caused severe pneumonia (Covid). By 10:00 on January 28, 2020, there were 4,529 domestic infections and 106 deaths. The transmission route of the 2019-nCoV virus is not fully understood. It is known to be transmitted through droplets and contact, and there are human-to-human transmission as well as medical staff infection exists, also possesses a certain risk of community transmission, and the virus may mutate. At present, there is no specific prevention and treatment for the diseases caused by the new coronavirus.

The 2019-nCoV coronavirus belongs to the genus Coronavirus of the Coronavirus family, is a single-stranded positive-sense RNA virus with an envelope. Similar to other known coronaviruses, the 2019-nCoV coronavirus also completes the proliferation of progeny viruses through several processes such as adsorption, penetration, uncoating, biosynthesis, and assembly and release of progeny viruses. The infection of host cells by the 2019-nCoV coronavirus starts with the spike glycoprotein on the surface of the virus envelope binds to the receptor on the surface of the host cell, then membrane fusion occurs. The virus enters the host cell and releases the genetic material of the virus (singlesense - stranded RNA) under the effect of organelles such as cell lysosomes, and the RNA is translated to produce polyproteins under the effect of protein synthesis elements such as mitochondria, ribosomes of host cell, and necessary raw materials. After that, the two essential cysteine protease of the 2019-nCoV coronavirus: papainlike protease (PL^{pro}) and 3C-like protease (3C-like protease, 3CL^{pro}) cleave and process polyprotein precursors at specific sites to produce several non-structural protein that are important to the virus life cycle. Under the effect of these non-structural proteins, the viral RNA replicates the nucleic acid material of progeny virus, and a large number of required structural proteins are translated to complete the assembly and release of the progeny virus. Any step or key enzyme in the life cycle of the 2019-nCoV coronavirus infected cell can be used as the research target of antiviral drugs, such as the cysteine proteases PL^{pro} and 3CL^{pro} that hydrolyze and cleave the polyprotein precursor, or RNA polymerase that is responsible for completing the genetic material replication of progeny viruses, etc.

3CL protease (3 chymotrypsin-like protease, 3CL^{pro}), also known as the main protease (M^{pro}), is key protease in the process of hydrolysis to produce multiple non-structural proteins after coronavirus RNA translation of the polyproteins pp1a and pp1ab, which is critical to virus replication and infection. Inhibiting the catalytic function of 3CL protease can effectively inhibit the cleavage of viral polyprotein precursors, block virus replication, and inhibit the generation of progeny viruses. 3CL^{pro} belongs to acysteine protease, which is key protease that catalyzes the proteolysis of single sense-strand RNA virus precursors, and plays an important role in the replication activity of coronaviruses such as 2019-nCoV. Therefore, 3CL^{pro} is currently recognized as an ideal target for the development of anti-coronavirus drugs.

At present, there are no specific vaccines or antiviral drugs for severe pneumonia caused by the 2019-nCoV coronavirus. These infectious diseases have seriously affected people's lives and health, and it is urgent for development of effective small-molecule antiviral drugs. It is of great social meaning to develop antiviral drugs with novel structure, low toxicity and high efficiency and independent intellectual property rights for the 2019-nCoV coronavirus 3CL^{pro} to meet the clinical needs of patients infected with 2019-nCoV coronavirus worldwide.

In conclusion, there is an urgent need in the art to develop inhibitors for the 2019-nCoV coronavirus 3CL protease for the treatment of pneumonia caused by novel coronavirus infection.

### SUMMARY OF THE INVENTION

The purpose of the present invention is to provide a novel use of aldehyde compound.

Specifically, the present invention provides a use of aldehyde compound according to General Formula I as 2019-nCov 3CL protease inhibitors for preparing drugs for the treatment, and/or prevention, alleviation of related diseases (such as respiratory infection, pneumonia and the like) caused by 2019-nCov infection.

In the first aspect of the present invention, a use of aldehyde compound according to General Formula I, or pharmaceutically acceptable salts, enantiomers, diastereomers or racemates thereof is provided, wherein it is used for preparing (a) 2019-nCov 3CL protease inhibitors; and (b) drugs for the treatment, and/or prevention, alleviation of related diseases caused by 2019-nCov infection: 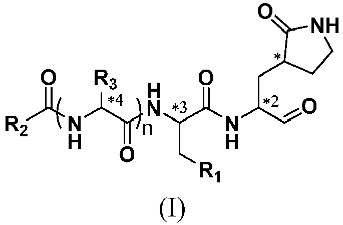 wherein,
the chiral carbon atoms C^{∗}, C^{∗2}, C^{∗3}, and C^{∗4} are each independently in S configuration, R configuration, or the combinations thereof;
n=0 or 1;
R₁ is a group unsubstituted or substituted with 1-3 substituents, and the group is selected from the group consisting of C3-C7 cycloalkyl, trifluoromethyl, C2-C6 alkynyl, 4-7 membered heterocyclyl, C5-C7 aryl, 5-7 membered heteroaryl; each heterocyclyl and heteroaryl contains 1-3 heteroatoms selected from oxygen, sulfur and nitrogen; and the substituents are each independently selected from the groups consisting of halogen, C1-C4 straight or branched alkyl, C1-C4 straight or branched alkenyl, C2-C4 straight or branched alkynyl, C1-C4 straight or branched alkoxy, C1-C4 straight or branched alkylcarbonyloxy, cyano, nitro, hydroxyl, amino, hydroxymethyl, trifluoromethyl, carboxyl, sulfydryl;, C1-C4 acyl, acylamino, sulfonyl, aminosulfonyl, C1-C4 alkyl-substituted sulfonyl, or two adjacent substituents together with the carbon atoms to which they are connected form 5-7 membered ring;
R₂ is a group unsubstituted or substituted with 1-3 substituents, and the group is selected from the group consisting of C3-C7 cycloalkyl, 5-12 membered heterocyclyl (preferably 5-7 membered heterocyclyl or 6-membered aryl fused 5-7 membered heterocyclyl), C6-C12 aryl, 5-12 membered heteroaryl, styryl, or -Cbz; wherein each of the heterocyclyl or heteroaryl contains 1-3 heteroatoms selected from oxygen, sulfur and nitrogen; the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, sulfydryl, C1-C4 acyl, amide, sulfonyl, aminosulfonyl, C1-C4 alkyl-substituted sulfonyl, or two adjacent substituents together with the carbon atoms to which they are connected form 5-7 membered ring;
R₃ is a group unsubstituted or substituted by 1-3 substituents, and the group is selected from the group consisting of C1-C6 straight or branched alkyl, C1-C6 straight or branched alkoxy, C3-C7 cycloalkyl, C6-C12 aryl, 5-12 membered heteroaryl, wherein the heteroaryl contains 1-3 heteroatoms selected from oxygen, sulfur and nitrogen; wherein, the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, sulfydry, C1-C4 acyl, amide, sulfonyl, aminosulfonyl, C1-C4 alkyl-substituted sulfonyl, or two adjacent substituents together with the carbon atoms to which they are connected form 5-7 membered ring.

In another preferred embodiment, the related diseases caused by 2019 n-Cov infection are selected from the groups consisting of respiratory infection, pneumonia and complications thereof, or the combinations thereof.

In another preferred embodiment, R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl.

In another preferred embodiment, R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocycle and 5-12 membered heteroaromatic ring are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline.

In another preferred embodiment, one or more of the chiral carbon atoms C^{∗}, C^{∗2}, C^{∗3}, and C^{∗4} are in S configuration.

In another preferred embodiment, the chiral carbon atoms C^{∗}, C^{∗2}, C^{∗3}, and C^{∗4} are in S configuration, and/or
R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocycle and 5-12 membered heteroaromatic ring are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline; and/or
R₃ is a group unsubstituted or substituted with 1-3 substituents, and the group is selected from the group consisting of C1-C6 straight or branched alkyl, C3-C7 cycloalkyl, phenyl.

In another preferred embodiment, the compounds in General formula I are selected from the group consisting of:

**Table A**

| No. | Name | Structure |
|---|---|---|
| 1 | 6-Chloro-N-{(S)-3-methyl-1-carbonyl-1-{{(S)-1-carbonyl-1-{{(S)-1-carbonyl-3-[(S)-2-carbonylpyrrolidine-3-yl]propan-2-yl} amino}-3-phenylpropan-2-yl}amino }butan-2-yl}-2*H*-chromene-3-carboxamide | 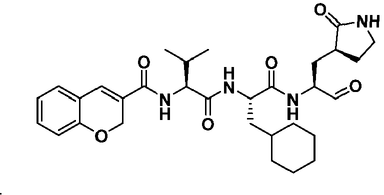 |
| 2 | 6-Chloro-N-{(S)-3-methyl-1-carbonyl-1-{{(S)-1-carbonyl-1-{{(S)-1-carbonyl-3-[(S)-2-carbonylpyrrolidin-3-yl]propan-2-yl} amino}-3-cyclohexylpropan-2-yl}amino } butan-2-yl}-2*H*-chromene-3-carboxamide | 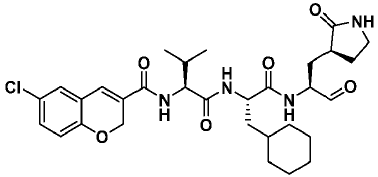 |
| 3 | 6-Chloro-N-{(S)-3-methyl-1-carbonyl-1-{{(S)-1-carbonyl-1-{{(S)-1-carbonyl-3-[(S)-2-carbonylpyrrolidin-3-yl]propan-2-yl} amino } -3 -cyclopentylpropan-2-yl}amino } butan-2-yl}-2*H*-chromene-3-carboxamide | 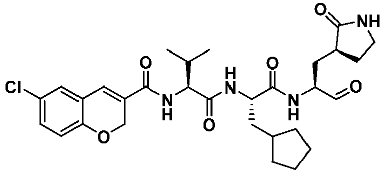 |
| 4 | 6-Chloro-N-{(S)-3-methyl-1-carbonyl-1-{{(S)-1-carbonyl-1-{{(S)-1-carbonyl-3-[(S)-2-carbonylpyrrolidin-3-yl]propan-2-yl} amino } -3 -4fluorophenylpropan-2-yl}amino}butan-2-yl}2*H*-chromene-3-carboxamide | 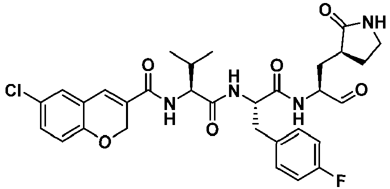 |
| 5 | N-{{(S)-3-methyl-1-carbonyl-1-{{(S)-1-carbonyl-1-{{(S)-1-carbonyl-3-[(S)-2-carbonylpyrrolidin-3-yl]propan-2-yl}amino } -3 -cyclohexylpropan-2 - yl}amino}butan-2-yl} indole-2-carboxamide | 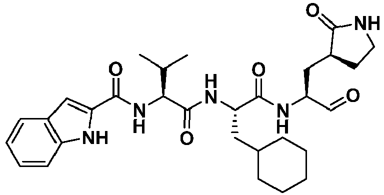 |
| 6 | 5-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl} amino} butan-2-yl} isoxazole-3 - carboxamide | 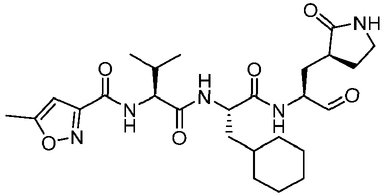 |
| 7 | N-{(S)-3-fluoro-3-methyl-1-carbonyl-1-{{(S)-1-carbonyl-1-{{(S)-1-carbonyl-3-[(S)-2-carbonylpyrrolidin-3-yl]propan-2-yl}amino}-3 -cyclohexylpropan-2-yl}amino}butan-2-yl} indole-2-carboxamide | 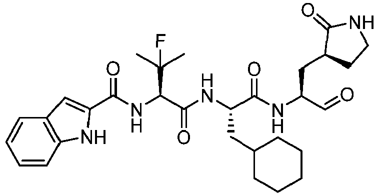 |
| 8 | 5-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl} isoxazole-3 - carboxamide | 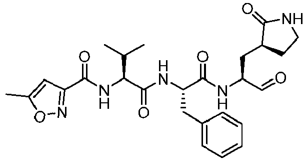 |
| 9 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}indole-2-carboxamide | 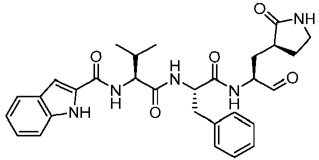 |
| 10 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}benzofuran-2-carboxamide | 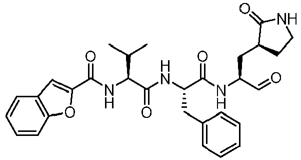 |
| 11 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}quinoline-2-carboxamide | 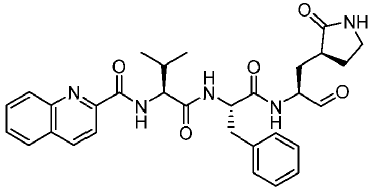 |
| 12 | N-{{(S)-1-carbonyl-1-{{(S)-1-carbonyl-3-[(S)-2-carbonylpyrrolidin-3-yl]propan-2-yl}amino}-3-(3,4-difluorophenyl)propan-2-yl}-1*H*-indole-2-carboxamide | 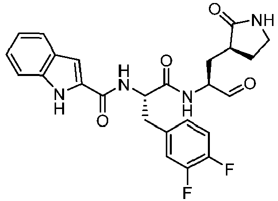 |
| 13 | 5-Methoxy-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl} indole-2-carboxamide | 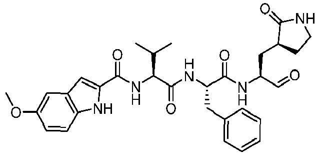 |
| 14 | 4,6-dichloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}indole-2-carboxamide | 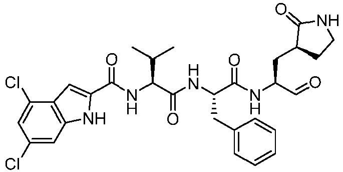 |
| 15 | N-{(S)-3-methyl-1-carbonyl-1-(S)-1-carbonyl-1-{{(S)-1-carbonyl-3-[(S)-2-carbonylpyrrolidin-3-yl]propan-2-yl}amino}-3-4fluorocyclohexylpropan-2-yl}amino}butan-2-yl}indole-2-carboxamide | 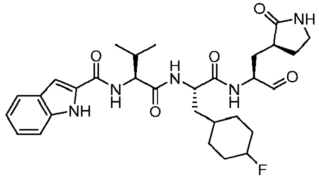 |
| 16 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl} quinoline-2-carboxamide | 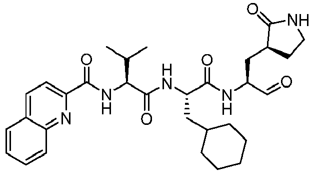 |
| 17 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}benzofuran-2-carboxamide | 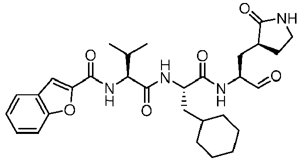 |
| 18 | 7-Bromo-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl} benzofuran-2-carboxamide | 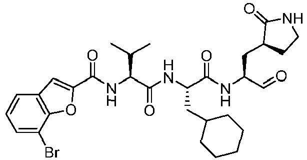 |
| 19 | 3-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}quinoline-2-carboxamide | 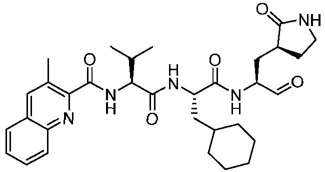 |
| 20 | 5-Chloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino } butan-2-yl} benzofuran-2-carboxamide | 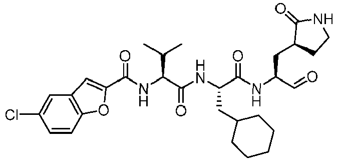 |
| 21 | N-{(S)-1-carbonyl-1-{{(S)-1-carbonyl-3-[(S)-2-carbonylpyrrolidin-3-yl]propan-2-yl}amino}-3-ethynylpropan-2-yl}-1*H-*indole-2-carboxamide | 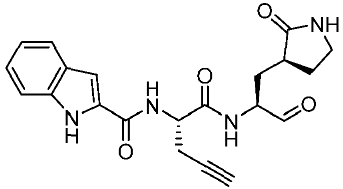 |
| 22 | N-{{(S)-1-carbonyl-1-{{(S)-1-carbonyl-3-[(S)-2-carbonylpyrrolidin-3-yl]propan-2-yl}amino}-3-cyclohexylpropan-2-yl}-1*H-*indole-2-carboxamide | 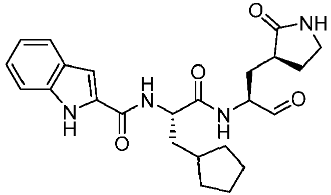 |
| 23 | 5-Fluoro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3 -phenylpropan-2-yl}amino}butan-2-yl}benzofuran-2-carboxamide | 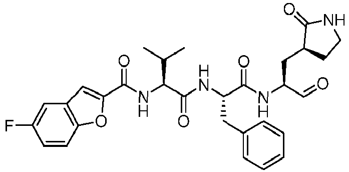 |
| 24 | N-{(S)-3-methyl-1-carbonyl-1-{{(S)-1-carbonyl-1-{{(S)-1-carbonyl-3-[(S)-2-carbonylpyrrolidin-3-yl]propan- 2-yl}amino}-3-cyclohexylpropan-2-yl}amino}butan-2-yl}benzothiophene-2-carboxamide | 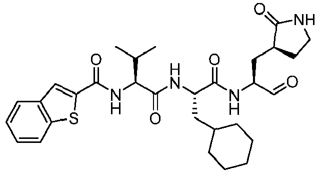 |
| 25 | 7-Bromo-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}quinoline-2-carboxamide | 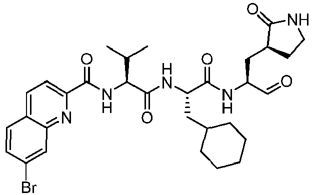 |
| 26 | 7-Methoxy-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino } butan-2-yl} benzofuran-2-carboxamide | 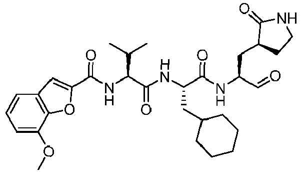 |
| 27 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}benzothiophene-2-carboxamide | 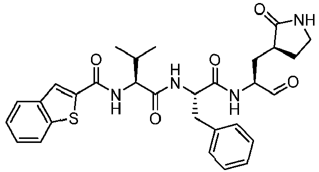 |
| 28 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}pentan-2-yl}indole-2-carboxamide | 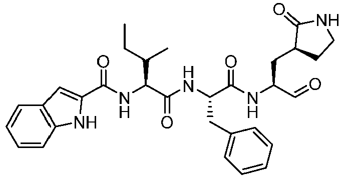 |
| 29 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}pentan-2-yl}indole-2-carboxamide | 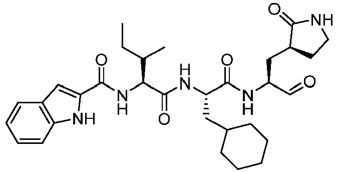 |
| 30 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino }-3-4fluorophenylpropan-2-yl}amino}butan-2-yl} indole-2-carboxamide | 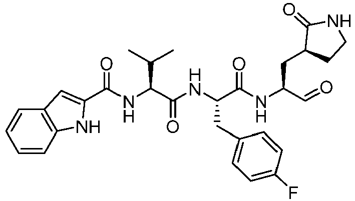 |
| 31 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclopentylpropan-2-yl}amino}butan-2-yl} indole-2-carboxamide | 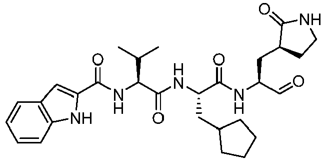 |
| 32 | 5-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino } -3 -4fluorophenylpropan-2-yl}amino}butan-2-yl} isoxazole-3 - carboxamide | 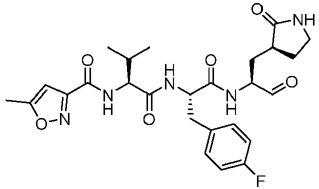 |
| 33 | N-{(S)-3,3-dimethyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl} indole-2-carboxamide | 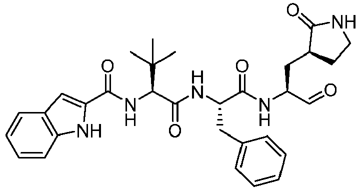 |
| 34 | N-{(S)-3,3-dimethyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl} indole-2-carboxamide | 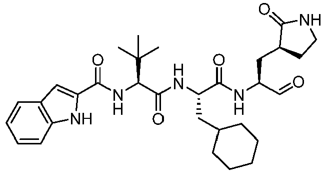 |
| 35 | N-{(S)-3-methyl-1-carbonyl-1-{{(S)-1-carbonyl-1-{{(S)-1-carbonyl-3-[(S)-2-carbonylpyrrolidin-3-yl]propan- 2-yl}amino}-3-(3,4-difluorocyclohexyl)propan-2-yl}amino}butan-2-yl} indole-2-carboxamide | 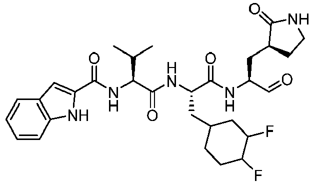 |
| 36 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclobutylpropan-2-yl}amino}butan-2-yl} indole-2-carboxamide | 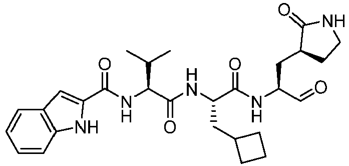 |
| 37 | N-{(S)-1-carbonyl-1-{{(S)-1-carbonyl-3-[(S)-2-carbonylpyrrolidin-3-yl]propan-2-yl}amino}-3-phenylpropan-2-yl}indole-2-carboxamide | 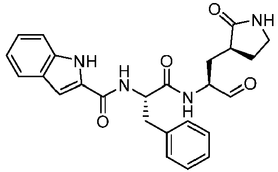 |
| 38 | 1-Methyl-N-{(S)-1-carbonyl-1-{{(S)-1-carbonyl-3-[(S)-2-carbonylpyrrolidin-3-yl]propan-2-yl} amino } -3 -phenylpropan-2-yl}-1*H*-indole-2-carboxamide | 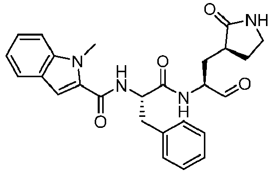 |
| 39 | 6-Trifluoromethyl-N-{(S)-1-carbonyl-1-{{(S)-1-carbonyl-3-[(S)-2-carbonylpyrrolidin-3-yl]propan-2-yl}amino}-3-phenylpropan-2-yl}-1*H-*indole-2-carboxamide | 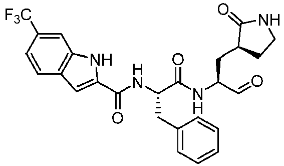 |
| 40 | 5-Methoxy-N-{(S)-1-carbonyl-1-{{(S)-1-carbonyl-3-[(S)-2-carbonylpyrrolidin-3-yl]propan-2-yl } amino } -3 -phenylpropan-2-yl}-1*H*-indole-2-carboxamide | 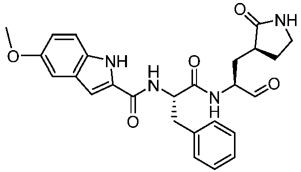 |
| 41 | 5-Chloro-N-{(S)-1-carbonyl-1-{{(S)-1-carbonyl-3-[(S)-2-carbonylpyrrolidin-3-yl]propan-2-yl} amino } -3 -phenylpropan-2-yl}-1*H*-indole-2-carboxamide | 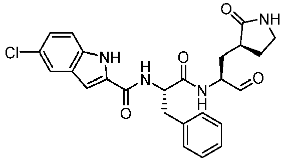 |
| 42 | 4-Fluoro-N-{(S)-1-carbonyl-1-{{(S)-1-carbonyl-3-[(S)-2-carbonylpyrrolidin-3-yl]propan-2-yl } amino } -3 -phenylpropan-2-yl}-1*H*-indole-2-carboxamide | 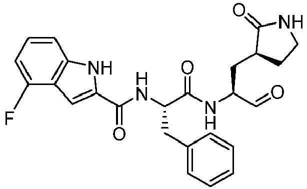 |
| 43 | 7-Fluoro-N-{(S)-1-carbonyl-1-{{(S)-1-carbonyl-3-[(S)-2-carbonylpyrrolidin-3-yl]propan-2-yl } amino } -3 -phenylpropan-2-yl}-1*H*-indole-2-carboxamide | 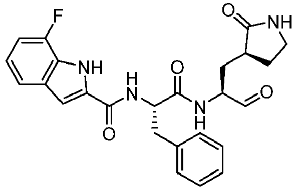 |
| 44 | 5,7-Difluoro-N-{(S)-1-carbonyl-1-{{(S)-1-carbonyl-3-[(S)-2-carbonylpyrrolidin-3-yl]propan-2-yl} amino } -3 -phenylpropan-2-yl}-1*H*-indole-2-carboxamide | 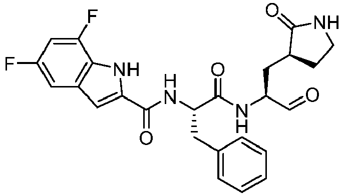 |
| 45 | 5-Fluoro-N-{(S)-1-carbonyl-1-{{(S)-1-carbonyl-3-[(S)-2-carbonylpyrrolidin-3-yl]propan-2-yl} amino } -3 -phenylpropan-2-yl} - 1*H*-indole-2-carboxamide | 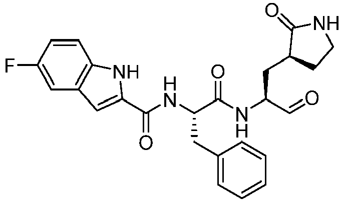 |
| 46 | 6-Fluoro-N-{(S)-1-carbonyl-1-{{(S)-1-carbonyl-3-[(S)-2-carbonylpyrrolidin-3-yl]propan-2-yl } amino } -3 -phenylpropan-2-yl}-1*H*-indole-2-carboxamide | 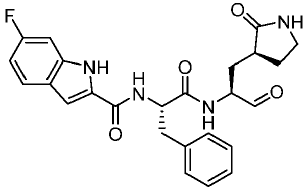 |
| 47 | 4,6-Dichloro-N-{(S)-1-carbonyl-1-{{(S)-1-carbonyl-3-[(S)-2-carbonylpyrrolidin-3-yl]propan-2-yl}amino}-3 - cyclohexylpropan-2-yl}-1*H-*indole-2-carboxamide | 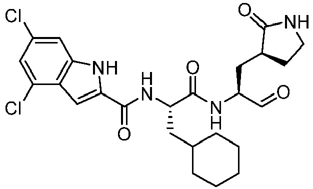 |
| 48 | N-{(S)-1-carbonyl-1-{{(S)-1-carbonyl-3-[(S)-2-carbonylpyrrolidin-3-yl]propan-2-yl} amino}-3-cyclohexylpropan-2-yl}-1*H-*indole-2-carboxamide | 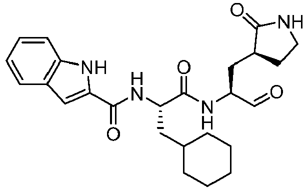 |
| 49 | 1 -Methyl-N- {(S)-1 -carbonyl- 1 - {{(S)-1-carbonyl-3-[(S)-2-carbonylpyrrolidin-3-yl]propan-2-yl}amino}-3 - cyclohexylpropan-2-yl}-1*H*-indole-2-carboxamide | 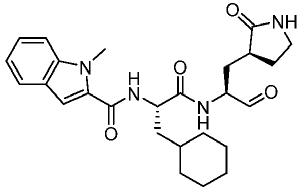 |
| 50 | 5-Methoxy-N-{(S)-1-carbonyl-1-{{(S)-1-carbonyl-3-[(S)-2-carbonylpyrrolidin-3-yl]propan-2-yl}amino}-3 - cyclohexylpropan-2-yl}-1-*H*-indole-2-carboxamide | 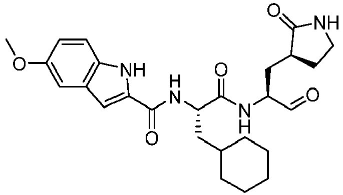 |
| 51 | 4-Fluoro-N-{(S)-1-carbonyl-1-{{(S)-1-carbonyl-3-[(S)-2-carbonylpyrrolidin-3-yl]propan-2-yl}amino}-3 - cyclohexylpropan-2-yl}-1*H*-indole-2-carboxamide | 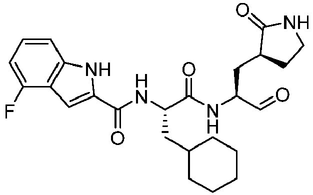 |
| 52 | 5-Fluoro-N-{(S)-1-carbonyl-1-{{(S)-1-carbonyl-3-[(S)-2-carbonylpyrrolidin-3-yl]propan-2-yl}amino}-3 - cyclohexylpropan-2-yl}-1*H*-indole-2-carboxamide | 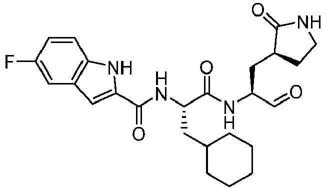 |
| 53 | 6-Fluoro-N-{(S)-1-carbonyl-1-{{(S)-1-carbonyl-3-[(S)-2-carbonylpyrrolidin-3-yl]propan-2-yl}amino}-3 - cyclohexylpropan-2-yl}-1*H*-indole-2-carboxamide | 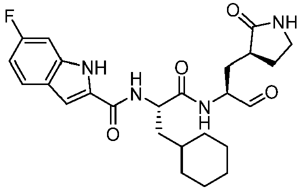 |
| 54 | 7-Fluoro-N-{(S)-1-carbonyl-1-{{(S)-1-carbonyl-3-[(S)-2-carbonylpyrrolidin-3-yl]propan-2-yl}amino}-3 - cyclohexylpropan-2-yl}-1*H*-indole-2-carboxamide | 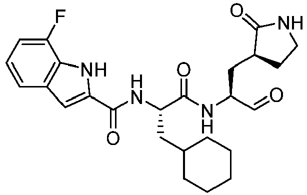 |
| 55 | N-{(S)-1-carbonyl-1-{{(S)-1-carbonyl-3-[(S)-2-carbonylpyrrolidin-3-yl]propan-2-yl} amino}-3-cyclohexylpropan-2-yl} - benzofuran-2-carboxamide | 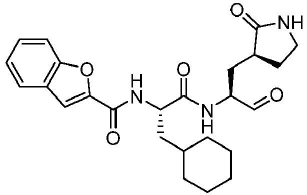 |
| 56 | N-{(S)-1-carbonyl-1-{{(S)-1-carbonyl-3-[(S)-2-carbonylpyrrolidin-3-yl]propan-2-yl} amino }-3-cyclohexylpropan-2-yl}-benzothiophene- 2-carboxamide | 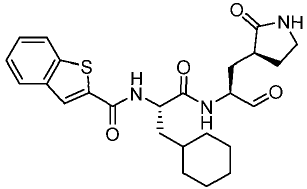 |
| 57 | N-{(S)-1-carbonyl-1-{{(S)-1-carbonyl-3-[(S)-2-carbonylpyrrolidin-3-yl]propan-2-yl} amino }-3-cyclohexylpropan-2-yl}-benzimidazole- 2-carboxamide | 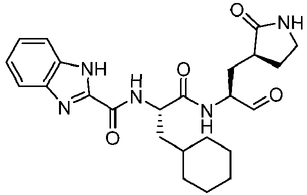 |
| 58 | N-{(S)-1-carbonyl-1-{{(S)-1-carbonyl-3-[(S)-2-carbonylpyrrolidin-3-yl]propan-2-yl} amino }-3-cyclohexylpropan-2-yl}-quinoline- 2-carboxamide | 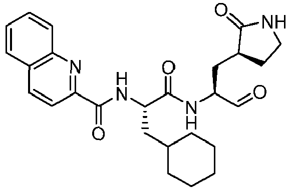 |
| 59 | N-{(S)-1-carbonyl-1-{{(S)-1-carbonyl-3-[(S)-2-carbonylpyrrolidin-3-yl]propan-2-yl} amino }-3-cyclohexylpropan-2-yl}-quinoxaline- 2-carboxamide | 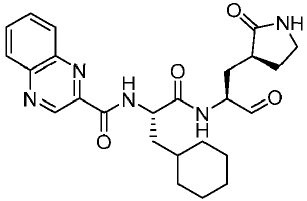 |
| 60 | N-{(S)-2-{{(S)-3-cyclohexyl-1-carbonyl-1-{{(S)-1-carbonyl-3-[(S)-2-carbonylpyrrolidin-3-yl]propan-2-yl} amino }-propan-2-yl}amino}-1-cyclopropylcarbonylethyl} -indole-2-carboxamide | 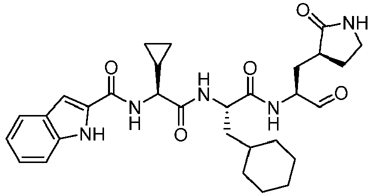 |
| 61 | N-{(S)-2-{{(S)-3-cyclohexyl-1-carbonyl-1-{{(S)-1-carbonyl-3-[(S)-2-carbonylpyrrolidin-3-yl]propan-2-yl} amino }-propan-2-yl}amino}-1-cyclopropylcarbonylethyl}-benzodioxol-5-carboxamide | 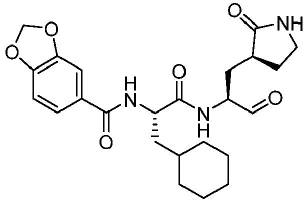 |
| 62 | 5-Methyl-N-{(S)-1-carbonyl-1-{{(S)-1-carbonyl-3-[(S)-2-carbonylpyrrolidin-3-yl]propan-2-yl } amino } -3 -phenylylpropan-2-yl} -isoxazole-3 -carboxamide | 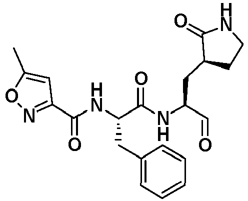 |
| 63 | 5-Fluoro-N-{(S)-1-carbonyl-1-{{(S)-1-carbonyl-3-[(S)-2-carbonylpyrrolidin-3-yl]propan-2-yl } amino } -3 -phenylpropan-2-yl}nicotinamide | 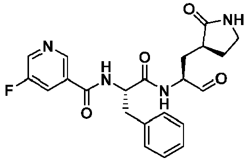 |
| 64 | N-{(S)-1-carbonyl-1-{{(S)-1-carbonyl-3-[(S)-2-carbonylpyrrolidin-3-yl]propan-2-yl}amino}-3-phenylylpropan-2-yl} - benzofuran-5-carboxamide | 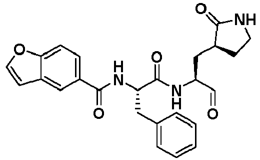 |
| 65 | N-{(S)-1-carbonyl-1-{{(S)-1-carbonyl-3-[(S)-2-carbonylpyrrolidin-3-yl]propan-2-yl}amino}-3-phenylpropan-2-yl}-2,3-dihydrobenzo[b][1,4]dioxane-6-carboxamide | 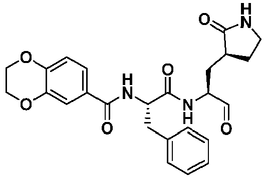 |
| 66 | N-{(S)-1-carbonyl-1-{{(S)-1-carbonyl-3-[(S)-2-carbonylpyrrolidin-3-yl]propan-2-yl}amino}-3-phenylpropan-2-yl}-2,3-dihydrobenzo[b][1,4]dioxane-5-carboxamide | 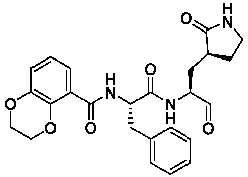 |
| 67 | N-{(S)-1-carbonyl-1-{{(S)-1-carbonyl-3-[(S)-2-carbonylpyrrolidin-3-yl]propan-2-yl}amino}-3-phenylpropan-2-yl}-1*H-*indole-5-carboxamide | 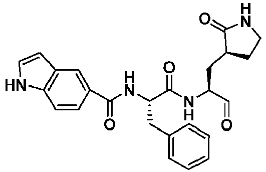 |
| 68 | 6-Bromo-N-{(S)-1-carbonyl-1-{{(S)-1-carbonyl-3-[(S)-2-carbonylpyrrolidin-3-yl]propan-2-yl}amino}-3-phenylpropan-2-yl}imidazo[1,2-a]pyridine-2-carboxamide | 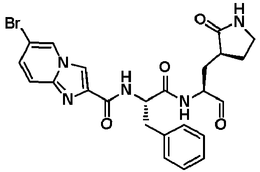 |
| 69 | N-{(S)-1-carbonyl-1-{{(S)-1-carbonyl-3-[(S)-2-carbonylpyrrolidin-3-yl]propan-2-yl}amino}-3-phenylpropan-2-yl}benzo[b]thiophene-2-carboxamide | 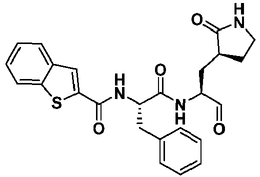 |
| 70 | N-{(S)-1-carbonyl-1-{{(S)-1-carbonyl-3-[(S)-2-carbonylpyrrolidin-3-yl]propan-2-yl}amino}-3-phenylpropan-2-yl} benzofuran-2-carboxamide | 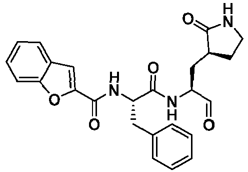 |
| 71 | N-{(S)-1-carbonyl-1-{{(S)-1-carbonyl-3-[(S)-2-carbonylpyrrolidin-3-yl]propan-2-yl}amino}-3-phenylpropan-2-yl} quinoxaline-2-carboxamide | 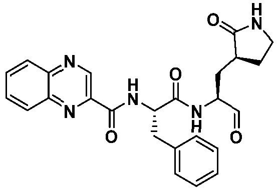 |
| 72 | 3-Methyl-N-{(S)-1-carbonyl-1-{{(S)-1-carbonyl-3-[(S)-2-carbonylpyrrolidin-3-yl]propan-2-yl} amino } -3-phenylpropan-2-yl} quinoxaline-2-carboxamide | 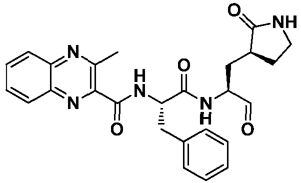 |
| 73 | N-{(S)-1-carbonyl-1-{{(S)-1-carbonyl-3-[(S)-2-carbonylpyrrolidin-3-yl]propan-2-yl}amino }-3-phenylpropan-2-yl}quinoline-2-carboxamide | 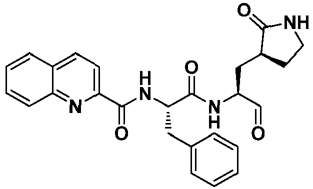 |
| 74 | N-{(S)-1-carbonyl-1-{{(S)-1-carbonyl-3-[(S)-2-carbonylpyrrolidin-3-yl]propan-2-yl}amino}-3-phenylpropan-2-yl}-4-trifluoromethylquinoline-2-carboxamide | 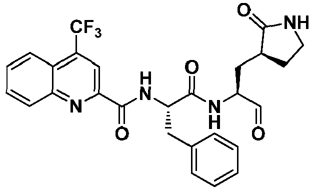 |
| 75 | 7-Bromo-N-{(S)-1-carbonyl-1-{{(S)-1-carbonyl-3-[(S)-2-carbonylpyrrolidin-3-yl]propan-2-yl } amino } -3 -phenylpropan-2-yl} quinoline-2-carboxamide | 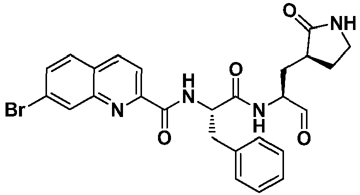 |
| 76 | 6-Chloro-N-{(S)-1-carbonyl-1-{{(S)-1-carbonyl-3-[(S)-2-carbonylpyrrolidin-3-yl]propan-2-yl } amino } -3 -phenylpropan-2-yl}-2*H*-chromene-3 -carboxamide | 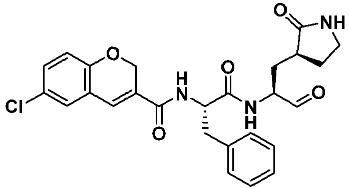 |
| 77 | N-{(S)-3-methyl-1-carbonyl-1-{{(S)-1-carbonyl-1-{{(S)-1-carbonyl-3-[(S)-2-carbonylpyrrolidin-3-yl]propan- 2-yl}amino}-3-cyclohexylpropan-2-yl}amino}butan-2-yl}-2,3-dihydrobenzo[b][1,4]dioxane-6-carboxamide | 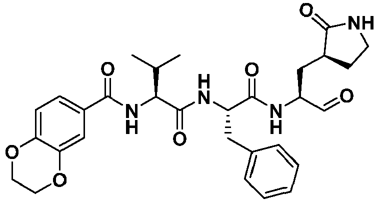 |
| 78 | 5-Methoxy-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3 -cyclohexylpropan-2-yl}amino}butan-2-yl} indole-2-carboxamide | 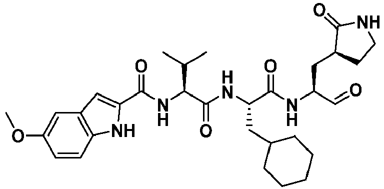 |
| 79 | 4,6-Dichloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}indole-2-carboxamide | 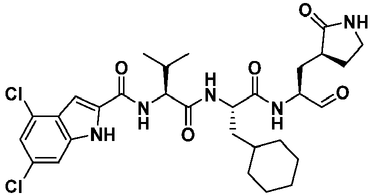 |
| 80 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}cinnamamide | 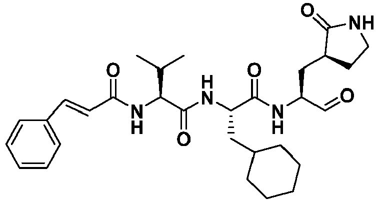 |
| 81 | N-{(S)-1-carbonyl-1-{{(S)-1-carbonyl-3-[(S)-2-carbonylpyrrolidin-3-yl]propan-2-yl}amino}-3-3-fluorophenylpropan-2-yl} - 1*H*-indole- 2-carboxamide | 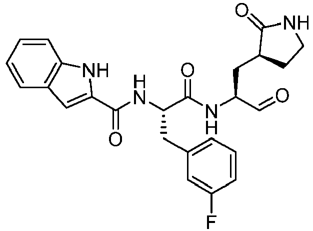 |
| 82 | N-{(S)-1-carbonyl-1-{{(S)-1-carbonyl-3-[(S)-2-carbonylpyrrolidin-3-yl]propan-2-yl}amino}-3-4-fluorophenylpropan-2-yl} - 1*H*-indole- 2-carboxamide | 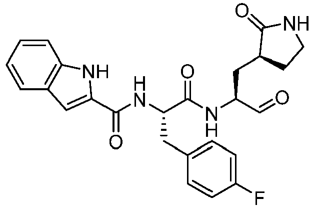 |
| 83 | 4,7-Difluoron-{(S)-1-carbonyl-1-{{(S)-1-carbonyl-3-[(S)-2-carbonylpyrrolidin-3-yl]propan-2-yl}amino}-3-3fluorophenylpropan-2-yl}-1*H*-indole-2-carboxamide | 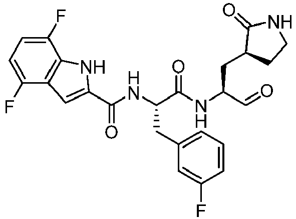 |
| 84 | 4-Methyl-N-{(S)-1-carbonyl-1-{{(S)-1-carbonyl-3-[(S)-2-carbonylpyrrolidin-3-yl]propan-2-yl}amino}-3 - cyclohexylpropan-2-yl}-1*H*-indole-2-carboxamide | 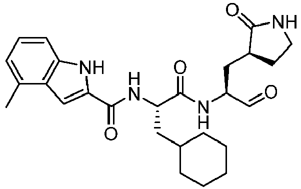 |
| 85 | N-{(S)-1-carbonyl-1-{{(S)-1-carbonyl-3-[(S)-2-carbonylpyrrolidin-3-yl]propan-2-yl}amino}-3-2-fluorophenylpropan-2-yl} - 1H-indole- 2-carboxamide | 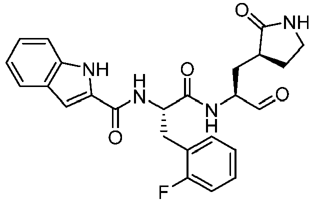 |
| 86 | N-{(S)-1-carbonyl-1-{{(S)-1-carbonyl-3-[(S)-2-carbonylpyrrolidin-3-yl]propan-2-yl}amino} -3 -3 -fluorophenylpropan-2 -yl} - benzimidazole- 2-carboxamide | 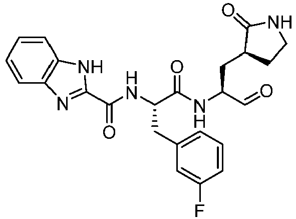 |
| 87 | N-{(S)-1-carbonyl-1-{{(S)-1-carbonyl-3-[(S)-2-carbonylpyrrolidin-3-yl]propan-2-yl}amino } -3 -3 -fluorophenylpropan-2 -yl} - benzofuran-2-carboxamide | 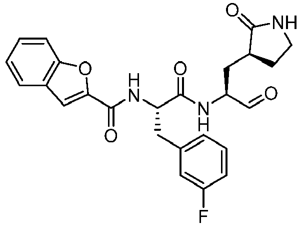 |
| 88 | N-{(S)-1-carbonyl-1-{{(S)-1-carbonyl-3-[(S)-2-carbonylpyrrolidin-3-yl]propan-2-yl}amino } -3 -3 -fluorophenylpropan-2 -yl} - quinoline- 2-carboxamide | 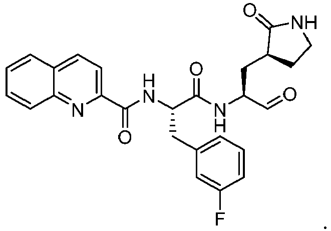 |

In the second aspect of the present invention, a pharmaceutical composition is provided, which comprising (a) a therapeutically effective amount of the aldehyde compounds according to Formula I, or pharmaceutically acceptable salts, enantiomers, diastereomers, racemates or prodrugs thereof; (b) a pharmaceutically acceptable carrier, wherein the aldehyde compounds according to Formula I are as described in the first aspect of the present invention.

In the third aspect of the present invention, the use of the pharmaceutical composition of the second aspect of the present invention is provided, which is used for preparing drugs for the treatment, prevention, and/or alleviation of diseases caused by 2019-nCov infection.

In another preferred embodiment, the diseases caused by 2019-nCov infection are selected from the groups consisting of respiratory tract infection, pneumonia and the complications thereof, or the combinations thereof.

In the third aspect of the present invention, an aldehyde compound according to Formula I, or pharmaceutically acceptable salts, enantiomers, diastereomers or racemates thereof is provided; 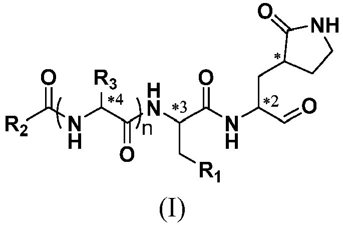 wherein,
the chiral carbon atoms C^{∗}, C^{∗2}, C^{∗3}, and C^{∗4} are each independently in S configuration, R configuration, or the combinations thereof;
n=0 or 1;
R₁ is a group unsubstituted or substituted with 1-3 substituents, and the group is selected from the group consisting of C3-C7 cycloalkyl, trifluoromethyl, C2-C6 alkynyl, 4-7 membered heterocyclyl, C5-C7 aryl, 5-7 membered heteroaryl; each of the heterocyclyl and heteroaryl contains 1-3 heteroatoms selected from oxygen, sulfur and nitrogen; the substituents are each independently selected from the group consisting of halogen, C1-C4 straight or branched alkyl, C1-C4 straight or branched alkenyl, C2-C4 straight or branched alkynyl, C1-C4 straight or branched alkoxy, C1-C4 straight or branched alkylcarbonyloxy, cyano, nitro, hydroxyl, amino, hydroxymethyl, trifluoromethyl, carboxyl, sulfydryl, C1-C4 acyl, amide, sulfonyl, aminosulfonyl, C1-C4 alkyl-substituted sulfonyl, or two adjacent substituents together with the carbon atoms to which they are connected form 5-7 membered ring;
R₂ is a group unsubstituted or substituted with 1-3 substituents, and the group is selected from the group consisting of C3-C7 cycloalkyl, 5-12 membered heterocyclyl (preferably 5-7 membered heterocyclyl or 6-membered aryl fused 5-7 membered heterocyclyl), C6-C12 aryl, 5-12 membered heteroaryl, styryl, or -Cbz; wherein each of the heterocyclyl or heteroaryl contains 1-3 heteroatoms selected from oxygen, sulfur and nitrogen; the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, sulfydryl, C1-C4 acyl, amide, sulfonyl, aminosulfonyl, C1-C4 alkyl-substituted sulfonyl, or two adjacent substituents together with the carbon atoms to which they are connected form 5-7 membered ring;
R₃ is a group unsubstituted or substituted with 1-3 substituents, and the group is selected from the group consisting of C1-C6 straight or branched alkyl, C1-C6 straight or branched alkoxy, C3-C7 cycloalkyl, C6-C12 aryl, 5-12 membered heteroaryl, wherein the heteroaryl contains 1-3 heteroatoms selected from oxygen, sulfur and nitrogen; wherein, the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, sulfydryl, C1-C4 acyl, amide, sulfonyl, aminosulfonyl, C1-C4 alkyl-substituted sulfonyl, or two adjacent substituents together with the carbon atoms to which they are connected form 5-7 membered ring.

In another preferred embodiment, R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl.

In another preferred embodiment, R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocycle and 5-12 membered heteroaromatic ring are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline.

In another preferred embodiment, the chiral carbon atoms C^{∗}, C^{∗2}, C^{∗3}, and C^{∗4} are in S configuration, and/or
R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocycle and 5-12 membered heteroaromatic ring are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline; and/or
R₃ is a group unsubstituted or substituted with 1-3 substituents, and the group is selected from the group consisting of C1-C6 straight or branched alkyl, C3-C7 cycloalkyl, phenyl.

In another preferred embodiment, the compound of formula I is any of compound 1-88 in Table A.

In the fifth aspect of the present invention, a method for treating, preventing, and/or alleviating diseases caused by 2019-nCov infection is provided, which comprises the steps: administering a safe and effective amount of the aldehyde compound according to Formula I, or pharmaceutically acceptable salts, enantiomers, diastereomers or racemates thereof, to the subject in need thereof, wherein the aldehyde compounds according to Formula I are as described above.

In another preferred embodiment, the subject is primate mammal, such as a human.

In the sixth aspect of the present invention, a method for inhibiting the 3CL protease activity of the 2019-nCov is provided, which comprises the steps: contacting the 3CL protease of 2019-nCov with the aldehyde compound according to Formula I, or pharmaceutically acceptable salts, enantiomers, diastereomers or racemates thereof so as to inhibit the 3CL protease activity of the 2019-nCov.

In another preferred embodiment, the method is non-therapeutic and nondiagnostic.

In another preferred embodiment, the method is *in vitro.*

In another preferred embodiment, the 3CL protease of 2019-nCov is recombinant or expressed by 2019-nCov.

It should be understood that, within the scope of the present invention, the above-mentioned technical features herein and the technical features specifically described in the following (such as the examples) can be combined with each other, thereby constituting new or preferred technical solutions which need not be specified again herein.

### DESCRIPTION OF THE DRAWINGS

Figure 1 shows that the compounds of the present invention can inhibit the replication of 2019 nCoV virus.
Figure 2 shows the inhibition curves and EC₅₀ values of some compounds of the present invention in inhibiting the 2019-nCov.
Figure 3 shows the structure of the crystal complex formed by Compound 48, 81 with SARS-CoV-2 3Cl^{pro}.

### EMBODIMENTS FOR CARRYING OUT THE INVENTION

After extensive and in-depth research and extensive screening, the inventors first time unexpectedly developed a class of active ingredient that can effectively inhibit the 2019-nCov, that is, the compounds according to Formula I, or pharmaceutically acceptable salts, enantiomers, diastereomers or racemates thereof. Tests have shown that the active ingredient of the present invention can effectively inhibit the 3CL protease activity of 2019-nCov, thereby inhibiting the replication and viability of 2019-nCov. The present invention has been completed on this basis.

### Terms

Unless otherwise specified, the term "substituted" herein refers to the substitution of one or more hydrogen atoms on the group with substituents selected from the group consisting of C₁-C₁₀ alkyl, C₃-C₁₀ cycloalkyl, C₁-C₁₀ alkoxy, halogen, hydroxyl, carboxyl (-COOH), C₁-C₁₀ aldehyde group, C₂-C₁₀ acyl, C₂-C₁₀ ester group, amino, phenyl; the phenyl includes unsubstituted or substituted phenyl with 1-3 substituents, the substituents are selected from halogen, C₁-C₁₀ alkyl, cyano, OH, nitro, C₃-C₁₀ cycloalkyl, C₁-C₁₀ alkoxy, and amino.

Unless otherwise specified, each chiral carbon atom in all the compounds herein may optionally be in the R configuration or the S configuration, or mixtures of the R configuration and the S configuration.

The term "C1-C6 alkyl" refers to straight or branched chain alkyl groups having 1 to 6 carbon atoms, such as methyl, ethyl, propyl, isopropyl, butyl, isobutyl, sec-butyl, tert-butyl, or similar groups.

The term "3-8 membered heterocyclyl" refers to groups formed by losing one hydrogen atom of 3-8 membered saturated rings with 1-3 heteroatoms selected from the group consisting of N, S, O; for example, pyrrolidinyl, piperidinyl, piperazinyl, morpholinyl, or similar groups.

The term "6-10 membered aryl" refers to groups formed by losing one hydrogen atom of 6-10 membered aryl groups; for example, phenyl group, naphthyl group, or similar groups.

The term "5-10 membered heteroaryl" refers to groups formed by losing one hydrogen atom of 5-8 membered aryl groups h with 1-3 heteroatoms selected from the group consisting of N, S, O, where each ring system of heteroaryl can be monocyclic or polycyclic; for example, pyrrolyl, pyridyl, thienyl, furyl, imidazolyl, pyrimidinyl, benzothienyl, indolyl, imidazopyridyl, quinolinyl, or similar groups.

The term "C1-C6 alkoxy" refers to straight or branched alkoxy groups having 1 to 6 carbon atoms, such as methoxy, ethoxy, propoxy, isopropoxy, butoxy, isobutoxy, sec-butoxy, tert-butoxy, or similar groups.

The term "C2-C6 ester group" refers to R-O-C(=O)- groups having 2-6 carbon atoms, such as -COOCH₃, -COOC₂H₅, -COOC₃H₇, -COOC₄H₉, or similar groups.

The term "C2-C6 alkenyl" refers to groups formed by losing one or two hydrogen atoms of olefins with 2-6 carbon atoms. The olefin may be monoolefin, diolefin or triolefin, for example, -CH= CH₂, -C₂H₄=CH₂, -CH=C₂H₄, or similar groups.

The term "halogen" refers to F, Cl, Br, and I.

Unless otherwise specified, the structural formula described herein are intended to include all isomeric forms (such as enantiomeric, diastereomeric, and geometric isomers (or conformational isomers)): for example, R, S configuration of asymmetrical centers, (Z), (E) isomers of double bonds, and (Z), (E) conformational isomers, etc. Therefore, the single stereochemical isomers or enantiomers, diastereomers or geometric isomers (or conformers) of the compounds of the invention, or mixtures thereof all fall within the scope of the invention.

The term "tautomer" means that structural isomers having different energies can exceed the low energy barrier and thereby transform between each other. For example, proton tautomers (proton shift) includes interconversion by proton transfer, such as 1H-indazole and 2H-indazole, 1H-benzo[d]imidazole and 3H-benzo[d]imidazole. Valence tautomers include interconversion through some bonding electron recombination.

The form "C1-C6" herein means that the group can have 1 to 6 carbon atoms, such as 1, 2, 3, 4, or 5.

### Active ingredient

In the present invention, an active ingredient that can effectively inhibit the replication of the 2019-nCov is provided. The active ingredient is the compound r according to General Formula I, and the active ingredient can effectively prevent, treat and/or alleviate 2019-nCov related diseases.

Tests have shown that the active ingredient of the present invention can effectively inhibit the 3CL protease of the 2019-nCov, thereby inhibiting the replication of the 2019-nCov, thereby preventing, treating and/or alleviating 2019-nCov related diseases. 2019-nCov is also known as SARS-CoV-2.

It should be understood that, the active ingredient of the present invention includes the aldehyde compounds according to Formula (I), or pharmaceutically acceptable salts, enantiomers, diastereomers, or racemates thereof, or prodrugs thereof. It should be understood that, the active ingredient of the present invention also includes the crystalline forms, amorphous compounds, and deuterated compounds of the compound of Formula (I).

The "pharmaceutically acceptable salt" refers to the conventional non-toxic salts formed by reacting the compounds of Formula (I) with inorganic acids or organic acids. For example, the conventional non-toxic salts can be prepared by reacting the compounds of Formula (I) with inorganic acids or organic acids, the inorganic acids include hydrochloric acid, hydrobromic acid, sulfuric acid, nitric acid, aminosulfonic acid and phosphoric acid, etc, organic acids include citric acid, tartaric acid, lactic acid, pyruvic acid, acetic acid, benzenesulfonic acid, p-toluenesulfonic acid, methanesulfonic acid, naphthalenesulfonic acid, ethanesulfonic acid, naphthalene disulfonic acid, maleic acid, malic acid, malonic acid, fumaric acid, succinic acid, propionic acid, oxalic acid, trifluoroacetic acid, stearic acid, pamoic acid, hydroxymaleic acid, phenylacetic acid, benzoic acid, salicylic acid, glutamic acid, ascorbic acid, p-aminobenzenesulfonic acid, 2-acetoxybenzoic acid and isethionic acid, etc.; or the sodium, potassium, calcium, aluminum or ammonium salts formed with inorganic bases after the compounds of Formula (I) formed esters with propionic acid, oxalic acid, malonic acid, succinic acid, fumaric acid, maleic acid, lactic acid, malic acid, tartaric acid, citric acid, aspartic acid, or glutamic acid; or amine, ethylamine or ethanolamine salts formed by the compounds of Formula (I) with organic bases; or the corresponding inorganic acid salts formed with hydrochloric acid, hydrobromic acid, hydrofluoric acid, sulfuric acid, nitric acid or phosphoric acid after the compounds of Formula (I) formed esters with lysine, arginine, ornithine or the corresponding organic acid salts formed with formic acid, acetic acid, picric acid, methanesulfonic acid or ethanesulfonic acid.

### Pharmaceutical composition and use

The present invention also provides the use of the mixtures of one or more of the aldehyde compounds according to Formula (I), or pharmaceutically acceptable salts, enantiomers, diastereomers or racemates and prodrugs thereof as active ingredients for the preparation of drugs for the treatment and/or prevention, alleviation of respiratory infections, pneumonia and other related diseases caused by 2019 n-Cov infection.

The pharmaceutical composition provided by the present invention preferably contains the active ingredient in a weight ratio of 0.001-99wt%, the preferred ratio is that the compound of Formula I as the active ingredient accounts for 0.1 wt%-90wt% of the total weight, and the rest is pharmaceutically acceptable carrier, diluent, solution or salt solution.

When needed, one or more pharmaceutically acceptable carriers can be added to the drug. The carriers include conventional diluents, excipients, fillers, binders, wetting agents, disintegrants, absorption promoters, surfactants, adsorption carriers, lubricants and the like in the pharmaceutical field.

The compounds and pharmaceutical compositions provided by the present invention can be in various forms, such as tablets, capsules, powders, syrups, solutions, suspensions and aerosols, etc., and can be present in suitable solid or liquid carriers or diluents, also in the sterilization equipments for injection or drip infusion.

Various dosage forms of the pharmaceutical compositions of the present invention can be prepared according to conventional preparation methods in the pharmaceutical field. The unit measurement of the preparation formula usually contains 0.05-400 mg of the compounds of Formula I, preferably, the unit measurement of the preparation formula contains 1 mg-500 mg of the compounds of Formula I.

The compounds and pharmaceutical compositions of the present invention can be used on mammals in clinical usage, including humans and animals, and can be administered via pathways such as oral, nose, skin, lung, or gastrointestinal. Most preferred is oral administration. The most preferred daily dose is 0.01-400 mg/kg body weight, taken at one time, or 0.01-200 mg/kg body weight, taken in divided doses. Regardless of the administration method, the individual's optimal dosage should be determined based on the specific treatment. Usually it should start with smaller dose and increase the dose gradually until the most suitable dose is found.

The drugs or inhibitors of the present invention can be administered in various ways, for example, it can be introduced into the body such as muscle, intradermal, subcutaneous, vein, mucosal tissue by methods such as injection, spraying, nose drops, eye drops, penetration, absorption, physical or chemically mediation; or be introduced into the body by mixed or wrapped by other substances.

### The main advantages of the present invention include:

(a) The compounds of the present invention can effectively inhibit 2019-nCoV 3CL protease, and some of the compounds have IC₅₀ values at about 70 nM.
(b) The compounds of the present invention has a higher inhibitory rate on 2019-nCoV at the viral level than the positive control CQ, showing a better anti-2019-nCoV potentiality.
(c) The compounds of the present invention have low toxic and side effects and good drug-making properties.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

The analytical data of samples were measured by the following instruments: NMR was measured by GEMINI-300, Bruker AMX-400 and INVOA-600 NMR instruments, TMS (tetramethylsilane) as the internal standard, the unit of chemical shift was ppm, the unit of coupling constant was Hz; the mass spectrum was measured by Finnigan MAT-711, MAT-95 and LCQ-DECA mass spectrometers and IonSpec 4.7 Tesla mass spectrometer.

200-300 Mesh silica gel (produced by Qingdao Ocean Chemical Plant) was used for column chromatography; TLC silica gel plate was HSGF-254 thin-layer chromatography prefabricated plate produced by Yantai Chemical Plant; boiling range of petroleum ether was 60-90 °C; UV lamp and iodine cylinder was used for color development. Unless otherwise specified, the conventional reagents and medicines used in the following examples were purchased from Sinopharm Group. The reagents and solvents used in the experiment were handled according to the specific conditions of the reaction.

### Example 1: Synthesis of Compound 1

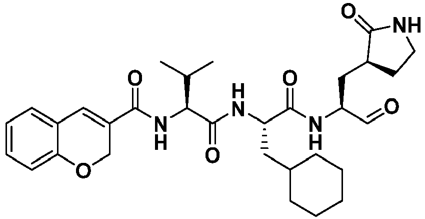

synthetic route:

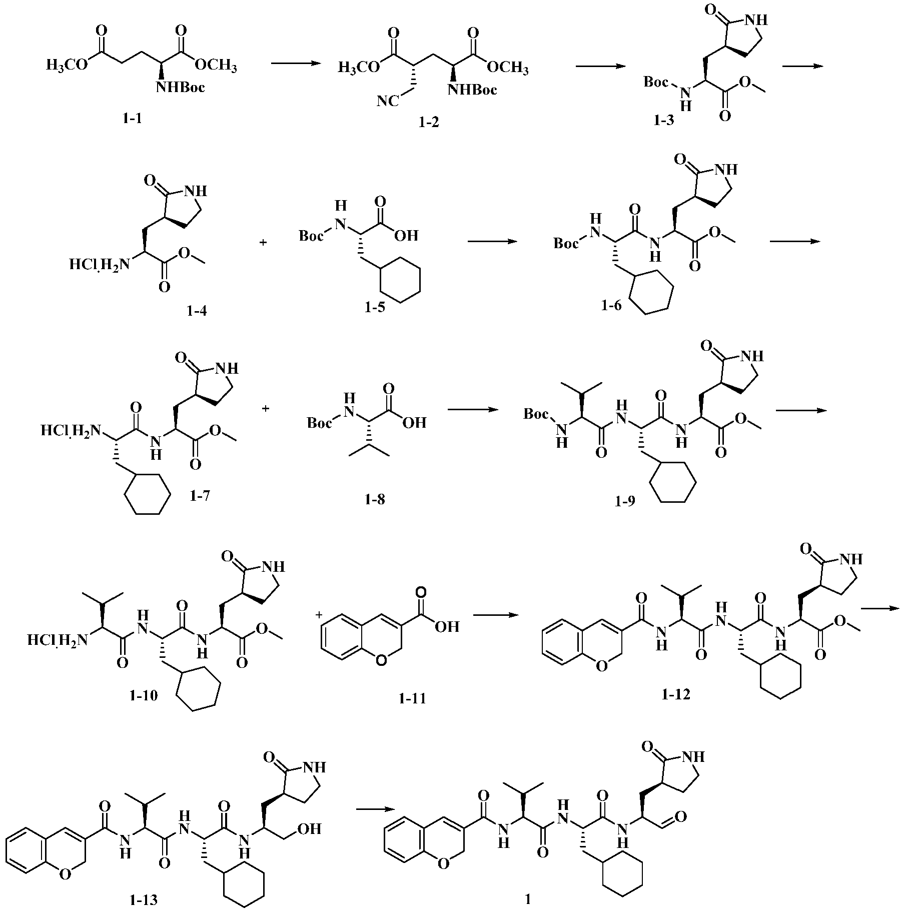

### Synthesis of Compound 1-2:

Under argon protection, dimethyl N-tert-butoxycarbonyl-L-glutamate 1-1 (6 g, 21.8 mmol) was dissolved in 60 mL of anhydrous tetrahydrofuran, and LiHMDS (1M in THF) in tetrahydrofuran solution (47 mL, 47 mmol)was slowly added dropwise at -78 °C, while the temperature was kept stable at -78 °C during the process for about 1 hour. The mixture was stirred at -78 °C for 1 hour. Bromoacetonitrile (2.79 g, 23.3 mmol) was dissolved in 20 ml of tetrahydrofuran, and then the solution was slowly added dropwise into the system for 1-2 hours. The temperature was controlled at -78°C, and the reaction was continued for 20 hours. After THL monitoring (alkaline potassium permanganate was used for color development) showed that the reaction was completed, 3 mL of methanol and 22.7 mL (v/v=1/7.5) of the mixture of glacial acetic acid and tetrahydrofuran were added to the reaction solution to quench. The mixture was warmed to room temperature after stirred for 10 min. 40 mL of saturated sodium chloride solution was poured and well stirred. The reaction system was observed to have layered. The organic layer was separated, and the aqueous phase was extracted with ethyl acetate (EA). The combined organic layer was dried over anhydrous sodium sulfate, concentrated, and separated by column chromatography (PE:EA=4:1) to obtain a pale yellow oily substance **1-2** 3.9 g, yield 58%.

### Synthesis of Compound 1-3:

Compound **1-2** (1 g, 3.15 mmol) was dissolved in 25 mL of anhydrous methanol, after stirred under an ice bath to 0 °C, cobalt dichloride hexahydrate (450 mg, 1.89 mmol) was added and the solution turned to purple-red. 10 minutes later, sodium borohydride (715 mg, 18.9 mmol) was added in small portions, and the color of the solution turned to purple-black. The reaction solution continued to react in an ice bath for 1 hour and then warmed to room temperature. After 15 h, the mixture was quenched with 5 mL of saturated NH₄Cl solution and stirred for 10 min.The solid was filtered off with diatomite, and the filtrate was evaporated to dryness under reduced pressure, anextracted with 20 mL of water and 30×3 mL of ethyl acetate. The combined organic phase was dried with anhydrous Na₂SO₄ for 1 h, then concentrated under reduced pressure and separated by column chromatography [PE: EA=1: 2] to obtain white powdery solid Compound **1-3** 460 mg, yield 51%.

### Synthesis of Compound 1-4:

Intermediate **1-3** (1 g, 3.5 mmol) was dissolved in dichloromethane, 4 M HCl in dioxane solution (9 mL, 35 mmol) was added at 0°C, and the reaction was stirred at room temperature for 12 hours. The solution was evaporated to dryness to obtain Intermediate **1-4** and used directly to the next reaction.

### Synthesis of Compound 1-6:

Compound **1-5** (1.1 g, 3.5 mmol) was dissolved in dichloromethane (40 mL), the reaction solution was cooled to -20°C, then HATU (1.9 g, 4.9 mmol) was added to the reaction solution. After stirred for twenty minutes, Intermediate **1-4** obtained in the previous step was added to the reaction solution and stirred at - 20°C for 30 minutes. DIPEA (1.7 mL, 10.5 mmol) was added dropwise to the reaction solution. After stirred for 12 hours, the reaction was extracted with ammonium chloride (40×3 mL), sodium bicarbonate (40×3 mL), and sodium chloride (40×3 mL). The combined organic phase was dried with anhydrous sodium sulfate for 1 hour, then distillated under reduced pressure and separated by column chromatography (DCM: CH₃OH, 40:1 v/v) to obtain white powder solid Compound **1-6** 1.3 g, yield 83%.

### Synthesis of Compound 1-7:

Compound **1-6** (1.5 g, 3.5 mmol) was dissolved in dichloromethane, 4 M HCl dioxane solution (9 mL, 35 mmol) was added at 0°C, and the reaction was stirred at room temperature for 12 hours. Then the solution was evaporated to dryness to obtain Intermediate **1-7** and used directly to the next step.

### Synthesis of Compound 1-9:

Compound **1-8** (0.76 g, 3.5 mmol) was dissolved in dichloromethane (40 mL), and the reaction solution was cooled to -20°C, then HATU (1.9 g, 4.9 mmol) was added to the reaction solution. After stirred for twenty minutes, Intermediate **1-7** obtained in the previous step was added to the reaction solution, then stirred at - 20°C for 30 minutes. DIPEA (1.7 mL, 10.5 mmol) was added dropwise to the reaction solution. After the reaction was stirred for 12 hours, it was extracted with ammonium chloride (40×3 mL), sodium bicarbonate (40×3 mL) and sodium chloride (40×3 mL). The combined organic phase was dried with anhydrous sodium sulfate for 1 hour, then distillated under reduced pressure and separated by column chromatography (DCM: CH₃OH, 40:1 v/v), to obtain white powder solid Compound **1-9** 1.6g, yield 85%.

### Synthesis of Compound 1-10:

Intermediate **1-9** (1.86 g, 3.5 mmol) was dissolved in dichloromethane, 4 M HCl dioxane solution (9 mL, 35 mmol) was added at 0°C, and the reaction was stirred at room temperature for 12h. Then the solution was evaporated to dryness to obtain Intermediate **1-10** and used directly in the next step.

### Synthesis of Compounds 1-12:

Compound **1-11** (0.61 g, 3.5 mmol) was dissolved in dichloromethane (40 mL), the reaction solution was cooled to -20°C, then HATU (1.9 g, 4.9 mmol) was added to the reaction solution. After stirred for twenty minutes, Intermediate (10) obtained in the previous step was added to the reaction solution, stirred at -20°C for 30 minutes, and then DIPEA (1.7 mL, 10.5 mmol) was added dropwise to the reaction solution. After stirred for 12 hours, the reaction was extracted with ammonium chloride (40×3 mL), sodium bicarbonate (40×3 mL) and sodium chloride (40×3 mL). The combined organic phase was dried with anhydrous sodium sulfate for 1 hour, distillated under reduced pressure and separated by column chromatography (DCM: CH₃OH, 20:1 v/v) to obtain white powder solid compound(12) 1.7g, yield 81%.

### Synthesis of Compound 1-13:

Compound **1-12** (304 mg, 0.51 mmol) was dissolved in 20 ml of dichloromethane, sodium borohydride (107 mg, 2.9 mmol) was slowly added in batches, then 1 ml of methanol was added dropwise. The mixture was stirred at room temperature for about 2 hours. After the reaction was completed, about 20 ml of saturated brine was added to quench the reaction, and dichloromethane was added for extraction. The organic phase was washed with saturated brine, dried with anhydrous sodium sulfate, and separated by column chromatography (DCM: CH₃OH, 20:1 v/v) to obtain white solid (13) 216 mg, yield 74%.

### Synthesis of Compound 1:

Compound **1-12** (165 mg, 0.29 mmol) was dissolved in 20 ml of dichloromethane, and Dess-Martin oxidant (147 mg, 0.35 mmol) was added, and stirred at room temperature. After TLC monitoring (UV) showed that the reaction was completed, the solution was extracted with sodium thiosulfate until clearation, then dried with anhydrous sodium sulfate and concentrated, column chromatography separated (DCM: CH₃OH, 20:1 v/v) to obtain Compound **1** 98 mg, yield 60%.
¹H NMR(600 MHz,Acetone-d6) δ 9.30 (s,1H), 7.75 (d,*J*=9.0Hz,1H), 7.66-7.57(m,3H), 7.25 (t,*J*=7.8Hz,1H), 7.07 (s,1H), 7.00-6.93(m,2H), 6.55 (t,*J*=4.4Hz,1H), 4.91 (s,2H), 4.76 (dd,J=9.1,6.5Hz,1H), 4.28-4.14(m,2H), 3.29(m,1H), 3.16(m,1H), 2.35(m,1H), 2.11-1.95(m,3H), 1.85-1.43(m,10H), 1.40-1.18(m,5H), 0.67 (dd,J=25.0,6.7Hz,6H). ESI-MS m/z 566.31[M+H]⁺

### Example 2: Synthesis of Compound 2

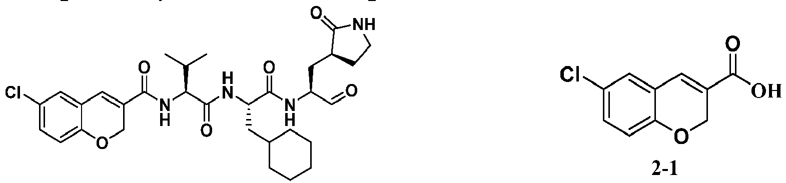

The Compound **2** was synthesized according to the synthesis of Compound **1** by using Compound **2-1** to replace the acid **1-11** in Example 1.
¹H NMR(600 MHz,Acetone-d6) δ 9.21 (s,1H), 7.73 (s,1H), 7.67-7.59(m,3H), 7.31-7.23(m,2H), 6.91 (d,J=8.4Hz,1H), 6.55 (t,J=4.4Hz,1H), 4.91 (s,2H), 4.67 (dd,J=9.1,6.5Hz,1H), 4.44 (dt,J=8.8,6.4Hz,1H), 4.29 (dt,J=9.3,6.9Hz,1H), 3.35-3.19(m,2H), 2.53(m,1H), 2.18 (dt,J=12.7,6.3Hz,1H), 2.13-2.00(m,1H), 1.97 (dt,J=12.9,6.4Hz,1H), 1.91-1.68(m,4H), 1.65-1.59(m,1H), 1.59-1.44(m,3H), 1.44-1.32(m,6H), 1.23(m,2H), 0.67 (dd,J=25.0,6.7Hz,6H). ESI-MS m/z 600.27[M+H]⁺

### Example 3: Synthesis of Compound 3

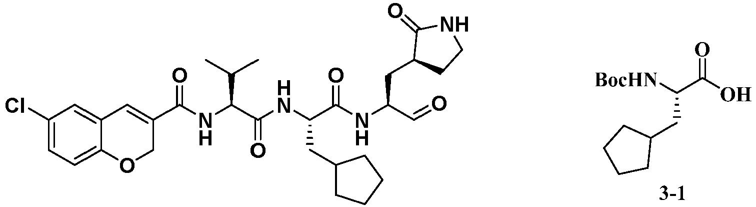

The Compound **3** was synthesized according to the synthesis of Compound **1** by using Compound **2-1** to replace the acid **1-11** in Example 1, and using **3-1** to replace the **1-5** in Example 1.
¹H NMR(600 MHz,Acetone-d6) δ 9.21 (s,1H), 7.82 (d,J=9.2Hz,1H), 7.74 (s,1H), 7.62 (dd,J=9.2,2.2Hz,2H), 7.28 (d,J=8.4Hz,1H), 7.23 (s,1H), 6.91 (d,J=8.4Hz,1H), 6.55 (t,J=4.4Hz,1H), 4.91 (s,2H), 4.78 (dd,J=9.1,6.5Hz,1H), 4.32 (dt,J=9.0,6.4Hz,1H), 4.19 (dt,J=9.3,6.9Hz,1H), 3.29(m,1H), 3.17(m,1H), 2.35(m,1H), 2.09 (dt,J=12.8,6.3Hz,1H), 2.05-1.94(m,1H), 1.94-1.87(m,1H), 1.87-1.77(m,2H), 1.77-1.67(m,3H), 1.67-1.64(m,1H), 1.64-1.56(m,3H), 1.56-1.45(m,4H), 0.67 (dd,J=25.0,6.7Hz,6H). ESI-MS m/z 586.26[M+H]⁺

### Example 4: Synthesis of Compound 4

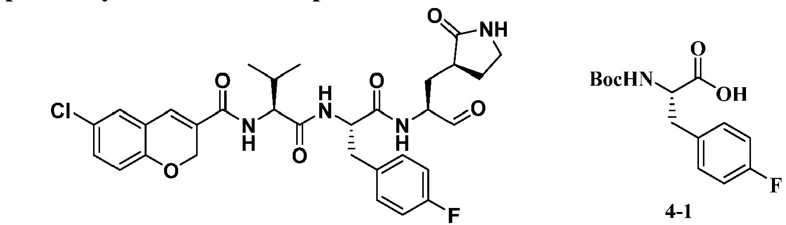

The Compound **4** was synthesized according to the synthesis of Compound **3** by using Compound **4-1** to replace the **3-1** in Example 3.
¹H NMR(600 MHz,Acetone-d6) δ 9.21 (s,1H), 7.77 (d,J=9.3Hz,1H), 7.66-7.58(m,3H), 7.41 (s,1H), 7.31-7.22(m,3H), 6.93 (t,J=8.1Hz,2H), 6.82 (d,J=8.4Hz,1H), 6.55 (t,J=4.4Hz,1H), 4.97 (dd,J=9.2,6.6Hz,1H), 4.91 (s,2H), 4.73 (dt,J=9.3,7.7Hz,1H), 4.40 (dt,J=9.0,6.4Hz,1H), 3.33(m,1H), 3.21(m,1H), 3.04 (dd,J=14.0,7.8Hz,1H), 2.92 (dd,J=14.0,7.8Hz,1H), 2.44(m,1H), 2.16-2.01(m,2H), 1.96-1.81(m,2H), 1.73(m,1H), 0.86 (dd,J=25.0,6.7Hz,6H). ESI-MS m/z 612.22[M+H]⁺

### Example 5: Synthesis of Compound 5

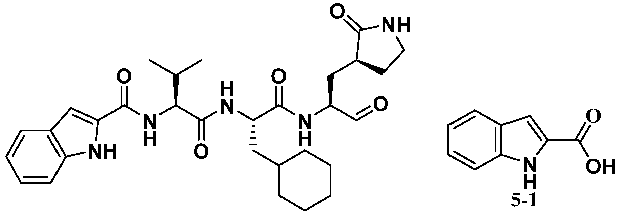

The Compound **5** was synthesized according to the synthesis of Compound **1** by using Compound **5-1** to replace the acid **1-11** in Example 1.
¹H NMR(600 MHz,Acetone-d6) δ 9.21 (s,1H), 9.12 (s,1H), 8.55 (d,J=9.2Hz,1H), 7.69-7.55(m,3H), 7.44-7.32(m,2H), 7.28-7.12(m,2H), 6.55 (t,J=4.4Hz,1H), 5.11 (dd,J=9.2,6.6Hz,1H), 4.50-4.23(m,2H), 3.34(m,1H), 3.20(m,1H), 2.40(m,1H), 2.29-2.19(m,J=6.7Hz,1H), 2.14 (dt,J=12.9,6.4Hz,1H), 1.99-1.70(m,5H), 1.58-1.32(m,10H), 1.26-1.18(m,2H), 0.87 (dd,J=25.0,6.7Hz,6H). ESI-MS m/z 552.31[M+H]⁺

### Example 6: Synthesis of Compound 6

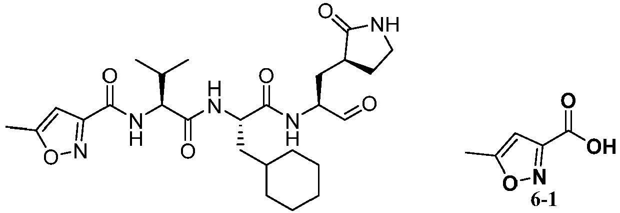

The Compound **6** was synthesized according to the synthesis of Compound **1** by using Compound **6-1** to replace the acid **1-11** in Example 1.
¹H NMR(600 MHz,Acetone-d6) δ 9.30 (s,1H), 8.18 (d,J=9.2Hz,1H), 7.86 (d,J=9.3Hz,1H), 7.65 (d,J=9.0Hz,1H), 6.58-6.52(m,2H), 5.18 (dd,J=9.1,6.5Hz,1H), 4.41 (dt,J=8.8,6.3Hz,1H), 4.33 (dt,J=9.3,6.9Hz,1H), 3.31(m,1H), 3.19(m,1H), 2.47 (s,3H), 2.39(m,1H), 2.18-2.04(m,2H), 1.92(m,2H), 1.82(m,1H), 1.78-1.69(m,2H), 1.55-1.43(m,5H), 1.43-1.31(m,5H), 1.19(m,2H), 0.88 (dd,J=24.9,6.6Hz,6H). ESI-MS m/z 517.29[M+H]⁺

### Example 7: Synthesis of Compound 7

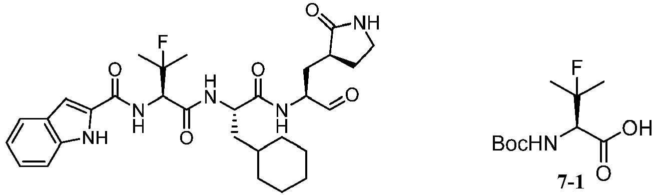

The Compound **7** was synthesized according to the synthesis of Compound **1** by using Compound **5-1** to replace the acid **1-11** in Example 1, and using Compound **7-1** to replace the **1-8** in Example 1.
¹H NMR(600 MHz,Acetone-d6) δ 9.21 (s,1H), 8.28 (d,J=9.0Hz,1H), 7.68 (d,J=9.3Hz,1H), 7.65 (d,J=8.8Hz,1H), 6.55 (t,J=4.4Hz,1H), 6.39 (d,J=6.0Hz,1H), 6.30 (dd,J=8.7,7.3Hz,1H), 6.21 (dd,J=8.7,7.3Hz,1H), 6.14 (d,J=8.6Hz,1H), 6.06 (dd,J=8.7,5.7Hz,1H), 5.84 (dd,J=8.7,5.3Hz,1H), 4.61 (dd,J=25.3,9.0Hz,1H), 4.50 (dt,J=9.3,6.9Hz,1H), 4.31 (dt,J=8.8,6.4Hz,1H), 3.77 (dt,J=8.6,5.2Hz,1H), 3.37-3.29(m,2H), 3.20(m,1H), 2.41(m,1H), 2.13 (dt,J=12.7,6.3Hz,1H), 1.95-1.71(m,5H), 1.65 (t,J=25.1Hz,6H), 1.59-1.34(m,9H), 1.29(m,2H). ESI-MS m/z 572.32[M+H]⁺.

### Example 8: Synthesis of Compound 8

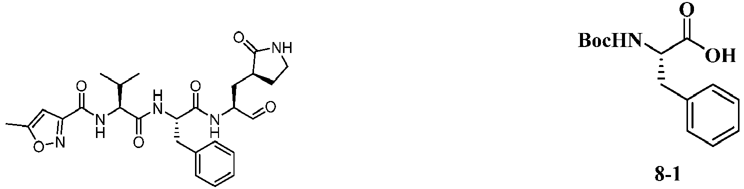

The Compound **8** was synthesized according to the synthesis of Compound **1** by using Compound **6-1** to replace the acid **1-11** in Example 1, and using compound **8-1** to replace the **1-5** in Example 1.
¹H NMR(600 MHz,Acetone-d6) δ 9.35 (s,1H), 8.30 (d,J=6.8Hz,1H), 7.81-7.69(m,1H), 7.58 (d,J=8.4Hz,1H), 7.31-7.22(m,4H), 7.17 (t,J=7.1Hz,1H), 6.94 (s,1H), 6.52 (d,J=0.7Hz,1H), 4.79(m,1H), 4.54-4.43(m,1H), 4.27(m,1H), 3.32-3.18(m,3H), 3.04 (dd,J=13.8,8.1Hz,1H), 2.44-2.28(m,2H), 2.22 (dd,J=13.4,6.7Hz,1H), 1.93 (d,J=5.6Hz,1H), 1.81-1.72(m,2H), 1.31 (s,2H), 0.93 (dd,J=10.3,6.8Hz,6H). ESI-MS m/z 511.24[M+H]⁺.

### Example 9: Synthesis of Compound 9

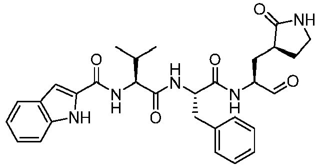

The Compound **9** was synthesized according to the synthesis of Compound **1** by using Compound **5-1** to replace the acid **1-11** in Example 1, and using Compound **8-1** to replace the **1-5** in Example 1.
¹H NMR(600 MHz,Acetone-d6) δ 11.20 (s,1H), 9.46 (s,1H), 8.22 (d,J=7.4Hz,1H), 7.96-7.88(m,1H), 7.75 (dd,J=11.9,4.0Hz,1H), 7.62(m,2H), 7.33-7.29(m,1H), 7.26-7.20(m,3H), 7.19-7.06(m,4H), 7.03 (dd,J=16.8,9.5Hz,1H), 4.88-4.80(m,1H), 4.54-4.32(m,2H), 3.38 (dd,J=19.0,10.1Hz,1H), 3.32-3.24(m,2H), 3.00(m,1H), 2.43(m,1H), 2.32(m,2H), 2.23(m,1H), 1.87-1.81(m,1H), 1.31 (d,J=1.7Hz,1H), 0.96 (dd,J=10.7,6.9Hz,6H). ESI-MS m/z 546.26[M+H]⁺.

### Example 10: Synthesis of Compound 10

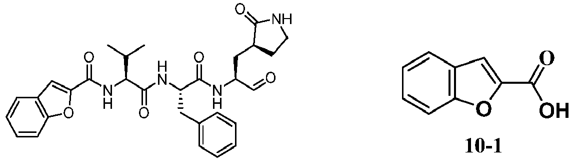

The Compound **10** was synthesized according to the synthesis of Compound **1** by using Compound **10-1** to replace the acid **1-11** in Example 1, and using Compound **8-1** to replace the **1-5** in Example 1.
¹H NMR(600 MHz,Acetone-d6) δ 9.21 (s,2H), 8.32 (d,J=9.2Hz,2H), 7.74-7.67(m,4H), 7.63 (dd,J=12.5,8.3Hz,4H), 7.58 (d,J=9.3Hz,2H), 7.39 (t,J=7.7Hz,2H), 7.33-7.23(m,7H), 7.23-7.18(m,1H), 7.16-7.08(m,4H), 6.55 (t,J=4.4Hz,2H), 5.01 (dd,J=9.2,6.6Hz,2H), 4.75 (dt,J=9.3,7.8Hz,2H), 4.38 (dt,J=8.8,6.3Hz,2H), 3.38-3.28(m,4H), 3.26-3.10(m,4H), 2.42(m,2H), 2.28-2.16(m,J=6.6Hz,2H), 2.13 (dt,J=12.8,6.3Hz,2H), 1.92 (dt,J=12.8,6.3Hz,2H), 1.83(m,2H), 1.74(m,2H), 0.88 (dd,J=25.1,6.6Hz,12H). ESI-MS m/z 547.25[M+H]⁺.

### Example 11: Synthesis of Compound 11

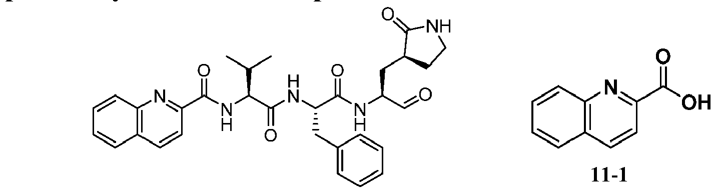

The Compound **11** was synthesized according to the synthesis of Compound **1** by using Compound **11-1** to replace the acid **1-11** in Example 1, and using compound **8-1** to replace the **1-5** in Example 1.
¹H NMR(600 MHz,Acetone-d6) δ 9.40 (s,1H), 8.74 (d,J=8.5Hz,1H), 8.52 (d,J=8.5Hz,1H), 8.27 (d,J=8.5Hz,1H), 8.14 (d,J=8.5Hz,1H), 8.06 (d,J=8.0Hz,1H), 7.96 (d,J=8.0Hz,1H), 7.88-7.82(m,1H), 7.72 (dd,J=11.2,4.0Hz,1H), 7.29 (d,J=7.4Hz,2H), 7.19 (t,J=7.6Hz,2H), 7.09 (d,J=7.5Hz,1H), 4.85 (dd,J=11.2,5.0Hz,1H), 4.62-4.55(m,1H), 4.40-4.30(m,1H), 3.33-3.20(m,3H), 3.06-3.00(m,1H), 2.50-2.41(m,1H), 2.38-2.27(m,2H), 2.06-2.00(m,1H), 1.80 (dt,J=12.9,9.6Hz,2H), 1.30 (d,J=5.1Hz,1H), 0.99 (dd,J=19.0,6.8Hz,6H). ESI-MS m/z 547.25[M+H]⁺.

### Example 12: Synthesis of Compound 12

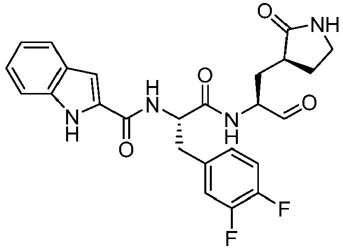

synthetic route:

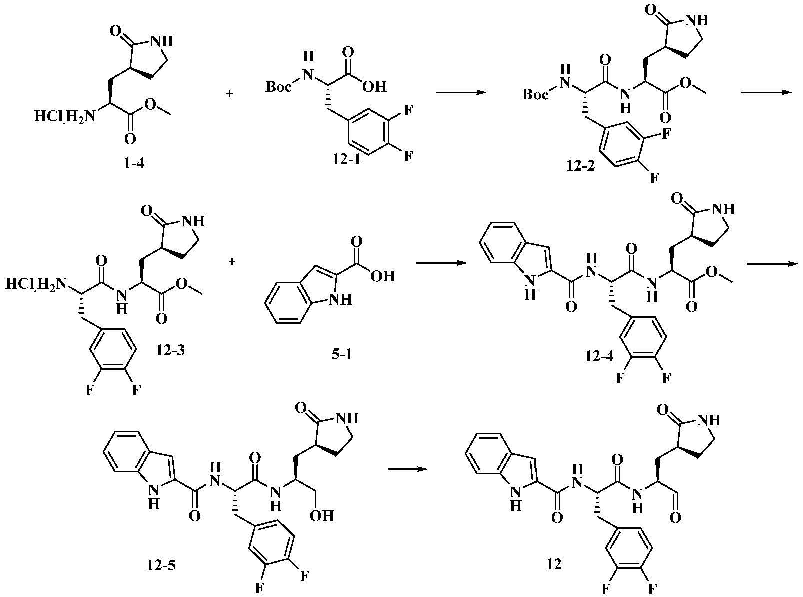

### Synthesis of Compound 12-2:

Compound **12-1** (1.05g,3.5 mmol) was dissolved in dichloromethane (40 mL), the reaction solution was cooled to -20°C, then HATU (1.9 g, 4.9 mmol) was added to the reaction solution. After stirred for twenty minutes, Compound **1-4** was added to the reaction solution, stirred at -20°C for 30 minutes, and then DIPEA (1.7 mL, 10.5 mmol) was added dropwise to the reaction solution. After stirred for 12 hours, the reaction was extracted with ammonium chloride (40×3 mL), sodium bicarbonate (40×3 mL), and sodium chloride (40×3 mL). The combined organic phases was dried with anhydrous sodium sulfate for 1 hour, then distillated under reduced pressure and separated by column chromatography (DCM: CH₃OH, 40:1 v/v) to obtain white powder solid Compound **12-2** 1.3 g, yield 79%.

### Synthesis of Compound 12-3:

Compound **12-2** (1.64 g,3.5 mmol) was dissolved in dichloromethane, 4 M HCl dioxane solution (9 mL, 35 mmol) was added at 0°C, and the reaction was stirred at room temperature for 12 hours. The solution was evaporated to dryness to obtain Compound **12-3** and used directly in the next step.

### Synthesis of Compound 12-4:

Compound **5-1** (0.56 g,3.5 mmol) was dissolved in dichloromethane (40 mL), the reaction solution was cooled to -20°C, then HATU (1.9 g, 4.9 mmol) was added to the reaction solution. After stirred for twenty minutes, Intermediate **12-3** obtained in the previous step was added to the reaction solution, stirred at -20°C for 30 minutes, and then DIPEA (1.7 mL, 10.5 mmol) was added dropwise to the reaction solution. After stirred for 12 hours, the reaction was extracted with ammonium chloride (40×3 mL), sodium bicarbonate (40×3 mL), and sodium chloride (40×3 mL). The combined organic phases was dried with anhydrous sodium sulfate for 1 hour, then distillated under reduced pressure and separated by column chromatography (DCM: CH₃OH, 40:1 v/v) to obtain white powder solid Compound **12-4** 1.4 g, with a yield of 78%.

### Synthesis of Compound 12-5:

Compound **12-4** (261 mg, 0.51 mmol) was dissolved in 20 ml of dichloromethane, sodium borohydride (107 mg, 2.9 mmol) was slowly added in batches, then 1 ml of methanol was added dropwise, and stirred at room temperature for about 2 hours. After the reaction was completed, about 20 ml of saturated brine was added to quench the reaction, and dichloromethane was added for extraction. The organic phase was washed with saturated brine, dried with anhydrous sodium sulfate, and separated by column chromatography (DCM: CH₃OH, 20:1 v/v) to obtain white solid compound **12-5** 173mg, yield 70%

### Synthesis of Compound 12:

Compound **12-5** (140 mg, 0.29 mmol) was dissolved in 20 ml of dichloromethane, and Dess-Martin oxidant (147 mg, 0.35 mmol) was added and stirred at room temperature. After TLC monitoring (UV)showed that the reaction was completed, the reaction solution was extracted with sodium thiosulfate until clearation, then dried with anhydrous sodium sulfate and concentrated. Column chromatography separated (DCM: CH₃OH, 20:1 v/v) to obtain Compound **12** 84 mg, yield 60%.

¹H NMR(600 MHz,Acetone-d6) δ 9.21 (s,1H), 9.11 (s,1H), 8.63 (d,J=9.3Hz,1H), 7.79 (d,J=8.8Hz,1H), 7.66 (d,J=7.7Hz,1H), 7.54 (s,1H), 7.39 (d,J=7.9Hz,1H), 7.25-7.13(m,3H), 7.06(m,2H), 6.55 (t,J=4.4Hz,1H), 5.01 (dt,J=9.3,7.8Hz,1H), 4.40 (dt,J=8.9,6.4Hz,1H), 3.31(m,1H), 3.19(m,1H), 3.07 (dd,J=14.0,7.8Hz,1H), 2.96 (dd,J=13.9,7.7Hz,lH), 2.41(m,1H), 2.16 (dt,J=12.8,6.3Hz,1H), 1.92 (dt,J=12.9,6.4Hz,1H), 1.84-1.70(m,2H). ESI-MS m/z 482.18[M+H]⁺.

### Example 13: Synthesis of Compound 13

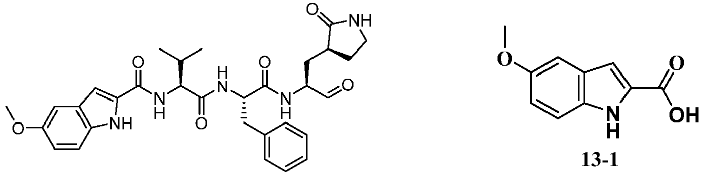

The Compound **13** was synthesized according to the synthesis of Compound **11** by using Compound **13-1** to replace the Compound **11-1** in Example 11, and using Compound **15-1** to replace the **1-5** in Example 1.

¹H NMR(600 MHz,Acetone-d6) δ 9.37 (s,1H), 9.21 (s,1H), 8.47 (d,J=9.1Hz,1H), 7.63 (d,J=8.8Hz,1H), 7.58 (d,J=9.3Hz,1H), 7.50 (d,J=8.4Hz,1H), 7.38 (s,1H), 7.29 (s,1H), 7.28-7.20(m,3H), 7.14-7.09(m,2H), 6.80 (d,J=8.4Hz,1H), 6.55 (t,J=4.4Hz,1H), 4.98 (dd,J=9.2,6.6Hz,1H), 4.74 (dt,J=9.3,7.8Hz,1H), 4.39 (dt,J=8.8,6.4Hz,1H), 3.83 (s,3H), 3.39-3.29(m,2H), 3.27-3.10(m,2H), 2.44(m,1H), 2.26(m,1H), 2.11 (dt,J=12.7,6.3Hz,1H), 1.94 (dt,J=12.9,6.4Hz,1H), 1.83(m,1H), 1.75(m,1H), 0.88 (dd,J=25.0,6.7Hz,6H). ESI-MS m/z 576.27[M+H]⁺.

### Example 14: Synthesis of Compound 14

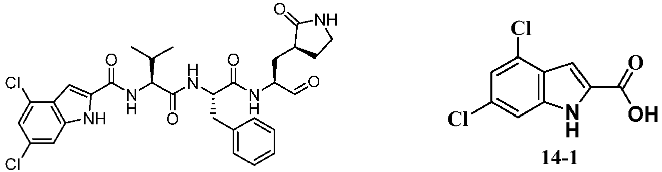

The Compound **14** was synthesized according to the synthesis of Compound **11** by using Compound **14-1** to replace the Compound **11-1** in Example 11.
¹H NMR(600 MHz,Acetone-d6) δ 9.21 (s,1H), 9.04 (s,1H), 8.44 (d,J=9.2Hz,1H), 7.67 (s,1H), 7.63 (dd,J=9.1,3.9Hz,2H), 7.43 (d,J=10.3Hz,2H), 7.28-7.20(m,3H), 7.14-7.08(m,2H), 6.55 (t,J=4.4Hz,1H), 4.93 (dd,J=9.2,6.6Hz,1H), 4.71 (dt,J=9.3,7.7Hz,1H), 4.42 (dt,J=9.0,6.4Hz,1H), 3.38-3.29(m,2H), 3.23-3.11(m,2H), 2.40(m,1H), 2.18(m,2H), 1.97 (dt,J=12.9,6.4Hz,1H), 1.85(m,1H), 1.74(m,1H), 0.90 (dd,J=25.0,6.7Hz,6H). ESI-MS m/z 614.18[M+H]⁺

### Example 15: Synthesis of Compound 15

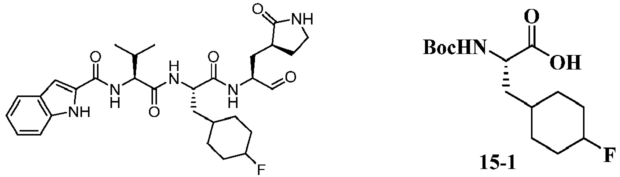

The Compound **15** was synthesized according to the synthesis of Compound **11** by using Compound **5-1** to replace the acid **1-11** in Example 1.
¹H NMR(600 MHz,Acetone-d6) δ 9.30 (s,2H), 9.12 (s,2H), 8.54 (d,J=9.0Hz,2H), 7.64 (dd,J=12.7,8.3Hz,4H), 7.58 (d,J=9.3Hz,2H), 7.42-7.36(m,4H), 7.22 (t,J=7.5Hz,2H), 7.19-7.13(m,2H), 6.55 (t,J=4.4Hz,2H), 4.92 (dd,J=9.1,6.5Hz,2H), 4.68(m,1H), 4.58(m,1H), 4.46-4.33(m,4H), 3.33(m,2H), 3.21(m,2H), 2.41(m,2H), 2.19(m,2H), 2.12 (dd,J=12.9,6.4Hz,2H), 2.01-1.55(m,22H), 1.47-1.38(m,2H), 1.32(m,4H), 0.87 (dd,J=25.0,6.7Hz,12H). ESI-MS m/z 569.30[M+H]⁺

### Example 16: Synthesis of Compound 16

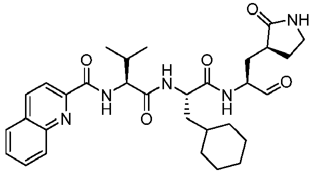

The Compound **16** was synthesized according to the synthesis of Compound **1** by using Compound **11-1** to replace the Compound **1-11** in Example 1.

¹H NMR(600 MHz,Acetone-d6) δ 9.21 (s,1H), 8.61 (d,J=9.2Hz,1H), 8.30 (s,2H), 8.15 (d,J=8.1Hz,1H), 7.95 (d,J=7.5Hz,1H), 7.84 (dd,J=8.2,7.4Hz,1H), 7.72-7.49(m,3H), 6.55 (t,J=4.4Hz,1H), 4.78 (dd,J=9.0,6.6Hz,1H), 4.46-4.28(m,2H), 3.33(m,1H), 3.23(m,1H), 2.54(m,1H), 2.27-2.16(m,J=6.6Hz,1H), 2.11 (dt,J=12.8,6.3Hz,1H), 2.02-1.90(m,3H), 1.89-1.80(m,2H), 1.74(m,1H), 1.62-1.47(m,5H), 1.45-1.24(m,5H), 0.88 (dd,J=25.0,6.7Hz,6H). ESI-MS m/z 564.31[M+H]⁺.

### Example 17: Synthesis of Compound 17

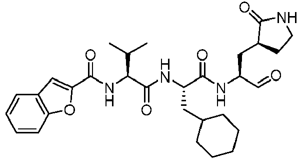

The Compound **17** was synthesized according to the synthesis of Compound **1** by using Compound **10-1** to replace the Compound **1-11** in Example 1.

¹H NMR(600 MHz,Acetone-d6) δ 9.21 (s,1H), 8.32 (d,J=9.2Hz,1H), 7.71 (s,1H), 7.68-7.56(m,4H), 7.39 (t,J=7.7Hz,1H), 7.29 (t,J=7.5Hz,1H), 6.55 (t,J=4.4Hz,1H), 4.82 (dd,J=9.0,6.6Hz,1H), 4.50 (dt,J=9.3,6.9Hz,1H), 4.37 (dt,J=8.8,6.3Hz,1H), 3.32(m,1H), 3.19(m,1H), 2.40(m,1H), 2.22-2.08(m,2H), 1.92 (dt,J=12.9,6.4Hz,1H), 1.88-1.79(m,2H), 1.78-1.68(m,2H), 1.58(m,2H), 1.54-1.37(m,7H), 1.27(m,2H), 0.88 (dd,J=25.0,6.7Hz,6H). ESI-MS m/z 553.29[M+H]⁺.

### Example 18: Synthesis of Compound 18

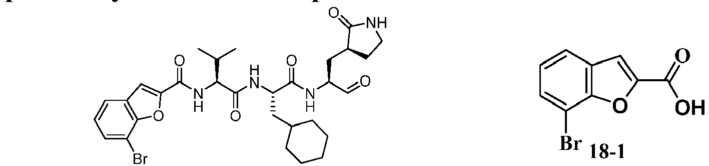

The Compound **18** was synthesized according to the synthesis of Compound **1** by using Compound **18-1** to replace the Compound **1-11** in Example 1.

¹H NMR(600 MHz,Acetone-d6) δ 9.21 (s,1H), 8.44 (d,J=9.2Hz,1H), 7.66-7.58(m,3H), 7.55 (d,J=7.9Hz,1H), 7.46 (d,J=7.9Hz,1H), 7.19 (t,J=7.8Hz,1H), 6.55 (t,J=4.4Hz,1H), 4.85 (dd,J=9.2,6.6Hz,1H), 4.41 (dt,J=9.0,6.3Hz,1H), 4.34 (dt,J=9.3,6.9Hz,1H), 3.33(m,1H), 3.23(m,1H), 2.51(m,1H), 2.26(m,1H), 2.12 (dt,J=12.8,6.3Hz,1H), 1.94 (dt,J=12.9,6.4Hz,1H), 1.86-1.43(m,11H), 1.39-1.17(m,6H), 0.90 (dd,J=25.0,6.7Hz,6H). ESI-MS m/z 631.20[M+H]⁺.

### Example 19: Synthesis of Compound 18

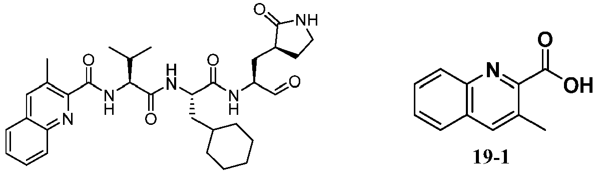

The Compound **19** was synthesized according to the synthesis of Compound **1** by using Compound **19-1** to replace the Compound **1-11** in Example 1.

¹H NMR(600 MHz,Acetone-d6) δ 9.21 (s,1H), 8.36 (d,J=9.0Hz,1H), 8.28 (s,1H), 8.07 (d,J=8.1Hz,1H), 7.81-7.74(m,2H), 7.62 (dd,J=9.2,4.4Hz,2H), 7.52 (t,J=7.7Hz,1H), 6.55 (t,J=4.4Hz,1H), 4.74 (dd,J=9.2,6.6Hz,1H), 4.38 (dt,J=9.3,6.9Hz,1H), 4.29 (dt,J=8.8,6.4Hz,1H), 3.35(m,1H), 3.18(m,1H), 2.49 (s,3H), 2.38(m,1H), 2.29-2.10(m,2H), 1.99-1.90(m,1H), 1.87-1.69(m,4H), 1.66-1.54(m,3H), 1.54-1.34(m,6H), 1.30-1.20(m,2H), 0.88 (dd,J=25.1,6.6Hz,6H). ESI-MS m/z 578.33[M+H]⁺.

### Example 20: Synthesis of Compound 20

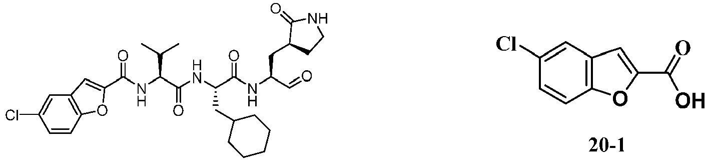

The Compound **20** was synthesized according to the synthesis of Compound **1** by using Compound **20-1** to replace the Compound **1-11** in Example 1.

¹H NMR(600 MHz,Acetone-d6) δ 9.30 (s,1H), 8.31 (d,J=9.0Hz,1H), 7.82 (s,1H), 7.76-7.71(m,2H), 7.60 (dd,J=11.4,9.1Hz,2H), 7.42 (d,J=8.4Hz,1H), 6.55 (t,J=4.4Hz,1H), 5.16 (dd,J=9.0,6.6Hz,1H), 4.48-4.29(m,2H), 3.27(m,2H), 2.41(m,1H), 2.21(m,1H), 2.13 (dt,J=12.8,6.3Hz,1H), 2.00-1.86(m,2H), 1.83(m,1H), 1.78-1.67(m,3H), 1.50(m,2H), 1.47-1.33(m,6H), 1.18(m,2H), 0.88 (dd,J=25.0,6.7Hz,6H). ESI-MS m/z 587.26[M+H]⁺.

### Example 21: Synthesis of Compound 21

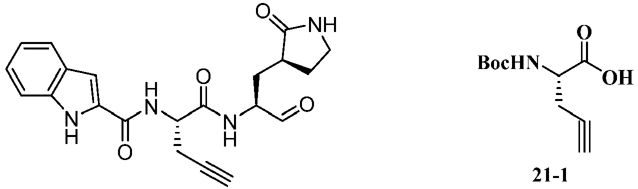

The Compound **21** was synthesized according to the synthesis of Compound **12** by using Compound **21-1** to replace the Compound **12-1** in Example 12.

¹H NMR(500 MHz,Chloroform ) δ 9.72 (s,4H), 8.62 (s,4H), 8.40 (s,4H), 7.98 (s,4H), 7.60 (s,4H), 7.23 (s,4H), 7.10 (s,3H), 6.98 (s,3H), 6.03 (s,4H), 5.82 (s,4H), 5.70 (s,4H), 5.04 (s,2H), 3.65 (s,2H), 3.55 (s,2H), 2.87 (d,J=12.0Hz,5H), 2.61 (s,3H), 2.23 (s,3H), 2.02 (s,4H), 1.93 (d,J=15.1Hz,6H). ESI-MS m/z 395.16[M+H]⁺.

### Example 22: Synthesis of Compound 22

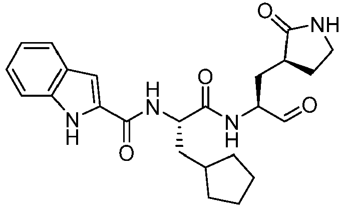

The Compound **22** was synthesized according to the synthesis of Compound **12** by using Compound **3-1** to replace the Compound **12-1** in Example 12

¹H NMR(500 MHz,Chloroform ) δ 9.72 (s,7H), 8.63 (s,7H), 7.98 (s,7H), 7.60 (s,7H), 7.10 (s,6H), 7.00 (d,J=18.2Hz,14H), 6.62 (s,7H), 6.16 (s,7H), 6.02 (s,7H), 5.29 (s,5H), 4.65 (s,4H), 3.65 (s,4H), 3.55 (s,4H), 2.80 (s,4H), 2.22 (s,5H), 2.02 (s,13H), 1.91 (s,3H), 1.76 (s,13H), 1.64 (d,J=15.3Hz,18H), 1.53 (s,3H), 1.33 (s,7H). ESI-MS m/z 439.23[M+H]⁺.

### Example 23: Synthesis of Compound 23

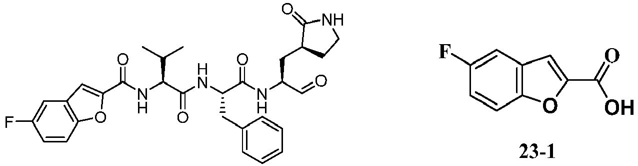

The Compound **23** was synthesized according to the synthesis of Compound **12** by using Compound **8-1** to replace the Compound **12-1** in Example 12, and using Compound **23-1** to replace the **5-1** in Example 1.

¹H NMR(600 MHz,Acetone-d6) δ 9.21 (s,1H), 8.31 (d,J=9.0Hz,1H), 7.75 (s,1H), 7.61(m,3H), 7.41 (d,J=8.1Hz,1H), 7.29-7.19(m,3H), 7.16-7.06(m,3H), 6.55 (t,J=4.4Hz,1H), 4.84 (dt,J=9.3,7.8Hz,1H), 4.68 (dd,J=9.2,6.6Hz,1H), 4.36 (dt,J=9.0,6.4Hz,1H), 3.38-3.27(m,2H), 3.22-3.10(m,2H), 2.39(m,1H), 2.14 (dt,J=12.8,6.3Hz,1H), 2.05(m,1H), 1.91 (dt,J=12.7,6.3Hz,1H), 1.83(m,1H), 1.74(m,1H), 0.88 (dd,J=25.0,6.7Hz,6H).ESI-MS m/z 564.24[M+H]⁺.

### Example 24: Synthesis of Compound 24

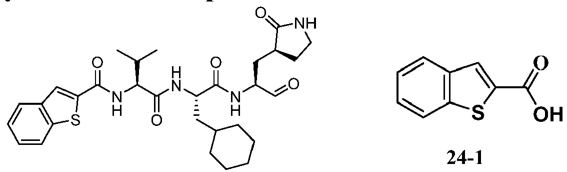

The Compound **24** was synthesized according to the synthesis of Compound **1** by using Compound **24-1** to replace the Compound **1-11** in Example 1.

¹H NMR(600 MHz,Acetone-d6) δ 9.30 (s,1H), 8.30 (s,1H), 8.24 (d,J=9.0Hz,1H), 7.99-7.93(m,1H), 7.80-7.75(m,1H), 7.63 (d,J=9.0Hz,1H), 7.58 (d,J=9.3Hz,1H), 7.37-7.32(m,2H), 6.55 (t,J=4.4Hz,1H), 5.16 (dd,J=9.0,6.6Hz,1H), 4.39(m,2H), 3.33(m,1H), 3.21(m,1H), 2.42(m,1H), 2.21-2.08(m,2H), 1.99-1.87(m,2H), 1.83(m,1H), 1.78-1.66(m,3H), 1.52(m,2H), 1.47-1.28(m,6H), 1.20(m,2H), 0.86 (dd,J=25.1,6.6Hz,6H). ESI-MS m/z 569.27[M+H]⁺.

### Example 25: Synthesis of Compound 25

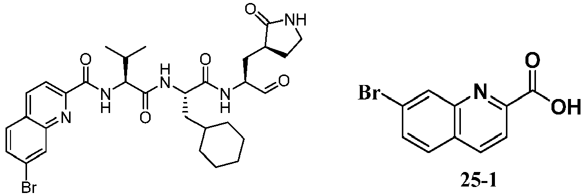

The Compound **25** was synthesized according to the synthesis of Compound **1** by using Compound **25-1** to replace the Compound **1-11** in Example 1.

### Example 26: Synthesis of Compound 26

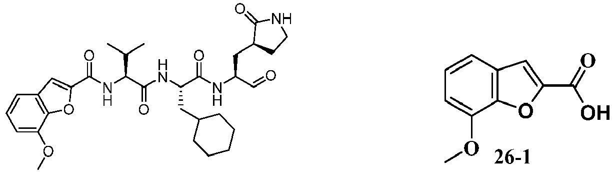

The Compound **26** was synthesized according to the synthesis of Compound **1** by using Compound **26-1** to replace the Compound **1-11** in Example 1.

¹H NMR(600 MHz,Acetone-d6) δ 9.21 (s,1H), 8.60 (d,J=9.0Hz,1H), 8.30-8.19(m,3H), 7.90 (d,J=8.4Hz,1H), 7.70 (d,J=8.4Hz,1H), 7.63 (dd,J=9.2,4.9Hz,2H), 6.55 (t,J=4.4Hz,1H), 4.90 (dd,J=9.1,6.5Hz,1H), 4.41 (dt,J=9.0,6.4Hz,1H), 4.30 (dt,J=9.3,6.9Hz,1H), 3.34(m,1H), 3.23(m,1H), 2.47(m,1H), 2.26-2.16(m,J=6.6Hz,1H), 2.12 (dt,J=12.9,6.4Hz,1H), 1.94 (dt,J=12.9,6.4Hz,1H), 1.90-1.78(m,3H), 1.74(m,1H), 1.68-1.57(m,1H), 1.54-1.41(m,4H), 1.41-1.34(m,4H), 1.22(m,2H), 0.90 (dd,J=25.0,6.7Hz,6H). ESI-MS m/z 642.22[M+H]⁺.

### Example 27: Synthesis of Compound 27

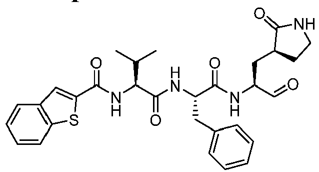

The Compound **27** was synthesized according to the synthesis of Compound **1** by using Compound **24-1** to replace the Compound **1-11** in Example 1, and using Compound **8-1** to replace the **1-5** in Example 1.

¹H NMR(600 MHz,Acetone-d6) δ 9.21 (s,1H), 8.34 (s,1H), 8.20 (d,J=9.2Hz,1H), 7.98-7.93(m,1H), 7.80-7.75(m,1H), 7.61 (dd,J=19.5,9.1Hz,2H), 7.38-7.32(m,2H), 7.29-7.20(m,3H), 7.15-7.08(m,2H), 6.55 (t,J=4.4Hz,1H), 5.14 (dd,J=9.1,6.5Hz,1H), 4.74 (dt,J=9.3,7.7Hz,1H), 4.39 (dt,J=8.8,6.4Hz,1H), 3.42-3.26(m,2H), 3.24-3.07(m,2H), 2.34(m,1H), 2.20-2.06(m,2H), 1.94 (dt,J=12.7,6.3Hz,1H), 1.82(m,1H), 1.74(m,1H), 0.86 (dd,J=25.1,6.6Hz,6H). ESI-MS m/z 562.22[M+H]⁺.

### Example 28: Synthesis of Compound 28

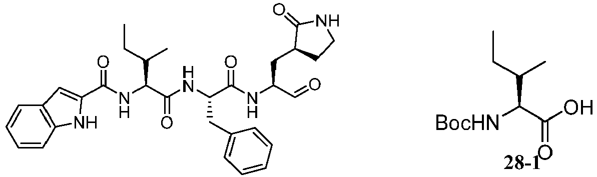

The Compound **28** was synthesized according to the synthesis of Compound **9** by using Compound **28-1** to replace the Compound **1-8** in Example 9.

¹H NMR(600 MHz,Acetone-d6) δ 9.21 (s,1H), 9.16 (s,1H), 8.46 (d,J=9.2Hz,1H), 7.68-7.56(m,3H), 7.39 (d,J=7.9Hz,1H), 7.32 (s,1H), 7.29-7.14(m,5H), 7.14-7.08(m,2H), 6.55 (t,J=4.4Hz,1H), 4.90 (dd,J=9.1,6.5Hz,1H), 4.72 (dt,J=9.3,7.8Hz,1H), 4.44 (dt,J=9.0,6.3Hz,1H), 3.40-3.30(m,2H), 3.21(m,1H), 3.12 (dd,J=14.0,7.8Hz,1H), 2.43(m,1H), 2.17 (dt,J=12.8,6.3Hz,1H), 1.97-1.80(m,3H), 1.79-1.61(m,2H), 1.46(m,1H), 0.90-0.83(m,6H). ESI-MS m/z 559.28[M+H]⁺.

### Example 29: Synthesis of Compound 29

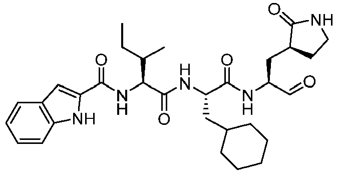

The Compound **29** was synthesized according to the synthesis of Compound **5** by using Compound **28-1** to replace the Compound **1-8** in Example 5.

¹H NMR(600 MHz,Acetone-d6) δ 9.30 (s,1H), 9.16 (s,1H), 8.46 (d,J=9.2Hz,1H), 7.68-7.60(m,3H), 7.44 (s,1H), 7.39 (d,J=7.9Hz,1H), 7.22 (t,J=7.5Hz,1H), 7.19-7.14(m,1H), 6.55 (t,J=4.4Hz,1H), 4.83 (dd,J=9.0,6.5Hz,1H), 4.41 (dt,J=8.9,6.4Hz,1H), 4.30 (dt,J=9.3,6.9Hz,1H), 3.38-3.10(m,2H), 2.42(m,1H), 2.08 (dt,J=12.9,6.4Hz,1H), 2.00-1.91(m,2H), 1.86-1.62(m,5H), 1.60-1.42(m,7H), 1.35-1.20(m,5H), 0.91-0.84(m,6H). ESI-MS m/z 565.33[M+H]⁺.

### Example 30: Synthesis of Compound 30

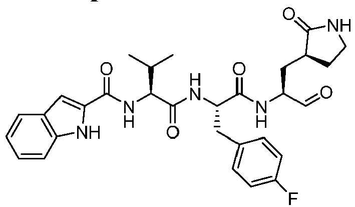

The Compound **30** was synthesized according to the synthesis of Compound **1** by using Compound **5-1** to replace the acid **1-11** in Example 1, and using Compound **4-1** to replace the **1-5** in Example 1.

¹H NMR(600 MHz,Acetone-d6) δ 9.21 (s,1H), 9.12 (s,1H), 8.48 (d,J=9.0Hz,1H), 7.68-7.56(m,3H), 7.45 (s,1H), 7.39 (d,J=7.9Hz,1H), 7.31-7.13(m,4H), 7.02-6.95(m,2H), 6.55 (t,J=4.4Hz,1H), 4.99 (dd,J=9.2,6.6Hz,1H), 4.75 (dt,J=9.2,7.7Hz,1H), 4.39 (dt,J=8.8,6.4Hz,1H), 3.33(m,1H), 3.22(m,1H), 3.04 (dd,J=14.1,7.7Hz,1H), 2.92 (dd,J=14.0,7.8Hz,1H), 2.44(m,1H), 2.33-2.18(m,J=6.6Hz,1H), 2.12 (dt,J=12.8,6.3Hz,1H), 1.94 (dt,J=12.9,6.3Hz,1H), 1.83(m,1H), 1.74(m,1H), 0.88 (dd,J=25.0,6.7Hz,6H). ESI-MS m/z 563.25[M+H]⁺.

### Example 31: Synthesis of Compound 31

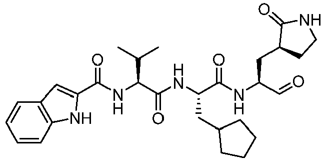

The Compound **31** was synthesized according to the synthesis of Compound **1** by using Compound **5-1** to replace the acid **1-11** in Example 1, and using Compound **3-1** to replace the **1-5** in Example 1.

¹H NMR(600 MHz,Acetone-d6) δ 9.21 (s,1H), 9.12 (s,1H), 8.55 (d,J=9.2Hz,1H), 7.71-7.61(m,3H), 7.39 (d,J=7.9Hz,1H), 7.32 (s,1H), 7.24-7.14(m,2H), 6.55 (t,J=4.4Hz,1H), 5.02 (dd,J=9.1,6.5Hz,1H), 4.41 (dt,J=9.0,6.4Hz,1H), 4.30 (dt,J=9.3,6.9Hz,1H), 3.34(m,1H), 3.23(m,1H), 2.47(m,1H), 2.27-2.07(m,2H), 1.95 (dt,J=12.9,6.4Hz,1H), 1.88-1.79(m,3H), 1.78-1.63(m,4H), 1.59-1.48(m,5H), 0.87 (dd,J=25.0,6.7Hz,6H). ESI-MS m/z 537.29[M+H]⁺.

### Example 32: Synthesis of Compound 32

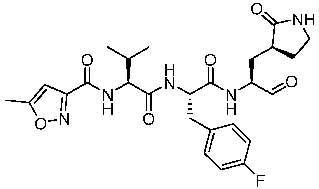

The Compound **32** was synthesized according to the synthesis of Compound **1** by using Compound **6-1** to replace the acid **1-11** in Example 1, and using Compound **4-1** to replace the **1-5** in Example 1.

¹H NMR(600 MHz,Acetone-d6) δ 9.21 (s,1H), 8.17 (d,J=9.0Hz,1H), 7.98 (d,J=9.3Hz,1H), 7.72 (d,J=8.8Hz,1H), 7.28 (dd,J=8.4,4.9Hz,2H), 7.03 (t,J=8.2Hz,2H), 6.58-6.52(m,2H), 4.75 (dd,J=9.0,6.6Hz,1H), 4.58-4.44(m,2H), 3.30(m,1H), 3.18(m,1H), 3.09 (dd,J=13.9,7.7Hz,1H), 2.92 (dd,J=14.1,7.7Hz,1H), 2.55-2.47(m,4H), 2.24-2.11(m,J=6.6Hz,1H), 2.01 (dt,J=12.8,6.3Hz,1H), 1.87-1.76(m,2H), 1.73(m,1H), 0.88 (dd,J=25.0,6.7Hz,6H). ESI-MS m/z 529.23[M+H]⁺.

### Example 33: Synthesis of Compound 33

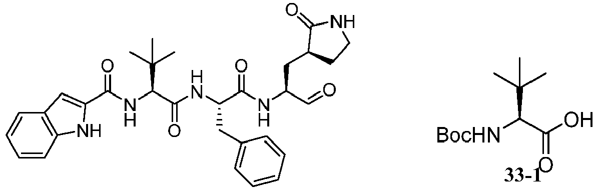

The Compound **33** was synthesized according to the synthesis of Compound **9** by using Compound **33-1** to replace the Compound **1-8** in Example 9.

¹H NMR(600 MHz,Acetone-d6) δ 9.30 (s,1H), 9.12 (s,1H), 8.49 (d,J=8.8Hz,1H), 7.81 (d,J=9.3Hz,1H), 7.69-7.60(m,2H), 7.41 (d,J=7.9Hz,1H), 7.32-7.08(m,8H), 6.55 (t,J=4.4Hz,1H), 4.79 (dt,J=9.3,7.7Hz,1H), 4.48 (dt,J=9.0,6.3Hz,1H), 4.42 (d,J=8.8Hz,1H), 3.37-3.30(m,2H), 3.24-3.11(m,2H), 2.43(m,1H), 2.24 (dt,J=12.7,6.3Hz,1H), 1.99 (dt,J=12.9,6.3Hz,1H), 1.87-1.71(m,2H), 0.98 (s,9H). ESI-MS m/z 559.28[M+H]⁺.

### Example 34: Synthesis of Compound 34

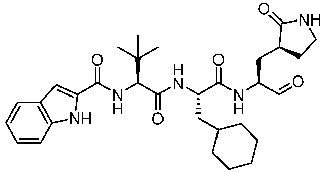

The Compound **34** was synthesized according to the synthesis of Compound **9** by using Compound **33-1** to replace the Compound **1-8** in Example 5.

¹H NMR(600 MHz,Acetone-d6) δ 9.30 (s,1H), 9.12 (s,1H), 8.53 (d,J=8.8Hz,1H), 7.69-7.55(m,3H), 7.39 (d,J=7.9Hz,1H), 7.35 (s,1H), 7.22 (t,J=7.5Hz,1H), 7.19-7.12(m,1H), 6.55 (t,J=4.4Hz,1H), 4.61-4.55(m,2H), 4.33 (dt,J=9.3,6.8Hz,1H), 3.34(m,1H), 3.22(m,1H), 2.46(m,1H), 2.06 (dt,J=12.8,6.3Hz,1H), 1.98-1.79(m,3H), 1.78-1.58(m,5H), 1.57-1.39(m,3H), 1.34-1.17(m,5H), 0.98 (s,9H). ESI-MS m/z 565.33[M+H]⁺.

### Example 35: Synthesis of Compound 35

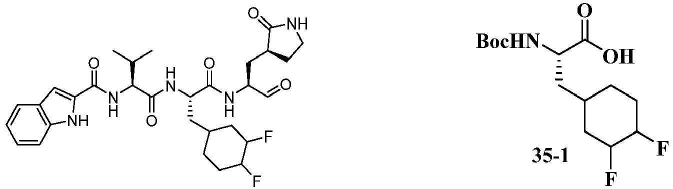

The Compound **35** was synthesized according to the synthesis of Compound **1** by using Compound **5-1** to replace the acid **1-11** in Example 1, and using Compound **35-1** to replace the **1-5** in Example 1.

¹H NMR(600 MHz,Acetone-d6) δ 9.21 (s,1H), 9.12 (s,1H), 8.54 (d,J=9.0Hz,1H), 7.67-7.56(m,3H), 7.42-7.36(m,2H), 7.23 (t,J=7.6Hz,1H), 7.16 (dd,J=8.0,7.4Hz,1H), 6.55 (t,J=4.4Hz,1H), 4.89 (dd,J=9.0,6.6Hz,1H), 4.74-4.52(m,2H), 4.52-4.43(m,1H), 4.33 (dt,J=8.8,6.4Hz,1H), 3.33(m,1H), 3.19(m,1H), 2.40(m,1H), 2.20(m,1H), 2.15-2.09(m,1H), 2.00-1.56(m,12H), 0.87 (dd,J=25.0,6.7Hz,6H). ESI-MS m/z 587.29[M+H]⁺.

### Example 36: Synthesis of Compound 36

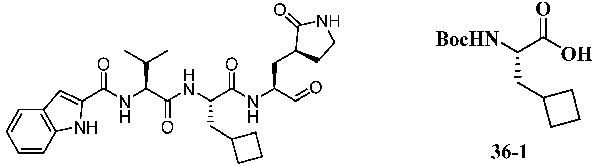

The Compound **36** was synthesized according to the synthesis of Compound **35** by using Compound **36-1** to replace the Compound **35-1** in Example 35.

¹H NMR(600 MHz,Acetone-d6) δ 9.21 (s,1H), 9.07 (s,1H), 8.54 (d,J=9.2Hz,1H), 7.73-7.62(m,3H), 7.39 (d,J=7.9Hz,1H), 7.32 (s,1H), 7.25-7.14(m,2H), 6.55 (s,1H), 4.99 (dd,J=9.2,6.6Hz,1H), 4.46 (dt,J=8.8,6.4Hz,1H), 4.42-4.32(m,1H), 3.38-3.18(m,2H), 2.45 (t,J=6.4Hz,1H), 2.25-2.15(m,J=6.7Hz,1H), 2.10 (dt,J=12.9,6.4Hz,1H), 2.01-1.91(m,2H), 1.89-1.55(m,10H), 0.87 (dd,J=25.0,6.7Hz,6H). ESI-MS m/z 523.28[M+H]⁺.

### Example 37: Synthesis of Compound 37

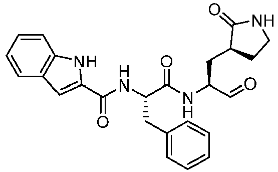

The Compound **37** was synthesized according to the synthesis of Compound **12** by using Compound **8-1** to replace the Compound **12-1** in Example 12.

¹H NMR(600 MHz,Acetone-d6) δ 9.21 (s,1H), 9.11 (s,1H), 8.53 (d,J=9.3Hz,1H), 7.80 (d,J=9.0Hz,1H), 7.67 (d,J=7.7Hz,1H), 7.39 (d,J=7.9Hz,1H), 7.36 (s,1H), 7.29-7.12(m,7H), 6.55 (t,J=4.4Hz,1H), 5.04 (dt,J=9.3,7.7Hz,1H), 4.36 (dt,J=8.8,6.4Hz,1H), 3.25(m,1H), 3.15(m,1H), 3.11-3.01(m,2H), 2.41(m,1H), 2.18 (dt,J=12.8,6.3Hz,1H), 1.87 (dt,J=12.9,6.4Hz,1H), 1.82-1.68(m,2H). ESI-MS m/z 446.19[M+H]⁺.

### Example 38: Synthesis of Compound 38

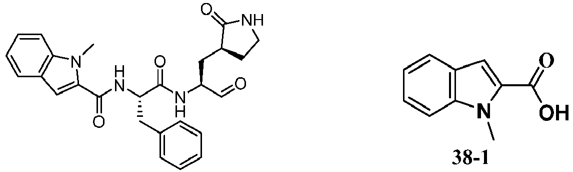

The Compound **38** was synthesized according to the synthesis of Compound **12** by using Compound **38-1** to replace the Compound **5-1** in Example 37.

¹H NMR(600 MHz,Acetone-d6) δ 9.21 (s,1H), 8.56 (d,J=9.3Hz,1H), 7.77 (d,J=9.0Hz,1H), 7.68 (d,J=7.8Hz,1H), 7.37 (d,J=8.6Hz,2H), 7.30 (dd,J=8.2,7.3Hz,1H), 7.27-7.18(m,6H), 6.55 (t,J=4.4Hz,1H), 4.97 (dt,J=9.3,7.8Hz,1H), 4.60 (dt,J=8.8,6.4Hz,1H), 3.94 (s,3H), 3.33(m,1H), 3.16(m,1H), 3.10 (dd,J=13.9,7.7Hz,1H), 3.00 (dd,J=14.1,7.7Hz,1H), 2.37(m,1H), 2.13 (dt,J=12.9,6.4Hz,1H), 1.88 (dt,J=12.9,6.4Hz,1H), 1.82-1.69(m,2H). ESI-MS m/z 460.21[M+H]⁺.

### Example 39: Synthesis of Compound 39

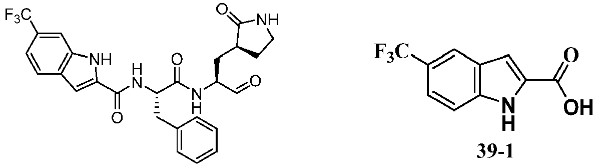

The Compound **39** was synthesized according to the synthesis of Compound **12** by using Compound **39-1** to replace the Compound **5-1** in Example 37.

¹H NMR(600 MHz,Acetone-d6) δ 9.30 (s,1H), 9.14 (s,1H), 8.53 (d,J=9.3Hz,1H), 7.86 (s,1H), 7.82 (d,J=8.4Hz,1H), 7.74 (d,J=8.8Hz,1H), 7.52 (s,1H), 7.46 (d,J=8.4Hz,1H), 7.27-7.18(m,5H), 6.55 (t,J=4.4Hz,1H), 4.91 (dt,J=9.3,7.7Hz,1H), 4.53 (dt,J=9.0,6.3Hz,1H), 3.37(m,1H), 3.22(m,1H), 3.09 (dd,J=14.0,7.8Hz,1H), 3.00 (dd,J=13.9,7.7Hz,1H), 2.44(m,1H), 2.17 (dt,J=12.9,6.4Hz,1H), 1.97 (dt,J=12.9,6.4Hz,1H), 1.84(m,1H), 1.76(m,1H). ESI-MS m/z 514.18[M+H]⁺.

### Example 40: Synthesis of Compound 40

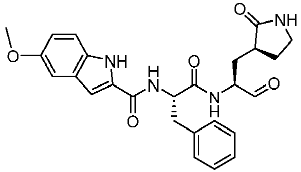

The Compound **40** was synthesized according to the synthesis of Compound **12** by using Compound **13-1** to replace the Compound **5-1** in Example 37.

¹H NMR(600 MHz,Acetone-d6) δ 9.21 (s,1H), 9.18 (s,1H), 8.51 (d,J=9.3Hz,1H), 7.80 (d,J=8.8Hz,1H), 7.51 (d,J=8.4Hz,1H), 7.33 (s,1H), 7.30 (s,1H), 7.28-7.18(m,5H), 6.80 (d,J=8.4Hz,1H), 6.55 (t,J=4.4Hz,1H), 5.05 (dt,J=9.3,7.8Hz,1H), 4.34 (dt,J=9.0,6.3Hz,1H), 3.84 (s,3H), 3.25(m,1H), 3.19-3.11(m,1H), 3.11-3.07(m,1H), 3.00 (dd,J=14.1,7.7Hz,1H), 2.41(m,1H), 2.18 (dt,J=12.9,6.4Hz,1H), 1.86 (dt,J=12.9,6.4Hz,1H), 1.82-1.68(m,2H). ESI-MS m/z 476.21[M+H]⁺.

### Example 41: Synthesis of Compound 41

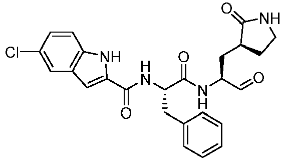

The Compound **41** was synthesized according to the synthesis of Compound **37** by using Compound **20-1** to replace the Compound **5-1** in Example 37.

¹H NMR(600 MHz,Acetone-d6) δ 9.30 (s,1H), 9.15 (s,1H), 8.53 (d,J=9.3Hz,1H), 7.80 (d,J=8.8Hz,1H), 7.61 (s,1H), 7.39 (d,J=8.4Hz,1H), 7.32-7.07(m,7H), 6.55 (t,J=4.4Hz,1H), 5.06 (dt,J=9.3,7.8Hz,1H), 4.48 (dt,J=8.8,6.4Hz,1H), 3.36(m,1H), 3.22(m,1H), 3.09 (dd,J=14.1,7.7Hz,1H), 3.00 (dd,J=13.9,7.7Hz,1H), 2.43(m,1H), 2.14 (dt,J=12.9,6.4Hz,1H), 1.99 (dt,J=12.9,6.3Hz,1H), 1.85-1.71(m,2H). ESI-MS m/z 480.16[M+H]⁺.

### Example 42: Synthesis of Compound 42

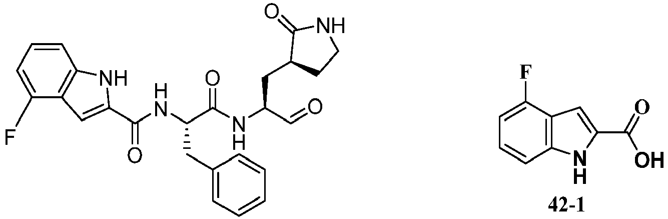

The Compound **42** was synthesized according to the synthesis of Compound **37** by using Compound **42-1** to replace the Compound **5-1** in Example 37.

¹H NMR(600 MHz,Acetone-d6) δ 9.30 (s,1H), 9.13 (s,1H), 8.61 (d,J=9.3Hz,1H), 7.83 (d,J=4.9Hz,1H), 7.77 (d,J=9.0Hz,1H), 7.33 (d,J=7.8Hz,1H), 7.29-7.19(m,4H), 7.16-7.05(m,3H), 6.55 (t,J=4.4Hz,1H), 5.01 (dt,J=9.3,7.7Hz,1H), 4.49 (dt,J=9.0,6.4Hz,1H), 3.33(m,1H), 3.21-3.00(m,3H), 2.33(m,1H), 2.11 (dt,J=12.9,6.4Hz,1H), 1.99 (dt,J=12.9,6.3Hz,1H), 1.84-1.71(m,2H). ESI-MS m/z 464.19[M+H]⁺.

### Example 43: Synthesis of Compound 43

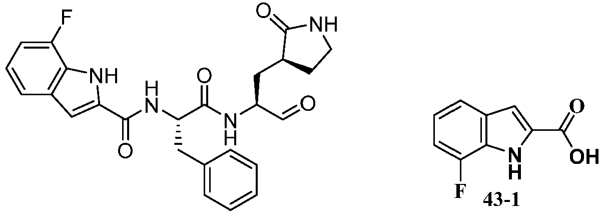

The Compound **43** was synthesized according to the synthesis of Compound **37** by using Compound **43-1** to replace the Compound **5-1** in Example 37.

¹H NMR(600 MHz,Acetone-d6) δ 9.77 (s,1H), 9.21 (s,1H), 8.61 (d,J=9.3Hz,1H), 7.77 (d,J=9.0Hz,1H), 7.59 (d,J=7.9Hz,1H), 7.39 (s,1H), 7.32 (td,J=7.9,4.9Hz,1H), 7.29-7.18(m,5H), 7.01 (t,J=8.0Hz,1H), 6.55 (t,J=4.4Hz,1H), 5.07 (dt,J=9.3,7.7Hz,1H), 4.38 (dt,J=8.8,6.4Hz,1H), 3.37(m,1H), 3.22(m,1H), 3.10 (dd,J=14.0,7.8Hz,1H), 3.00 (dd,J=13.9,7.7Hz,1H), 2.44(m,1H), 2.20 (dt,J=12.8,6.3Hz,1H), 1.93 (dt,J=12.9,6.4Hz,1H), 1.85-1.70(m,2H). ESI-MS m/z 464.19[M+H]⁺.

### Example 44: Synthesis of Compound 44

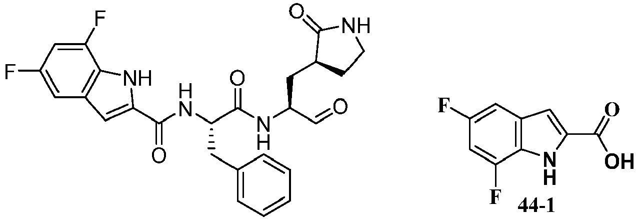

The Compound **44** was synthesized according to the synthesis of Compound **37** by using Compound **44-1** to replace the Compound **5-1** in Example 37.

¹H NMR(600 MHz,Acetone-d6) δ 9.76 (s,1H), 9.21 (s,1H), 8.58 (d,J=9.3Hz,1H), 7.74 (d,J=8.8Hz,1H), 7.44-7.36(m,2H), 7.28-7.18(m,5H), 6.90 (t,J=8.1Hz,1H), 6.55 (t,J=4.4Hz,1H), 5.06 (dt,J=9.3,7.7Hz,1H), 4.41 (dt,J=9.0,6.3Hz,1H), 3.37(m,1H), 3.22(m,1H), 3.10 (dd,J=13.9,7.7Hz,1H), 3.00 (dd,J=14.1,7.7Hz,1H), 2.44(m,1H), 2.19 (dt,J=12.9,6.4Hz,1H), 1.92 (dt,J=12.8,6.3Hz,1H), 1.85-1.70(m,2H). ESI-MS m/z 482.18[M+H]⁺.

### Example 45: Synthesis of Compound 45

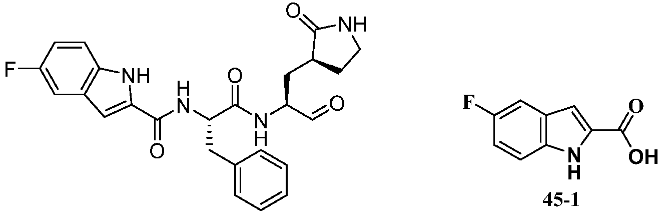

The Compound **45** was synthesized according to the synthesis of Compound **37** by using Compound **45-1** to replace the Compound **5-1** in Example 37.

¹H NMR(600 MHz,Acetone-d6) δ 9.30 (s,1H), 9.11 (s,1H), 8.51 (d,J=9.3Hz,1H), 7.82 (d,J=9.0Hz,1H), 7.57 (d,J=7.9Hz,1H), 7.50 (dd,J=8.4,4.9Hz,1H), 7.36 (s,1H), 7.29-7.18(m,3H), 7.13 (dd,J=6.1,1.1Hz,2H), 7.03 (t,J=8.2Hz,1H), 6.55 (t,J=4.4Hz,1H), 5.06 (dt,J=9.3,7.8Hz,1H), 4.48 (dt,J=9.0,6.4Hz,1H), 3.36(m,1H), 3.22(m,1H), 3.09 (dd,J=14.1,7.7Hz,1H), 3.00 (dd,J=13.9,7.7Hz,1H), 2.44(m,1H), 2.14 (dt,J=12.9,6.3Hz,1H), 1.99 (dt,J=12.8,6.3Hz,1H), 1.85-1.70(m,2H). ESI-MS m/z 464.19[M+H]⁺.

### Example 46: Synthesis of Compound 46

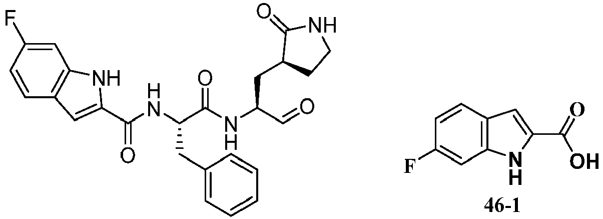

The Compound **46** was synthesized according to the synthesis of Compound **37** by using Compound **46-1** to replace the Compound **5-1** in Example 37.

¹H NMR(600 MHz,Acetone-d6) δ 9.30 (s,1H), 9.23 (s,1H), 8.51 (d,J=9.3Hz,1H), 7.93 (dd,J=8.4,4.9Hz,1H), 7.82 (d,J=9.0Hz,1H), 7.45 (s,1H), 7.29-7.22(m,3H), 7.22-7.16(m,1H), 7.16-7.06(m,3H), 6.55 (t,J=4.4Hz,1H), 5.07 (dt,J=9.3,7.8Hz,1H), 4.48 (dt,J=9.0,6.4Hz,1H), 3.36(m,1H), 3.22(m,1H), 3.10 (dd,J=13.9,7.7Hz,1H), 3.00 (dd,J=14.1,7.7Hz,1H), 2.44(m,1H), 2.15 (dt,J=12.8,6.3Hz,1H), 1.99 (dt,J=12.9,6.3Hz,1H), 1.85-1.70(m,2H). ESI-MS m/z 464.19[M+H]⁺.

### Example 47: Synthesis of Compound 47

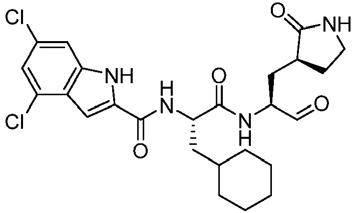

The Compound **47** was synthesized according to the synthesis of Compound **37** by using Compound **14-1** to replace the Compound **5-1** in Example 37, and using **1-5** to replace the compound **8-1.**

¹H NMR(600 MHz,Acetone-d6) δ 9.30 (s,1H), 9.17 (s,1H), 8.57 (d,J=9.3Hz,1H), 7.81 (s,1H), 7.74 (d,J=8.8Hz,1H), 7.41 (s,1H), 7.31 (s,1H), 6.55 (t,J=4.4Hz,1H), 4.51 (dt,J=8.8,6.4Hz,1H), 4.43 (dt,J=9.3,6.9Hz,1H), 3.36(m,1H), 3.21(m,1H), 2.43(m,1H), 2.16 (dt,J=12.8,6.3Hz,1H), 1.97 (dt,J=12.8,6.3Hz,1H), 1.88-1.66(m,5H), 1.64-1.44(m,5H), 1.41-1.20(m,5H). ESI-MS m/z 520.16[M+H]⁺.

### Example 48: Synthesis of Compound 48

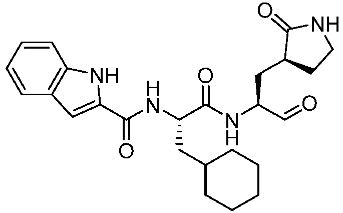

The Compound **48** was synthesized according to the synthesis of Compound **37** by using Compound **5-1** to replace the Compound **5-1** in Example 37, and using **1-5** to replace the Compound **8-1.**

¹H NMR(600 MHz,Acetone-d6) δ 9.30 (s,1H), 9.11 (s,1H), 8.58 (d,J=9.2Hz,1H), 7.80 (d,J=8.8Hz,1H), 7.67 (d,J=7.6Hz,1H), 7.44 (s,1H), 7.39 (d,J=7.9Hz,1H), 7.25-7.13(m,2H), 6.55 (t,J=4.4Hz,1H), 4.48(m,2H), 3.35(m,1H), 3.20(m,1H), 2.42(m,1H), 2.15 (dt,J=12.9,6.4Hz,1H), 2.02-1.89(m,2H), 1.85-1.44(m,9H), 1.39-1.20(m,5H). ESI-MS m/z 452.24[M+H]⁺.

### Example 49: Synthesis of Compound 49

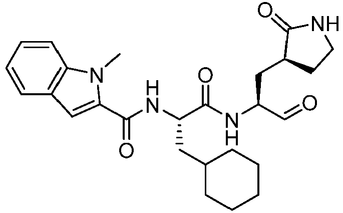

The Compound **49** was synthesized according to the synthesis of Compound **47** by using Compound **38-1** to replace the Compound **14-1** in Example 47.

¹H NMR(600 MHz,Acetone-d6) δ 9.21 (s,1H), 8.62 (d,J=9.3Hz,1H), 7.76 (d,J=8.8Hz,1H), 7.68 (d,J=7.8Hz,1H), 7.37 (d,J=8.3Hz,1H), 7.33-7.26(m,2H), 7.20 (t,J=7.6Hz,1H), 6.55 (t,J=4.4Hz,1H), 4.33(m,2H), 3.94 (s,3H), 3.24(m,1H), 3.13(m,1H), 2.39(m,1H), 2.18 (dt,J=12.9,6.3Hz,1H), 1.98 (dt,J=13.8,6.9Hz,1H), 1.91-1.84(m,2H), 1.84-1.74(m,2H), 1.74-1.67(m,1H), 1.67-1.44(m,7H), 1.39-1.21(m,5H). ESI-MS m/z 466.26[M+H]⁺.

### Example 50: Synthesis of Compound 50

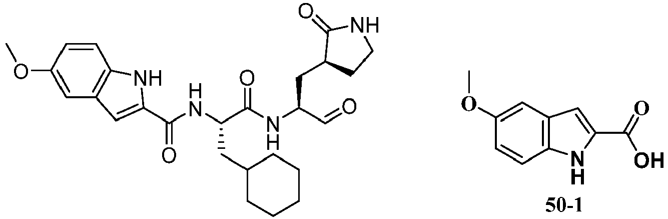

The Compound **50** was synthesized according to the synthesis of Compound **47** by using Compound **50-1** to replace the Compound **14-1** in Example 47.

¹H NMR(600 MHz,Acetone-d6) δ 9.30 (s,1H), 9.15 (s,1H), 8.58 (d,J=9.3Hz,1H), 7.79 (d,J=8.8Hz,1H), 7.40 (d,J=8.9Hz,2H), 7.27 (s,1H), 6.80 (d,J=8.4Hz,1H), 6.55 (t,J=4.4Hz,1H), 4.55 (dt,J=9.0,6.4Hz,1H), 4.42 (dt,J=9.3,6.9Hz,1H), 3.84 (s,3H), 3.35(m,1H), 3.20(m,1H), 2.46(m,1H), 2.13 (dt,J=12.8,6.4Hz,1H), 1.98 (dt,J=12.9,6.4Hz,1H), 1.86-1.65(m,5H), 1.64-1.53(m,2H), 1.53-1.43(m,3H), 1.40-1.20(m,5H). ESI-MS m/z 482.25[M+H]⁺.

### Example 51: Synthesis of Compound 51

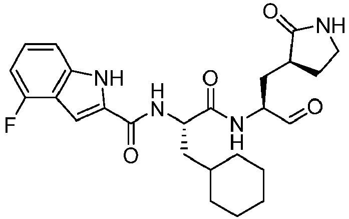

The Compound **51** was synthesized according to the synthesis of Compound **47** by using Compound **42-1** to replace the Compound **14-1** in Example 47.

¹H NMR(600 MHz,Acetone-d6) δ 9.30 (s,1H), 9.13 (s,1H), 8.63 (d,J=9.3Hz,1H), 7.80-7.71(m,2H), 7.27 (d,J=7.8Hz,1H), 7.21 (td,J=7.8,4.9Hz,1H), 7.09 (t,J=7.8Hz,1H), 6.55 (t,J=4.4Hz,1H), 4.51 (dt,J=9.0,6.4Hz,1H), 4.43 (dt,J=9.3,6.9Hz,1H), 3.36(m,1H), 3.21(m,1H), 2.42(m,1H), 2.16 (dt,J=12.9,6.4Hz,1H), 2.00 (dt,J=12.9,6.3Hz,1H), 1.86-1.66(m,5H), 1.64-1.44(m,5H), 1.41-1.20(m,5H). ESI-MS m/z 470.23[M+H]⁺.

### Example 52: Synthesis of Compound 52

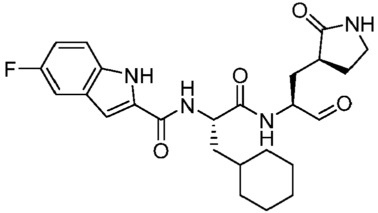

The Compound **52** was synthesized according to the synthesis of Compound **47** by using Compound **45-1** to replace the Compound **14-1** in Example 47.

¹H NMR(600 MHz,Acetone-d6) δ 9.30 (s,1H), 9.11 (s,1H), 8.54 (d,J=9.2Hz,1H), 7.79 (d,J=8.8Hz,1H), 7.53 (d,J=7.9Hz,1H), 7.47 (dd,J=8.4,4.9Hz,1H), 7.37 (s,1H), 7.03 (t,J=8.2Hz,1H), 6.55 (t,J=4.4Hz,1H), 4.52 (dt,J=9.0,6.4Hz,1H), 4.45 (dt,J=9.3,6.9Hz,1H), 3.35(m,1H), 3.20(m,1H), 2.43(m,1H), 2.12 (dt,J=12.8,6.3Hz,1H), 1.99 (dt,J=12.9,6.3Hz,1H), 1.92-1.68(m,4H), 1.68-1.44(m,7H), 1.40-1.24(m,5H). ESI-MS m/z 470.23[M+H]⁺.

### Example 53: Synthesis of Compound 53

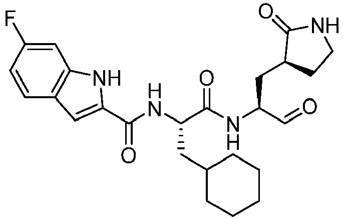

The Compound **53** was synthesized according to the synthesis of Compound **47** by using Compound **46-1** to replace the Compound **14-1** in Example 47.

¹H NMR(600 MHz,Acetone-d6) δ 9.30 (s,1H), 9.23 (s,1H), 8.54 (d,J=9.2Hz,1H), 7.90 (dd,J=8.4,4.9Hz,1H), 7.79 (d,J=8.8Hz,1H), 7.49 (s,1H), 7.18-7.07(m,2H), 6.55 (t,J=4.4Hz,1H), 4.51 (dt,J=8.8,6.3Hz,1H), 4.43 (dt,J=9.3,6.9Hz,1H), 3.36(m,1H), 3.21(m,1H), 2.42(m,1H), 2.16 (dt,J=12.9,6.4Hz,1H), 1.99 (dt,J=12.8,6.3Hz,1H), 1.86-1.66(m,5H), 1.59(m,2H), 1.55-1.50(m,2H), 1.50-1.44(m,2H), 1.41-1.20(m,5H). ESI-MS m/z 470.23[M+H]⁺.

### Example 54: Synthesis of Compound 54

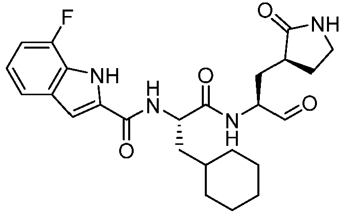

The Compound **54** was synthesized according to the synthesis of Compound **47** by using Compound **43-1** to replace the Compound **14-1** in Example 47.

¹H NMR(600 MHz,Acetone-d6) δ 9.78 (s,1H), 9.21 (s,1H), 8.63 (d,J=9.3Hz,1H), 7.74 (d,J=8.8Hz,1H), 7.57 (d,J=7.9Hz,1H), 7.47 (s,1H), 7.30 (td,J=7.9,5.0Hz,1H), 7.00 (t,J=7.9Hz,1H), 6.55 (t,J=4.4Hz,1H), 4.47-4.35(m,2H), 3.37(m,1H), 3.22(m,1H), 2.43(m,4.5Hz,1H), 2.21 (dt,J=12.8,6.3Hz,1H), 1.94 (dt,J=12.9,6.3Hz,1H), 1.88-1.66(m,5H), 1.64-1.44(m,5H), 1.41-1.21(m,5H). ESI-MS m/z 470.23[M+H]⁺.

### Example 55: Synthesis of Compound 55

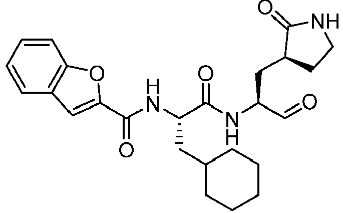

The Compound **55** was synthesized according to the synthesis of Compound **47** by using Compound **23-1** to replace the Compound **14-1** in Example 47.

¹H NMR(600 MHz,Acetone-d6) δ 9.21 (s,1H), 8.50 (d,J=9.3Hz,1H), 7.83-7.76(m,2H), 7.64 (dd,J=9.8,7.7Hz,2H), 7.39 (t,J=7.5Hz,1H), 7.32 (t,J=7.4Hz,1H), 6.55 (t,J=4.4Hz,1H), 4.38 (dt,J=8.8,6.4Hz,1H), 4.25 (dt,J=9.3,6.9Hz,1H), 3.32(m,1H), 3.20(m,1H), 2.42(m,1H), 2.16 (dt,J=12.9,6.4Hz,1H), 1.98-1.65(m,6H), 1.59(m,2H), 1.57-1.44(m,3H), 1.41-1.18(m,5H).ESI-MS m/z 453.23 [M+H]⁺.

### Example 56: Synthesis of Compound 56

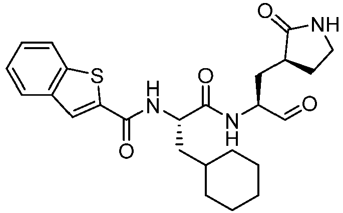

The Compound **56** was synthesized according to the synthesis of Compound **47** by using Compound **24-1** to replace the Compound **14-1** in Example 47.

¹H NMR(600 MHz,Acetone-d6) δ 9.30 (s,1H), 8.46 (d,J=9.3Hz,1H), 8.30 (s,1H), 7.96 (d,J=7.8Hz,1H), 7.83-7.77(m,2H), 7.42 (t,J=7.6Hz,1H), 7.35 (t,J=7.6Hz,1H), 6.55 (t,J=4.4Hz,1H), 4.48 (dt,J=8.8,6.4Hz,1H), 4.29 (dt,J=9.3,6.9Hz,1H), 3.32-3.16(m,2H), 2.36(m,1H), 2.17 (dt,J=12.9,6.4Hz,1H), 1.96(m,2H), 1.86-1.44(m,10H), 1.41-1.17(m,6H).ESI-MS m/z 469.20[M+H]⁺.

### Example 57: Synthesis of Compound 57

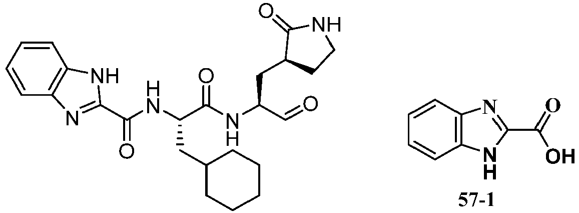

The Compound **49** was synthesized according to the synthesis of Compound **47** by using Compound **38-1** to replace the Compound **14-1** in Example 47.

¹H NMR(600 MHz,Acetone-d6) δ 9.30 (s,1H), 8.63 (d,J=9.3Hz,1H), 7.83-7.73(m,2H), 7.65-7.57(m,1H), 7.29-7.21(m,2H), 6.55 (t,J=4.4Hz,1H), 4.56-4.47(m,2H), 3.36(m,1H), 3.21(m,1H), 2.42(m,1H), 2.15 (dt,J=12.8,6.3Hz,1H), 1.98 (dt,J=12.8,6.3Hz,1H), 1.85-1.66(m,5H), 1.63-1.45(m,6H), 1.42-1.21(m,6H). ESI-MS m/z 453.24[M+H]⁺.

### Example 58: Synthesis of Compound 58

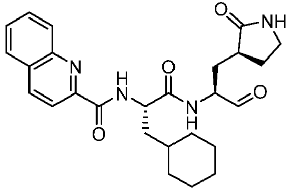

The Compound **58** was synthesized according to the synthesis of Compound **47** by using Compound **11-1** to replace the Compound **14-1** in Example 47.

¹H NMR(600 MHz,Acetone-d6) δ 9.21 (s,1H), 8.57 (d,J=9.3Hz,1H), 8.35-8.26(m,2H), 8.15 (d,J=8.1Hz,1H), 7.93 (d,J=7.5Hz,1H), 7.85 (dd,J=8.2,7.4Hz,1H), 7.71 (d,J=8.8Hz,1H), 7.59 (t,J=7.4Hz,1H), 6.55 (t,J=4.4Hz,1H), 4.45-4.35(m,2H), 3.27(m,1H), 3.12(m,1H), 2.40(m,1H), 2.10 (dt,J=12.8,6.3Hz,1H), 2.00-1.89(m,2H), 1.84-1.65(m,3H), 1.65-1.43(m,6H), 1.39-1.19(m,5H). ESI-MS m/z 464.24[M+H]⁺.

### Example 59: Synthesis of Compound 59

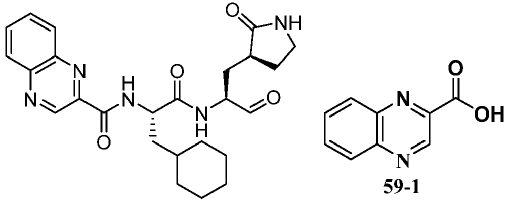

The Compound **59** was synthesized according to the synthesis of Compound **47** by using Compound **59-1** to replace the Compound **14-1** in Example 47.

¹H NMR(600 MHz,Acetone-d6) δ 9.36 (s,1H), 9.21 (s,1H), 8.64 (d,J=9.3Hz,1H), 7.99 (dd,J=7.8,3.7Hz,2H), 7.86 (t,J=7.6Hz,1H), 7.75 (d,J=9.0Hz,1H), 7.61-7.54(m,1H), 6.55 (t,J=4.4Hz,1H), 4.43 (dt,J=9.3,6.9Hz,1H), 4.37 (dt,J=8.8,6.4Hz,1H), 3.23(m,1H), 3.02(m,1H), 2.36(m,1H), 2.09 (dt,J=12.7,6.3Hz,1H), 1.99-1.89(m,2H), 1.83-1.44(m,10H), 1.40-1.19(m,6H).ESI-MS m/z 465.24[M+H]⁺.

### Example 60: Synthesis of Compound 60

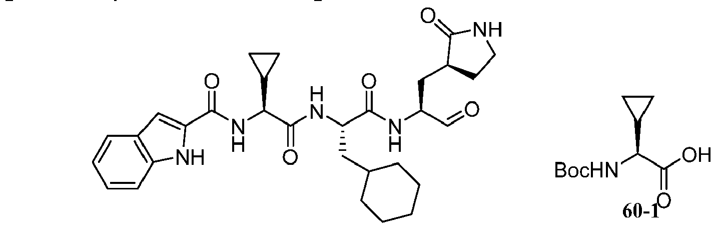

The Compound **60** was synthesized according to the synthesis of Compound **9** by using Compound **60-1** to replace the Compound **1-8** in Example 5.

¹H NMR(600 MHz,Acetone-d6) δ 9.30 (s,1H), 9.12 (s,1H), 8.79 (d,J=9.1Hz,1H), 7.71-7.60(m,3H), 7.42-7.35(m,2H), 7.23 (t,J=7.6Hz,1H), 7.20-7.13(m,1H), 6.55 (t,J=4.4Hz,1H), 4.50 (dt,J=8.8,6.4Hz,1H), 4.38 (dt,J=9.3,6.8Hz,1H), 4.18 (dd,J=9.1,5.2Hz,1H), 3.30(m,1H), 3.15(m,1H), 2.39(m,1H), 2.11 (dt,J=12.8,6.3Hz,1H), 1.97 (dt,J=12.9,6.3Hz,1H), 1.92-1.65(m,5H), 1.62-1.52(m,1H), 1.52-1.31(m,9H), 1.23-1.08(m,4H), 0.89-0.80(m,2H).ESI-MS m/z 549.29[M+H]⁺.

### Example 61: Synthesis of Compound 61

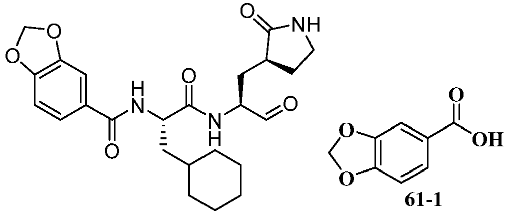

The Compound **61** was synthesized according to the synthesis of Compound **47** by using Compound **61-1** to replace the Compound **14-1** in Example 47.

¹H NMR(600 MHz,Acetone-d6) δ 9.21 (s,1H), 8.48 (d,J=9.3Hz,1H), 7.66 (d,J=8.8Hz,1H), 7.54 (d,J=8.4Hz,1H), 7.42 (s,1H), 6.88 (d,J=8.4Hz,1H), 6.55 (t,J=4.4Hz,1H), 6.05 (d,J=6.8Hz,2H), 4.35(m,2H), 3.32(m,1H), 3.19(m,1H), 2.40(m,1H), 2.19 (dt,J=12.8,6.3Hz,1H), 1.93-1.67(m,6H), 1.59(m,2H), 1.57-1.44(m,3H), 1.41-1.20(m,6H).ESI-MS m/z 457.22[M+H]⁺.

### Example 62: Synthesis of Compound 62

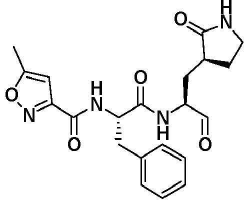

The Compound **62** was synthesized according to the synthesis of Compound **47** by using Compound **6-1** to replace the Compound **14-1** in Example 47.

¹H NMR(600 MHz,Acetone-d6) δ 11.14 (d,J=8.4Hz,1H), 9.41 (s,1H), 8.28-8.23(m,1H), 7.78 (dd,J=12.5,8.8Hz,1H), 7.58 (d,J=1.8Hz,1H), 7.47 (d,J=8.8Hz,1H), 7.37-7.33(m,2H), 7.23 (d,J=7.7Hz,2H), 7.16 (dd,J=6.3,1.9Hz,4H), 5.44 (dd,J=70.6,8.0Hz,1H), 5.06 (dd,J=7.1,4.5Hz,1H), 4.51(m,1H), 4.13-3.99(m,1H), 3.32 (d,J=2.0Hz,1H), 3.20(m,2H), 2.44-2.34(m,1H), 2.33-2.25(m,1H), 1.79-1.55(m,2H). ESI-MS m/z 413.17[M+H]⁺.

### Example 63: Synthesis of Compound 63

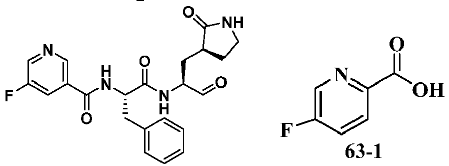

The Compound **63** was synthesized according to the synthesis of Compound **47** by using Compound **63-1** to replace the Compound **14-1** in Example 47.

¹H NMR(600 MHz,Acetone-d6) δ 9.42 (s,1H), 8.87 (d,J=12.0Hz,1H), 8.75 (d,J=6.0Hz,1H), 8.63 (d,J=2.7Hz,1H), 8.24 (d,J=8.1Hz,1H), 7.98-7.94(m,1H), 7.37 (d,J=7.3Hz,2H), 7.30 (dd,J=10.4,4.7Hz,2H), 7.22(m,1H), 5.01(m,1H), 4.32-4.27(m,1H), 3.38-3.34(m,1H), 3.31-3.27(m,1H), 3.19 (dd,J=13.9,8.9Hz,1H), 2.51-2.40(m,1H), 2.39-2.33(m,1H), 1.97-1.88(m,1H), 1.82(m,1H), 1.39 (s,1H), 1.31 (d,J=2.5Hz,2H). ESI-MS m/z 427.16[M+H]⁺.

### Example 64: Synthesis of Compound 64

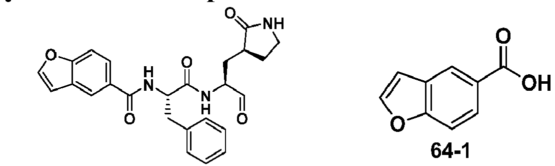

The Compound **64** was synthesized according to the synthesis of Compound **12** by using Compound **64-1** to replace the Compound **5-1** in Example 12, and using Compound **8-1** to replace the Compound **12-1** in Example 1.

¹H NMR(600 MHz,Acetone-d6) δ 9.43 (s,1H), 8.59 (d,J=6.8Hz,1H), 8.18 (dd,J=15.6,1.8Hz,1H), 8.08-7.99(m,1H), 7.92-7.82(m,2H), 7.53 (t,J=8.1Hz,1H), 7.38 (d,J=7.3Hz,2H), 7.28 (t,J=7.5Hz,2H), 7.19 (t,J=7.3Hz,1H), 7.13 (s,1H), 6.97-6.90(m,1H), 5.07(m,1H), 4.37(m,1H), 3.37-3.33(m,1H), 3.29-3.20(m,3H), 2.52-2.24(m,2H), 2.05-1.98(m,1H), 1.87-1.62(m,2H). ESI-MS m/z 448.17[M+H]⁺.

### Example 65: Synthesis of Compound 65

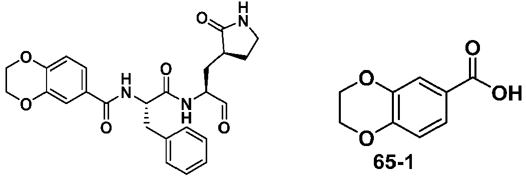

The Compound **65** was synthesized according to the synthesis of Compound **12** by using Compound **65-1** to replace the Compound **5-1** in Example 12, and using Compound **8-1** to replace the Compound **12-1** in Example 1.

¹H NMR(600 MHz,Acetone-d6) δ 9.22 (s,1H), 8.36 (d,J=6.3Hz,1H), 7.32 (d,J=2.0Hz,1H), 7.23 (s,5H), 7.02 (d,J=8.1Hz,1H), 6.83 (d,J=8.4Hz,1H), 6.55 (s,1H), 5.07(m,1H), 4.27-4.21(m,6H), 3.31-3.24(m,2H), 3.20 (dd,J=14.4,6.7Hz,2H), 2.35-2.28(m,2H), 1.90 (dd,J=10.2,6.4Hz,1H), 1.80(m,2H). ESI-MS m/z 466.18[M+H]⁺.

### Example 66: Synthesis of Compound 66

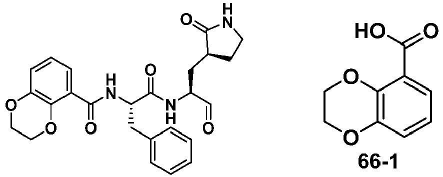

The Compound **66** was synthesized according to the synthesis of Compound **12** by using Compound **66-1** to replace the Compound **5-1** in Example 12, and using Compound **8-1** to replace the Compound **12-1** in Example 1.

¹H NMR(600 MHz,Acetone-d6) δ 11.14 (d,J=8.8Hz,1H), 9.41 (s,1H), 8.28-8.23(m,1H), 7.78(m,1H), 7.58 (d,J=1.8Hz,1H), 7.47 (d,J=8.8Hz,1H), 7.37-7.33(m,2H), 7.23 (d,J=7.7Hz,2H), 7.16 (dd,J=6.3,1.9Hz,4H), 5.44 m,1H), 5.06 (dd,J=7.1,4.5Hz,1H), 4.51(m,1H), 4.13-3.99(m,1H), 3.32 (d,J=2.0Hz,1H), 3.20(m,2H), 2.44-2.34(m,1H), 2.33-2.25(m,1H), 1.79-1.55(m,2H). ESI-MS m/z 466.18[M+H]⁺.

### Example 67: Synthesis of Compound 67

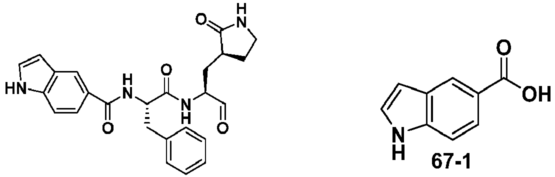

The Compound **67** was synthesized according to the synthesis of Compound **12** by using Compound **67-1** to replace the Compound **5-1** in Example 12, and using Compound **8-1** to replace the Compound **12-1** in Example 1.

¹H NMR(600 MHz,Acetone-d6) δ 10.53 (s,1H), 9.39 (s,1H), 8.19-8.16(m,1H), 7.73 (d,J=8.0Hz,1H), 7.66 (dd,J=8.5,1.7Hz,1H), 7.43 (d,J=8.5Hz,1H), 7.40-7.38(m,1H), 7.37-7.33(m,2H), 7.26(m,2H), 7.19-7.15(m,1H), 6.96 (s,1H), 6.54(m,1H), 5.01 (dt,J=8.2,4.0Hz,1H), 4.31-4.26(m,1H), 3.31 (dd,J=8.2,5.6Hz,1H), 3.26-3.18(m,3H), 2.45-2.36(m,1H), 2.32-2.21(m,1H), 1.98-1.95(m,1H), 1.81-1.68(m,2H). ESI-MS m/z 447.19[M+H]⁺.

### Example 68: Synthesis of Compound 68

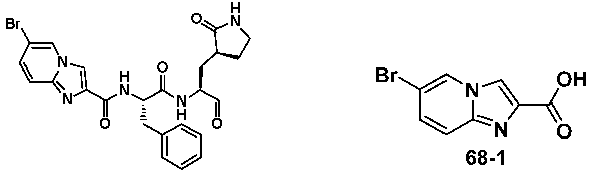

The Compound **68** was synthesized according to the synthesis of Compound **12** by using Compound **68-1** to replace the Compound **5-1** in Example 12, and using Compound **8-1** to replace the Compound **12-1** in Example 1.

¹H NMR(600 MHz,Acetone-d6) δ 9.40 (s,1H), 8.79 (s,1H), 8.53 (d,J=6.2Hz,1H), 8.27 (d,J=4.9Hz,1H), 8.04 (d,J=7.3Hz,1H), 7.58-7.52(m,1H), 7.47-7.40(m,1H), 7.32 (t,J=6.4Hz,2H), 7.28-7.22(m,2H), 7.18 (t,J=7.2Hz,1H), 6.91 (s,1H), 5.03-4.93(m,1H), 4.44(m,1H), 3.32 (t,J=6.9Hz,1H), 3.27-3.23(m,1H), 2.45-2.26(m,2H), 1.99-1.94(m,1H), 1.80-1.75(m,1H), 1.37 (s,1H), 1.29 (d,J=3.3Hz,2H). ESI-MS m/z 526.11[M+H]⁺.

### Example 69: Synthesis of Compound 69

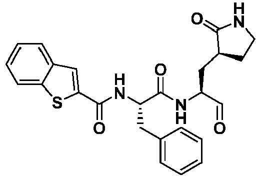

The Compound **69** was synthesized according to the synthesis of Compound **12** by using Compound **24-1** to replace the Compound **5-1** in Example 12, and using Compound **8-1** to replace the Compound **12-1** in Example 1.

¹H NMR(600 MHz,Acetone-d6) δ 11.14 (d,J=8.4Hz,1H), 9.41 (s,1H), 8.28-8.23(m,1H), 7.78 (dd,J=12.5,8.8Hz,1H), 7.58 (d,J=1.8Hz,1H), 7.47 (d,J=8.8Hz,1H), 7.37-7.33(m,2H), 7.23 (d,J=7.7Hz,2H), 7.16 (dd,J=6.3,1.9Hz,4H), 5.44(m,1H), 5.06 (dd,J=7.1,4.5Hz,1H), 4.51(m,1H), 4.13-3.99(m,1H), 3.32 (d,J=2.0Hz,1H), 3.20(m,2H), 2.44-2.34(m,1H), 2.33-2.25(m,1H), 1.79-1.55(m,2H). ESI-MS m/z 464.15[M+H]⁺.

### Example 70: Synthesis of Compound 70

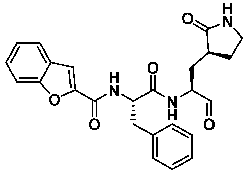

The Compound **70** was synthesized according to the synthesis of Compound **12** by using Compound **10-1** to replace the Compound **5-1** in Example 12, and using Compound **8-1** to replace the Compound **12-1** in Example 1.

¹H NMR(600 MHz,Acetone-d6) δ 9.43 (s,1H), 8.64 (d,J=6.8Hz,1H), 8.11 (d,J=8.0Hz,1H), 7.71 (d,J=7.5Hz,1H), 7.56-7.50(m,1H), 7.49-7.41(m,2H), 7.37 (dd,J=9.2,2.3Hz,2H), 7.32-7.22(m,3H), 7.19 (d,J=7.5Hz,1H), 7.12 (s,1H), 5.10-5.01(m,1H), 4.58-4.32(m,1H), 3.38(m,1H), 3.25 (dd,J=9.0,2.5Hz,3H), 2.53-2.27(m,2H), 2.04(m,1H), 1.86-1.62(m,2H). ESI-MS m/z 448.17[M+H]⁺.

### Example 71: Synthesis of Compound 71

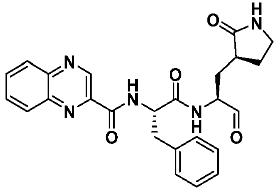

The Compound **71** was synthesized according to the synthesis of Compound **12** by using Compound **59-1** to replace the Compound **5-1** in Example 12, and using Compound **8-1** to replace the Compound **12-1** in Example 1.

¹H NMR(600 MHz,Acetone-d6) δ 9.47 (s,1H), 8.69 (d,J=8.0Hz,1H), 8.15(m,2H), 7.95(m,2H), 7.80 (d,J=8.0Hz,1H), 7.34 (d,J=7.3Hz,2H), 7.25 (t,J=7.3Hz,2H), 7.18 (t,J=7.3Hz,1H), 6.83 (s,1H), 5.03-4.95(m,1H), 3.99 (dd,J=7.3,4.3Hz,1H), 3.50(m,2H), 3.31-3.20(m,3H), 2.39-2.28(m,2H), 1.95-1.87(m,1H), 1.78-1.68(m,1H), 1.62-1.51(m,1H). ESI-MS m/z 460.18[M+H]⁺.

### Example 72: Synthesis of Compound 72

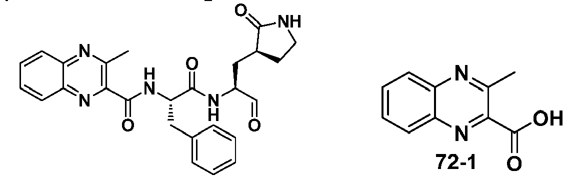

The Compound **72** was synthesized according to the synthesis of Compound **1** by using Compound **72-1** to replace the acid **1-11** in Example 1, and using Compound **8-1** to replace the Compound **1-5** in Example 1.

¹H NMR(600 MHz,Acetone-d6) δ 9.30 (s,1H), 8.63 (d,J=8.5Hz,1H), 8.33 (d,J=6.2Hz,1H), 8.06 (d,J=7.5Hz,1H), 8.02 (d,J=8.5Hz,1H), 7.80(m,1H), 7.75 (dd,J=11.0,4.0Hz,1H), 7.39-7.27(m,5H), 7.25 (d,J=7.1Hz,1H), 6.56 (s,1H), 5.12(q,J=7.1Hz,1H), 4.32(m,1H), 3.31 (dd,J=12.8,6.2Hz,3H), 3.02 (s,3H), 2.40-2.30(m,2H), 1.99-1.93(m,1H), 1.88-1.75(m,2H). ESI-MS m/z 474.21[M+H]⁺.

### Example 73: Synthesis of Compound 73

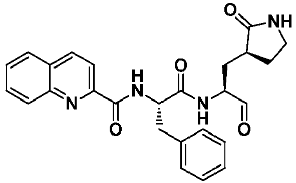

The Compound **73** was synthesized according to the synthesis of Compound **1** by using Compound **11-1** to replace the acid **1-11** in Example 1, and using Compound **8-1** to replace the Compound **1-5** in Example 1.

¹H NMR(600 MHz,Acetone-d6) δ 9.22 (s,1H), 8.84 (d,J=8.5Hz,1H), 8.28 (d,J=8.5Hz,1H), 8.22 (dd,J=10.1,6.0Hz,1H), 8.11(m,2H), 7.86 (d,J=8.1Hz,1H), 7.77 (dd,J=11.2,4.1Hz,1H), 7.62(m,1H), 7.36-7.27(m,4H), 7.24 (t,J=7.2Hz,1H), 5.08(m,1H), 4.33-4.28(m,1H), 3.35-3.29(m,2H), 3.25 (t,J=6.8Hz,2H), 2.38-2.30(m,2H), 1.93-1.84(m,2H), 1.79-1.71(m,2H). ESI-MS m/z 459.19[M+H]⁺.

### Example 74: Synthesis of Compound 74

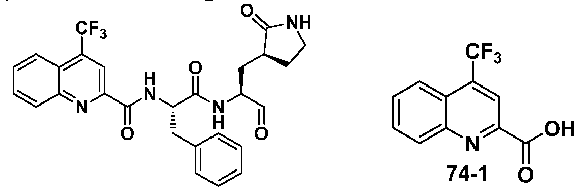

The Compound **74** was synthesized according to the synthesis of Compound **1** by using Compound **74-1** to replace the acid **1-11** in Example 1, and using Compound **8-1** to replace the Compound **1-5** in Example 1.

¹H NMR(600 MHz,Acetone-d6) δ 9.46 (s,1H), 8.91 (dd,J=7.9,4.6Hz,1H), 8.71 (d,J=6.6Hz,1H), 8.44-8.39(m,1H), 8.24(m,1H), 8.17 (t,J=7.9Hz,1H), 7.98 (dd,J=14.5,7.3Hz,1H), 7.90(m,1H), 7.42-7.38(m,2H), 7.30-7.25(m,2H), 7.20 (dd,J=10.4,4.4Hz,1H), 7.02(m,1H), 5.16-5.07(m,1H), 4.62-4.32(m,1H), 3.42-3.37(m,1H), 3.30 (s,2H), 2.53-2.30(m,2H), 1.99(m,1H), 1.88-1.62(m,2H). ESI-MS m/z 527.18[M+H]⁺.

### Example 75: Synthesis of Compound 75

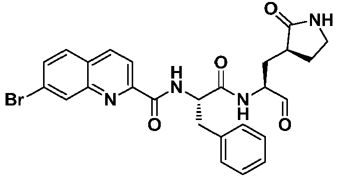

The Compound **75** was synthesized according to the synthesis of Compound **1** by using Compound **24-1** to replace the acid **1-11** in Example 1, and using Compound **8-1** to replace the Compound **1-5** in Example 1.

¹H NMR(600 MHz,Acetone-d6) δ 11.14 (d,J=8.4Hz,1H), 9.41 (s,1H), 8.28-8.23(m,1H), 7.78 (dd,J=12.5,8.8Hz,1H), 7.58 (d,J=1.8Hz,1H), 7.47 (d,J=8.8Hz,1H), 7.37-7.33(m,2H), 7.23 (d,J=7.7Hz,2H), 7.16 (dd,J=6.3,1.9Hz,4H), 5.44(m,1H), 5.06 (dd,J=7.1,4.5Hz,1H), 4.51(m,1H), 4.13-3.99(m,1H), 3.32 (d,J=2.0Hz,1H), 3.20(m,2H), 2.44-2.34(m,1H), 2.33-2.25(m,1H), 1.79-1.55(m,2H). ESI-MS m/z 537.10[M+H]⁺.

### Example 76: Synthesis of Compound 76

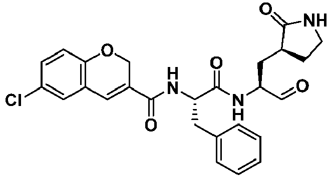

The Compound **76** was synthesized according to the synthesis of Compound **1** by using Compound **2-1** to replace the acid **1-11** in Example 1, and using Compound **8-1** to replace the Compound **1-5** in Example 1.

¹H NMR(600 MHz,Acetone-d6) δ 9.24 (s,1H), 8.55 (d,J=6.0Hz,1H), 7.29-7.26(m,2H), 7.22(m,5H), 7.10(m,1H), 6.99 (d,J=2.6Hz,1H), 6.95 (s,1H), 6.72 (d,J=8.6Hz,1H), 6.58 (s,1H), 5.01 (dd,J=14.9,6.9Hz,1H), 4.91-4.85(m,2H), 4.25-4.20(m,1H), 3.32-3.26(m,2H), 3.18(m,2H), 2.34-2.29(m,2H), 1.94-1.87(m,1H), 1.82-1.77(m,1H). ESI-MS m/z 496.15[M+H]⁺.

### Example 77: Synthesis of Compound 77

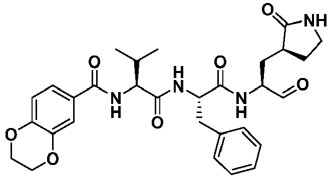

The Compound **77** was synthesized according to the synthesis of Compound **1** by using Compound **65-1** to replace the acid **1-11** in Example 1, and using Compound **8-1** to replace the Compound **1-5** in Example 1.

¹H NMR(600 MHz,Acetone-d6 ) δ 9.72 (s,1H), 8.05 (s,1H), 7.65-7.46(m,3H), 7.21-7.09(m,3H), 5.99 (s,1H), 5.78 (s,1H), 5.08 (s,1H), 4.91(m,2H), 4.29(m,4H), 3.64 (s,1H), 3.54 (s,1H), 3.29 (s,1H), 3.04 (s,1H), 2.81 (s,1H), 2.73 (s,1H), 2.22 (s,1H), 2.02 (s,1H), 1.91 (s,1H), 0.96 (s,6H). ESI-MS m/z 565.26[M+H]⁺.

### Example 78: Synthesis of Compound 78

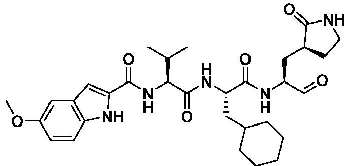

The Compound **78** was synthesized according to the synthesis of Compound **1** by using Compound **13-1** to replace the acid **1-11** in Example 1.

¹H NMR(600 MHz,Acetone-d6 ) δ 9.69 (s,1H), 8.57 (s,1H), 8.39 (s,1H), 7.56 (s,1H), 7.42 (s,1H), 7.14 (s,1H), 6.89 (s,1H), 6.77 (s,1H), 5.95 (s,1H), 5.43 (s,1H), 5.16 (s,1H), 4.92 (s,1H), 4.49 (s,1H), 3.80 (s,3H), 3.64 (s,1H), 3.53 (s,1H), 2.85 (s,1H), 2.72 (s,1H), 2.22 (s,1H), 2.02 (s,1H), 1.91 (s,1H), 1.75 (s,1H), 1.60 (s,1H), 1.54-1.36(m,7H), 1.29 (s,1H), 0.96 (s,6H). ESI-MS m/z 582.32[M+H]⁺.

### Example 79: Synthesis of Compound 79

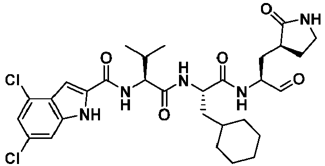

The Compound **79** was synthesized according to the synthesis of Compound **1** by using Compound **14-1** to replace the acid **1-11** in Example 1.

¹H NMR(600 MHz,Acetone-d6 ) δ 9.72 (s,1H), 8.59 (s,1H), 7.41 (s,1H), 7.41 - 7.28(m,3H), 7.10 (s,1H), 6.67 (s,1H), 6.04 (s,1H), 4.99 (s,1H), 4.78 (s,1H), 4.50 (s,1H), 3.65 (s,1H), 3.53 (s,1H), 2.73 (s,1H), 2.56 (s,1H), 2.21 (s,1H), 2.02 (s,1H), 1.91 (s,1H), 1.76 (s,2H), 1.60 (s,1H), 1.51(m,3H), 1.48 -1.41(m,4H), 1.38 (s,1H), 0.96 (s,6H).ESI-MS m/z 620.23[M+H]⁺.

### Example 80: Synthesis of Compound 80

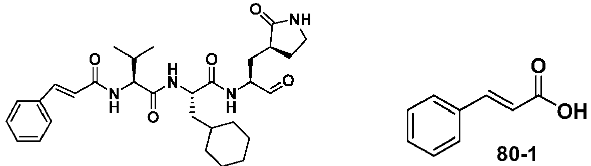

The Compound **80** was synthesized according to the synthesis of Compound **1** by using Compound **80-1** to replace the acid **1-11** in Example 1.

¹H NMR(600 MHz,Acetone-d6 ) δ 9.72 (s,1H), 7.91 (s,2H), 7.59 (s,2H), 7.51 (s,1H), 7.32(m,1H), 6.99 (s,1H), 6.08 (s,1H), 6.01 (s,1H), 5.53 (s,1H), 4.91(m,2H), 4.59 (s,1H), 3.65 (s,1H), 3.54 (s,1H), 2.73(m,2H), 2.22 (s,1H), 2.02 (s,1H), 1.92 (s,1H), 1.76 (s,1H), 1.58 (s,1H), 1.55-1.41(m,5H), 1.28 (s,1H), 0.96 (s,6H). ESI-MS m/z 539.31[M+H]⁺.

### Example 81: Synthesis of Compound 81

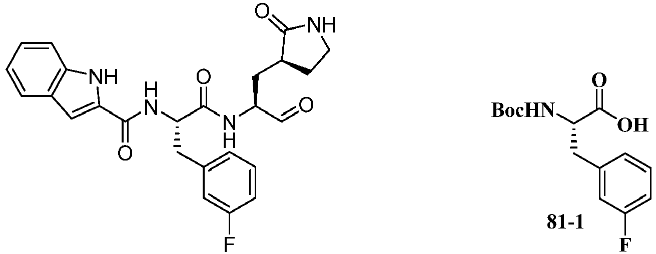

The Compound **81** was synthesized according to the synthesis of Compound **12** by using Compound **81-1** to replace the Compound **12-1** in Example 12.

¹H NMR(600 MHz,Acetone-d6) δ 9.30 (s,1H), 9.11 (s,1H), 8.57 (d,J=9.3Hz,1H), 7.80 (d,J=9.0Hz,1H), 7.74 (s,1H), 7.66 (d,J=7.6Hz,1H), 7.39 (d,J=7.8Hz,1H), 7.30 (td,J=7.9,4.9Hz,1H), 7.25-7.13(m,2H), 6.98 (td,J=8.1,2.9Hz,3H), 6.55 (t,J=4.4Hz,1H), 5.07 (dt,J=9.3,7.7Hz,1H), 4.46 (dt,J=8.8,6.3Hz,1H), 3.33(m,1H), 3.16(m,1H), 3.03 (dd,J=14.0,7.8Hz,1H), 2.96 (dd,J=13.9,7.7Hz,1H), 2.28(m,1H), 2.11 (dt,J=12.9,6.4Hz,1H), 1.95 (dt,J=12.9,6.3Hz,1H), 1.84-1.69(m,2H). ESI-MS m/z 464.19[M+H]+.

### Example 82: Synthesis of Compound 82

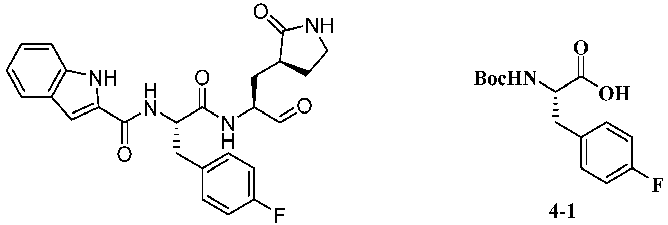

The Compound **82** was synthesized according to the synthesis of Compound **12** by using Compound **4-1** to replace the Compound **12-1** in Example 12.

¹H NMR (500 MHz, Chloroform) δ 11.66 (s, 1H), 9.72 (s, 1H), 7.98 (dt, *J* = 14.9, 3.1 Hz, 1H), 7.60 (dd, *J* = 14.9,3.2 Hz, 1H), 7.31 (d, *J* = 3.0 Hz, 1H), 7.29 - 6.93 (m, 7H), 6.44 (s, 1H), 5.20 (dd, *J* = 25.8, 13.3 Hz, 1H), 5.04 (t*, J* = 10.4 Hz, 1H), 3.76 - 3.48 (m, 2H), 3.29 (dd, *J* = 24.7, 10.3 Hz, 1H), 3.04 (dd, *J* = 24.8, 10.4 Hz, 1H), 2.59 (ddd, *J* = 23.1, 15.0, 9.2 Hz, 1H), 2.32-2.14 (m, 1H), 2.07-1.88 (m, 3H). ESI-MS m/z 465.19[M+H]⁺.

### Example 83: Synthesis of Compound 83

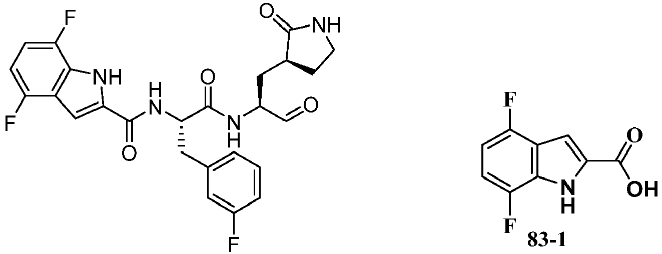

The Compound **83** was synthesized according to the synthesis of Compound **81** by using Compound **83-1** to replace the Compound **5-1** in Example 81.

¹H NMR (500 MHz, Chloroform ) δ 10.96 (s, 1H), 9.72 (s, 1H), 7.86 (s, 1H), 7.43 (d*, J* = 5.0 Hz, 1H), 7.39 - 7.26 (m, 2H), 7.14 - 6.95 (m, 5H), 5.04 (t, *J* = 5.4 Hz, 1H), 4.84 (q, *J* = 6.0 Hz, 1H), 3.69 (dt*, J* = 12.7, 6.5 Hz, 1H), 3.56 (dt, *J* = 12.7, 6.5 Hz, 1H), 3.29 (dd, *J* = 12.4, 5.3 Hz, 1H), 3.04 (dd, *J* = 12.4, 5.3 Hz, 1H), 2.61 (tt, *J* = 9.1, 6.5 Hz, 1H), 2.23 (ddt, *J* = 12.9, 9.1, 6.4 Hz, 1H), 2.02 (t, *J* = 6.2 Hz, 2H), 1.99-1.88 (m, 1H). ESI-MS m/z 501.17[M+H]⁺.

### Example 84: Synthesis of Compound 84

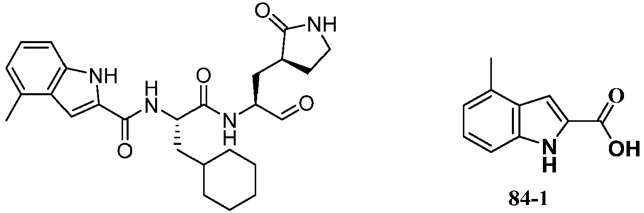

The Compound **84** was synthesized according to the synthesis of Compound **47** by using Compound **84-1** to replace the Compound **14-1** in Example 47.

¹H NMR (500 MHz, Chloroform ) δ 11.97 (s, 1H), 9.72 (s, 1H), 7.50 (dd, *J* = 14.9, 3.2 Hz, 1H), 7.11 (s, 1H), 7.03 (t, *J* = 15.0 Hz, 1H), 6.85 (dd, *J* = 15.0, 3.1 Hz, 1H), 6.45 (s, 2H), 5.09 (dd, *J* = 25.7, 13.2 Hz, 1H), 4.46 (t*, J* = 11.8 Hz, 1H), 3.74 - 3.48 (m, 2H), 2.66 - 2.53 (m, 4H), 2.21 (ddd, *J* = 25.3, 16.7, 12.5 Hz, 1H), 2.07 - 1.88 (m, 4H), 1.86 - 1.63 (m, 7H), 1.31 (p*, J* = 11.6 Hz, 2H), 1.17 - 0.97 (m, 3H). ESI-MS m/z 467.26[M+H]⁺.

### Example 85: Synthesis of Compound 85

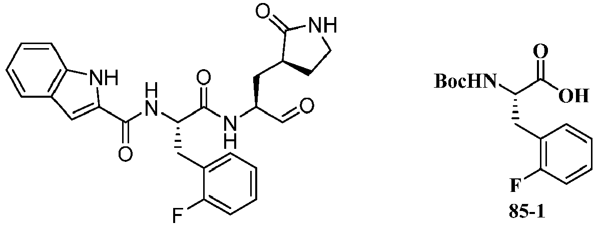

The Compound **85** was synthesized according to the synthesis of Compound **12** by using Compound **85-1** to replace the Compound **12-1** in Example 12.

¹H NMR (500 MHz, Chloroform) δ11.30 (s, 1H),9.72 (s, 1H), 8.78 (s, 1H), 7.98 (dt*, J* = 14.9, 3.1 Hz, 1H), 7.60 (dtd, *J* = 14.7, 8.0, 3.2 Hz, 2H), 7.29 - 6.93 (m, 6H), 6.35 (s, 1H), 6.02 (s, 1H), 5.66 (s, 1H), 5.36 (dd, *J* = 23.9, 11.7 Hz, 1H), 4.96 (t, *J* = 10.8 Hz, 1H), 3.72 - 3.47 (m, 2H), 3.29 (dd, *J* = 24.7, 10.8 Hz, 1H), 3.04 (dd, *J* = 24.7, 10.8 Hz, 1H), 2.88 (tt, *J* = 18.3, 12.5 Hz, 1H), 2.23 (ddt, *J* = 25.5, 18.3, 12.8 Hz, 1H), 2.07-1.82 (m, 3H). ESI-MS m/z 465.19[M+H]⁺.

### Example 86: Synthesis of Compound 86

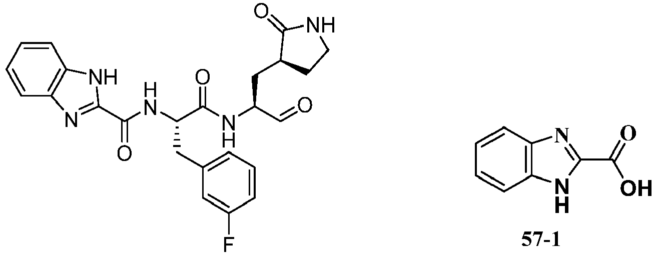

The Compound **86** was synthesized according to the synthesis of Compound **81** by using Compound **57-1** to replace the Compound **5-1** in Example 81.

¹H NMR (500 MHz, Chloroform) δ 13.79 (s, 1H), 9.71 (s, 1H), 7.59 (dt, *J* = 14.9, 7.4 Hz, 2H), 7.36 - 7.21 (m, 3H), 7.07 - 6.94 (m, 3H), 6.35 (s, 1H), 6.15 (s, 1H), 5.41 (s, 1H), 5.15 (dt, *J* = 12.4, 10.3 Hz, 1H), 4.87 (t, *J* = 14.7 Hz, 1H), 3.75 - 3.60 (m, 1H), 3.60 - 3.45 (m, 1H), 3.29 (dd, *J* = 24.9, 14.5 Hz, 1H), 3.04 (dd, *J* = 24.9, 14.5 Hz, 1H), 2.69 (tt, *J* = 18.3, 6.8 Hz, 1H), 2.28 - 2.10 (m, 1H), 2.06-1.89 (m, 3H). ESI-MS m/z 466.18[M+H]⁺.

### Example 87: Synthesis of Compound 87

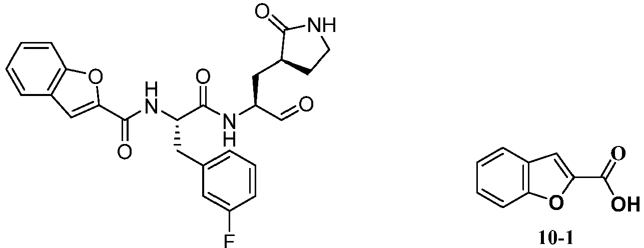

The Compound **87** was synthesized according to the synthesis of Compound **81** by using Compound **10-1** to replace the Compound **5-1** in Example 81.

¹H NMR (500 MHz, Chloroform ) δ 9.72 (s, 1H), 8.04 (s, 1H), 7.63 - 7.55 (m, 1H), 7.50 (dd, *J* = 14.3, 3.6 Hz, 1H), 7.36 - 7.15 (m, 5H), 7.06-6.94 (m, 2H), 6.06 (s, 1H), 5.65 (td, *J* = 15.4, 12.4 Hz, 1H), 5.55 (s, 1H), 4.90 (t*, J* = 11.8 Hz, 1H), 3.70 - 3.44 (m, 2H), 3.29 (dd, *J* = 24.8, 11.8 Hz, 1H), 3.04 (dd, *J* = 24.8, 11.8 Hz, 1H), 2.54 (td, *J* = 31.2, 16.3 Hz, 1H), 2.20 (ddt, *J* = 25.0, 16.3, 13.0 Hz, 1H), 2.07-1.84 (m, 3H). ESI-MS m/z 466.18[M+H]⁺.

### Example 88: Synthesis of Compound 88

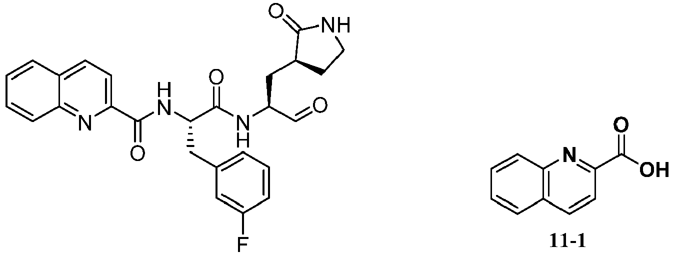

The Compound **88** was synthesized according to the synthesis of Compound **81** by using Compound **11-1** to replace the Compound **5-1** in Example 81.

¹H NMR (500 MHz, Chloroform ) δ 12.09 (s, 1H), 9.72 (s, 1H), 8.59 (dd, *J* = 7.5, 1.5 Hz, 1H), 8.49 (s, 1H), 8.20 (dd, *J* = 7.4, 1.3 Hz, 1H), 7.93 - 7.82 (m, 2H), 7.76 (d, *J* = 7.5 Hz, 1H), 7.57 (td, *J* = 7.5, 1.5 Hz, 1H), 7.39 - 7.26 (m, 2H), 7.00 (tdd, *J* = 5.6, 2.9, 1.6 Hz, 2H), 6.10 (s, 1H), 5.23 (t, *J* = 7.0 Hz, 1H), 4.55 (q, *J* = 6.5 Hz, 1H), 3.65 (dt, *J* = 12.8, 6.5 Hz, 1H), 3.52 (dt,*J* = 12.6, 6.5 Hz, 1H), 3.29 (dd,*J* = 12.5, 7.0 Hz, 1H), 3.04 (dd, *J* = 12.5, 7.0 Hz, 1H), 2.44 (tt, *J* = 9.1, 6.1 Hz, 1H), 2.21 (ddt, *J* = 12.8, 9.1, 6.5 Hz, 1H), 2.02 (t, *J* = 6.4 Hz, 2H), 1.92 (ddt, *J* = 12.7, 9.3, 6.5 Hz, 1H). ESI-MS m/z 477.19[M+H]⁺.

### Test Example 1: Evaluation of Inhibitory Activity to 3CL Protease of2019 Novel Coronavirus

Determination of the inhibitory activity of the compound against the 3CL protease of 2019 Novel Coronavirus (2019-nCoV 3CL^{pro}): Fluorescence resonance energy transfer (FRET) technology was used to determine the enzyme level inhibitory activity of the 3C protease inhibitor. In a 96-well plate, 27.5 µL of buffer (20mM Tris, 100mM NaCl, 1mM EDTA, pH 7.4) was added to each well, and 2.5 µL of compound (final concentrations 2 µM, 4 µM, 6 µM, 8 µM, 10 µM, 12 µM, 14 µM, 16 µM, 18 µM, 20 µM) and 5 µL of EV713Cpro (final concentration 3 µM) were added together. The system was incubated for 15 min at 37°C, and then 15 µL of fluorescent substrate diluted in buffer (of which the final concentration was 20 µM) was added. The fluorescence parameters were measured with a Ge n5 fluorometer at 340 nm excitation wavelength and 490 nm emission wavelength, respectively. The system was kept at 37°C for 10 min before the data was read. A negative control without any compound was used (the rest are the same). The obtained data was processed using GraphPad Prism 5 software, and the experimental results are shown in Tables 1A and 1B.

**Table 1A: Inhibitory Activity of 3CL protease of 2019 Novel Coronavirus**

| **Compound number** | **(1µM) Inhibition rate** | **Compound number** | **(1µM) Inhibition rate** |
|---|---|---|---|
| 5(DC402265) | 95.7 | 45 | 85.2 |
| 6(DC402264) | 95.8 | 46 | 89.3 |
| 7 | 88.7 | 47 | 90.4 |
| 8 | 86.5 | 48(DC402234) | 100.4 |
| 9(DC402240) | 95.8 | 49 | 91.2 |
| 10(DC402237) | 88.2 | 50 | 89.1 |
| 11 (DC40223 8) | 89.7 | 51 | 84.6 |
| 12 | 103.1 | 56 | 81.3 |
| 16(DC402267) | 89.1 (IC₅₀=74 nM)) | 57 | 92.3 |
| 17(DC402266) | 105.7 | 58 | 94.3 |
| 18(DC402306) | 97.8 | 59 | 86.7 |
| 19(DC402308) | 98.3 | 60 | 92.6 |
| 20(DC402310) | 100.7 | 61(DC402207) | 88.3 |
| 24(DC402307) | 93.3 | 65 | 86.7 |
| 25(DC402309) | 101.9 | 66 | 68.4 |
| 26(DC402311) | 101.8 | 81(DC402259) | 96.3 |
| 35 | 94.3 | 82 | 100.7 |
| | | 83 | 97.8 |

| | | | |
|---|---|---|---|
| Note: Compound 48, or DC402234, or 2234 ("DC40" in "DC40XXXX" was omitted) referred to the same compound; other compounds were deduced by analogy. | | | |

The IC₅₀ values of some preferred compounds were listed in Table IB.

**Table 1B: Inhibition Activity(IC₅₀) of 2019 n-Cov 3CL Protease Inhibitory**

| Compound number | IC₅₀ (nM) of SARS-CoV-2 3CL protease |
|---|---|
| 5 | 33.61±3.45 |
| 6 | 48.53±3.17 |
| 9 | 68.14±12.48 |
| 10 | 67.46±13.88 |
| 11 | 178.90±29.39 |
| 12 | 87± 8 |
| 16 | 74 |
| 17 | 61.03±12.50 |
| 18 | 238.33±18.67 |
| 19 | 62.73±8.97 |
| 20 | 150.10±9.55 |
| 24 | 131.83±14.73 |
| 25 | 169.23±37.87 |
| 26 | 105.29±10.12 |
| 48 | 53 ± 0.5 |
| 81 | 40 ± 0.2 |
| 82 | 103 ±13 |
| 83 | 113 ± 9 |

The experimental results show that most compounds have good inhibitory activity against 2019-nCoV 3CL protease at 1µM, and IC₅₀ values of some compounds (Compounds 48, 81, 5, 6, 9, 10, 12, 16, 17, 19) were less than 100 nM, in which the inhibitory activity (IC₅₀) of Compound 16 (DC402267) against 2019-nCoV 3CL protease reached 73.5 nM, and the IC₅₀ of Compound 48 and 81 reached 53 nM and 40 nM.

### Test Example 2: Evaluation of the Inhibitory Activity of Compounds against the Replication of 2019 n-Cov and Determination of Half Toxic Concentration thereof

### 2.1 EC₅₀ determination

Determination of the compound's inhibitory activity against the replication of 2019-nCov: 100 µl/well compounds at gradient concentration was added to 96 wells, then 50 µl/well of virus buffer was added, then 50 µl/well of cultured RD cells (rhabdomyosarcoma cells) was immediately added. The system was cultured at 37°C for 3-4 days until the maximum cytopathic effect was observed. The medium was aspirated, 75 µl 5% MTS phenol red medium was added, and incubated at 37°C with 5% CO₂ for 1.5 hours. The fluorescence value of each well was measured at 498nM wavelength, and a graph of compound concentration vs. cell response was drawn, and the EC₅₀ of the compound against the virus was calculated using customized software purchased from Accelrys Corporation.

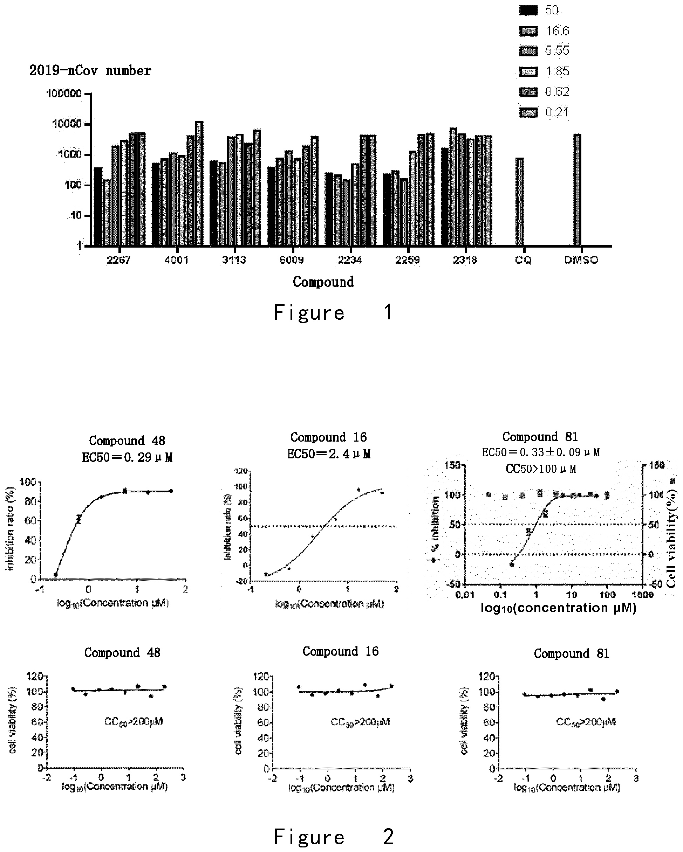

The test results are shown in Figures 1 and 2.

The results show that the aldehyde compounds of the present invention can effectively inhibit the replication of the 2019 n-Cov (Figure 1), and can inhibit different isolated virus strains to some extent.

The existing anti-2019-nCoV positive compound CQ has an inhibitory rate against 2019-nCoV virus replication at EC₅₀=1.13 µM. The results showed that: when using CQ as a positive control to test the compounds of the application at different concentration gradients, all of Compound 2234 (DC402234, namely Compound 48), 2259 (DC402259, namely Compound 81), 2267 (DC402267, namely Compound 16) have shown excellent anti-virus activity, in which the EC₅₀ of 2234 was 0.29 µM, and the EC₅₀ of 2259 was 0.33±0.09 µM, respectively. Therefore, the inhibitory rate of Compounds 48 and 81 to 2019-nCoV at viral level was better than that of the positive control CQ, thus showing good anti-2019-nCoV potentiality (Figure 2).

### 2.2 Determination of half-toxic concentration

In this example, the half-toxic concentrations (CC₅₀) of some compounds of the present invention to Vero E6 cells were determined with CCK8 kit in duplicate.

The results were shown in Figure 2. The CC₅₀ of Compounds 16, 48 and 81 are much more than 100 µM, suggesting the compounds of the present invention were of good safety.

### Activity Example 3: Crystal complex formed by Compound and SARS-CoV-2 3CL^{pro}

### 3.1 Cloning, expression, purification and crystallization of SARS-CoV-2 3CL^{pro} (M^{pro})

The full-length gene encoding SARS-CoV-2 3CL^{pro} was optimized and synthesized, and inserted into the BamHI and XhoI sites of pGEX-6p-1 plasmid DNA (Amersham Biosciences) for *Escherichia coli* (*E. coil*) Expression (GENEWIZ). SARS-CoV-2 3CL^{pro} was further purified and then co-crystallized. SARS-CoV-2 3CL^{pro} was incubated with 10mM of Compound 48 or Compound 81 for 30 mins, and the crystallized by pendant drop vapor diffusion method at 20°C (5 mg/ml). The best crystals were grown with buffer containing 2% polyethylene glycol (PEG) 6000, 3% DMSO, 1 mM DTT, 0.1 M MES (pH 6.0). The cryoprotectant solution contained 30% PEG 400, 0.1 M MES (pH 6.0).

### 3.2 Data collection and refinement statistics of crystal complexes

All data was collected on beamline BL19U1 of Shanghai Synchrotron Radiation Facility (SSRF) using Pilatus3 6M image plate detector at wavelength 0.9785 Å and 100 K. XDS25 program was used for data integration and expansion. SARS-CoV 3CL^{pro} (PDB: 2H2Z) was used as the search model and the PHASER26 program was used for molecular substitution (MR) to determine the structure. The output model of MR was manually adjusted and iterative looped by Coot27, and refined with Phenix. Compounds 48 and 81 were constructed according to the outline.

### 3.3 Results

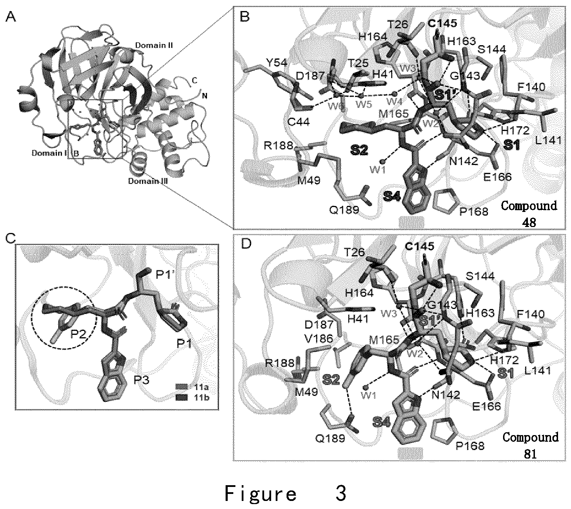

The results are shown in Table 2 and Figure 3.

**Table 2: Data collection and refinement statistics of crystal complexes**

| | | **Mpro-48** | **Mpro-81** |
|---|---|---|---|
| **PDB code** | | 6LZE | 6M0K |
| **Data collection** | | | |
| Space group | | C2 | C2 |
| Unit cell size | | | |
| | *a, b, c* (Å) | 97.70, 80.94, 51.74 | 98.15, 81.70, 51.67 |
| | *α*, *β*, *γ* (°) | 90, 114.27, 90 | 90, 114.69, 90 |
| Wavelength (Å) | | 0.97852 | 0.97852 |
| Resolution (Å) | | 50.00-1.51 (1.54-1.51)^{a} | 50.00-1.50 (1.54-1.50)^{a} |
| Completeness (%) | | 98.0 (91.6) | 98.8 (90.3) |
| *R*_{merge} (%) | | 4.2 (55.5) | 3.0 (77.1) |
| Redundancy | | 3.3 (2.7) | 3.4 (2.8) |
| *I*/σ(*I*) | | 13.99 (1.80) | 18.69 (1.30) |
| **Refinement** | | | |
| Resolution (Å) | | 47.16-1.50 | 43.45-1.50 |
| Number of reflections | | 57,378 | 58,412 |
| *R*_{work}/*R*_{free} (%) | | 17.8/20.1 | 18.34 / 19.66 |
| Number of atoms | | | |
| | Protein | 2340 | 2,347 |
| | Ligand | 49 | 50 |
| | Water | 209 | 163 |
| *B* factor (Å²) | | | |
| | Protein | 28.75 | 31.92 |
| | Ligand | 37.60 | 52.56 |
| | Water | 37.95 | 40.62 |
| R.m.s deviation | | | |
| | Bond length (Å) | 0.014 | 0.017 |
| | Bond angle (°) | 1.280 | 1.440 |
| Ramachandran graph (%) | | | |
| | Favored | 98.0 | 98.0 |
| | Allowed | 2.0 | 2.0 |
| | Outliers | 0.0 | 0.0 |

| | | | |
|---|---|---|---|
| ^{a}The value in parentheses was for the highest resolution structure. | | | |

The coordinates and structure factors of SARS-CoV-2 3CL^{pro} and Compounds 48 and 81 have been stored in the protein database, of which the PDB numbers were 6LZE and 6M0K, respectively.

The structures of crystal complexes of Compounds 48, 81 and SARS-CoV-2 3CL^{pro} were shown in Figure 3.

The experimental results showed that the aldehyde group C of Compound 48 formed a standard 1.8 Å C-S covalent bond with the catalytic site Cys145 of SARS-CoV-2 M^{pro} (Figure 3B), indicating that the Michael addition reaction has occurred. In addition, the oxygen atom of the aldehyde group formed hydrogen bond with the Cys145 residue and the Gly143 residue of the S1 pocket, which also plays vital role in stabilizing the conformation of the compound (Figure 3B). The five-membered lactam ring can insert into the S1 pocket smoothly (Figure 3B). The oxygen atom of lactam formed hydrogen bond with the His163 residue of the side chain. The main chain and side chain Glu166 of Phe140 also participateed in stabilizing the five-membered ring lactam by forming hydrogen bonds with NH. In addition, the amide bond on the main chain of Compound 48 formed hydrogen bonds with His164 and the main chain of Glu166, respectively (Figure 3B). The cyclohexyl of Compound 48 has penetrated into the S2 pocket, and was surrounded by the side chains of Met49, Tyr54, Met165 and Asp187, thus leading to extensive hydrophobic interactions (Figure 3B). The indole group of Compound 48 was exposed to the solvent (S4 pocket) and stabilized by Glu166 through hydrogen bonding (Figure 3B). The side chains of Pro168 and Gln189 residues interacted with the indole group of Compound 48 through hydrophobic interactions. Also, multiple water molecules (named W1-W6) played an important role in binding Compound 48 (Figure 3B). W1 interacted with the amide bond of Compound 48 through hydrogen bond, while W2-6 formed multiple hydrogen bonds with the aldehyde group of Compound 48 and the residues of Asn142, Gly143, Thr26, Thr25, His41, and Cys44, which help stabilizing Compound 48 in the binding pocket (Figure 3B).

The crystal structure of Compound 81 and SARS-CoV-2 M^{pro} was very similar that of Compound 48, which shows similar inhibitor binding modes (Figure 3C, 3D). The difference in binding may be caused by aryl of Compound 81. Compared with cyclohexyl of Compound 48, aryl of Compound 81 has rotated significantly (Figure 3C). The side chains of His41, Met49, Met165, and Val186 residues interacted with aryl through hydrophobic interactions (Figure 3D). The side chain of Gln189 stabilizeed aryl by forming an additional hydrogen bond with fluorine atom (Figure 3D).

In short, these two crystal structures revealed a same inhibitory mechanism, that is, the two compounds occupying the substrate binding pocket, mimicking the intermediate in the catalytic reaction, thus blocking the enzyme activity of SARS-CoV-2 M^{pro}.

All literatures mentioned in the present invention are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A use of aldehyde compound according to General Formula I, or pharmaceutically acceptable salts, enantiomers, diastereomers or racemates thereof, wherein it is used for preparing (a) 2019-nCov 3CL protease inhibitors; and (b) drugs for the treatment, prevention and/or alleviation of related diseases caused by 2019-nCov infection: 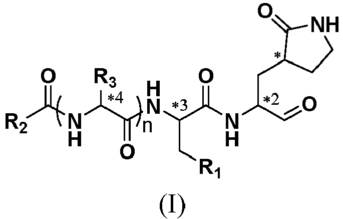 wherein,
the chiral carbon atoms C^{∗}, C^{∗2}, C^{∗3}, and C^{∗4} are each independently in S configuration, R configuration, or the combinations thereof;
n=0 or 1;
R₁ is a group unsubstituted or substituted with 1-3 substituents, and the group is selected from the group consisting of C3-C7 cycloalkyl, trifluoromethyl, C2-C6 alkynyl, 4-7 membered heterocyclyl, C5-C7 aryl, 5-7 membered heteroaryl; each heterocyclyl and heteroaryl contains 1-3 heteroatoms selected from oxygen, sulfur and nitrogen; and the substituents are each independently selected from the groups consisting of halogen, C1-C4 straight or branched alkyl, C1-C4 straight or branched alkenyl, C2-C4 straight or branched alkynyl, C1-C4 straight or branched alkoxy, C1-C4 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring;
R₂ is a group unsubstituted or substituted with 1-3 substituents, and the group is selected from the group consisting of C3-C7 cycloalkyl, 5-12 membered heterocyclyl (preferably 5-7 membered heterocyclyl or 6-membered aryl fused 5-7 membered heterocyclyl), C6-C12 aryl, 5-12 membered heteroaryl, styryl, or -Cbz; wherein each of the heterocyclyl or heteroaryl contains 1-3 heteroatoms selected from oxygen, sulfur and nitrogen; the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring;
R₃ is a group unsubstituted or substituted with 1-3 substituents, and the group is selected from the group consisting of C1-C6 straight or branched alkyl, C1-C6 straight or branched alkoxy, C3-C7 cycloalkyl, C6-C12 aryl, 5-12 membered heteroaryl, wherein the heteroaryl contains 1-3 heteroatoms selected from oxygen, sulfur and nitrogen; wherein, the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring.

2. The use of claim 1, wherein the related diseases caused by 2019 n-Cov infection are selected from the groups consisting of respiratory tract infection, pneumonia and complications thereof, or the combinations thereof.

3. The use of claim 1 or 2, wherein R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl.

4. The use of claim 1 or 2, wherein R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocyclyl and 5-12 membered heteroaryl are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline.

5. The use of claim 1 or 2, wherein the chiral carbon atoms C^{∗}, C^{∗2}, C^{∗3}, and C^{∗4} are in S configuration, and/or
R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocyclyl and 5-12 membered heteroaromatic ring are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline; and/or
R₃ is a group unsubstituted or substituted with 1-3 substituents, and the group is selected from the group consisting of C1-C6 straight or branched alkyl, C3-C7 cycloalkyl, phenyl.

6. The use of claim 1 or 2, wherein the compounds in General formula I are selected from the following groups:
| No | Name | Structure |
|---|---|---|
| 1 | 6-Chloro-N-{(S)-3-methyl-1-carbonyl-1-{{(S)-1-carbonyl-1-{{(S)-1-carbonyl-3-[(S)-2-carbonylpyrrolidine-3-yl]propan-2-yl}amino}-3-phenylpropan-2-yl}amino}butan-2-yl}-2*H*chromene-3-carboxamide | 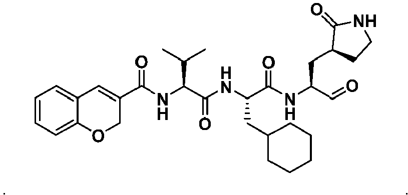 |
| 2 | 6-Chloro-N- {(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}-2*H*-chromene-3-carboxamide | 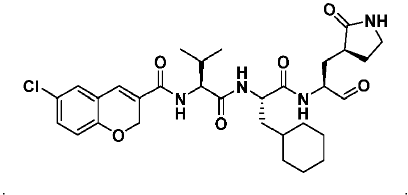 |
| 3 | 6-Chloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclopentylpropan-2-yl}amino}butan-2-yl}-2*H*chromene-3-carboxamide | 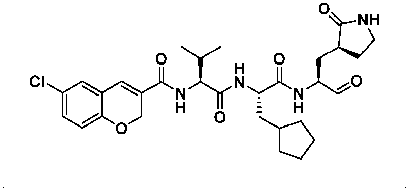 |
| 4 | 6-Chloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-4-fluorophenylpropan-2-yl}amino}butan-2-yl}-2*H*-chromene-3-carboxamide | 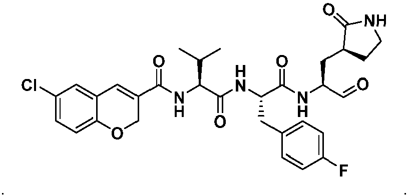 |
| 5 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}indole-2-carboxamide | 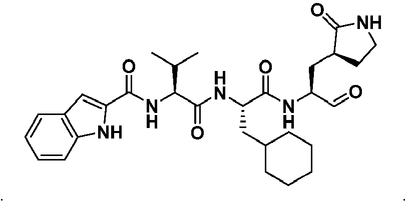 |
| 6 | 5-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}isoxazole-3-carboxamide | 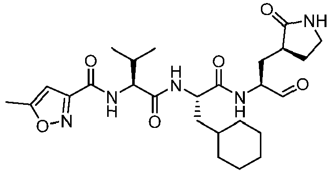 |
| 7 | N-{(S)-3-fluoro-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}indole-2-carboxamide | 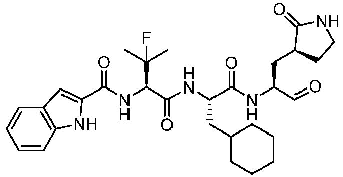 |
| 8 | 5-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}isoxazole-3-carboxamide | 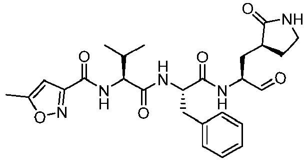 |
| 9 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}indole-2-carboxamide | 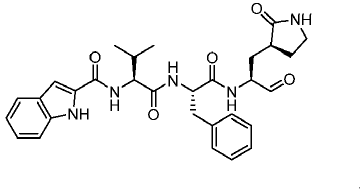 |
| 10 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}benzofuran-2-carboxamide | 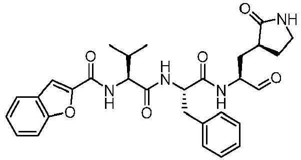 |
| 11 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}quinoline-2-carboxamide | 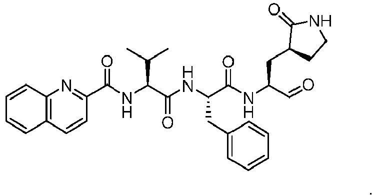 |
| 12 | N-{(S)-1-carbonyl-1-{{(S)-1-carbonyl-3-[(S)-2-carbonylpyrrolidin-3-yl]propan-2-yl}amino}-3-(3,4-difluorophenyl)propan-2-yl}-1*H*-indole-2-carboxamide | 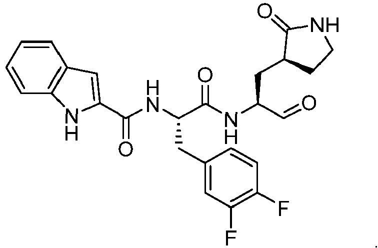 |
| 13 | 5-Methoxy-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}indole-2-carboxamide | 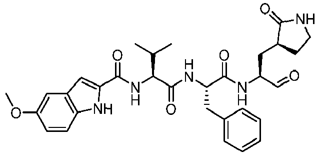 |
| 14 | 4,6-dichloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}indole-2-carboxamide | 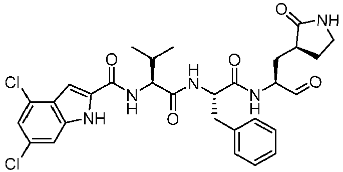 |
| 15 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-4fluorocyclohexylpropan-2-yl}amino}butan-2-yl}indole-2-carboxamide | 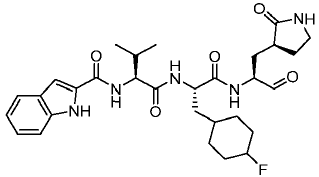 |
| 16 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}quinoline-2-carboxamide | 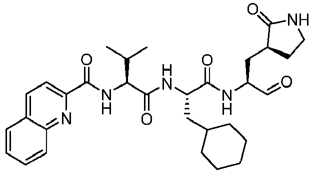 |
| 17 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}benzofuran-2-carboxamide | 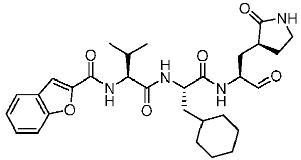 |
| 18 | 7-Bromo-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}benzofuran-2-carboxamide | 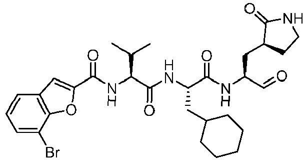 |
| 19 | 3-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}quinoline-2-carboxamide | 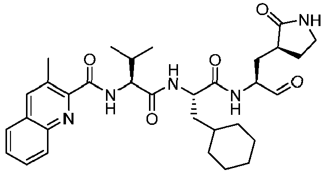 |
| 20 | 5-Chloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}benzofuran-2-carboxamide | 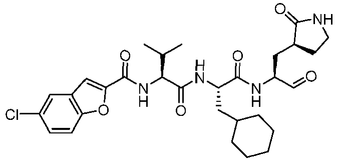 |
| 21 | N-{(S)-1-carbonyl-1-{{(S)-1-carbonyl-3-[(S)-2-carbonylpyrrolidin-3-yl]propan-2-yl}amino}-3-ethynylpropan-2-yl}-1*H-*indole-2-carboxamide | 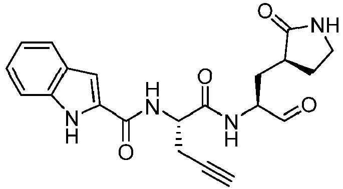 |
| 22 | N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H-*indole-2-carboxamide | 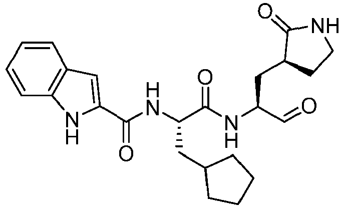 |
| 23 | 5-Fluoro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}benzofuran-2-carboxamide | 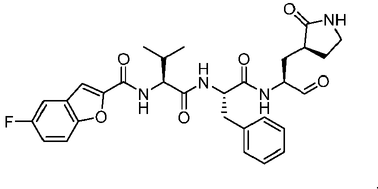 |
| 24 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}benzothiophene-2-carboxamide | 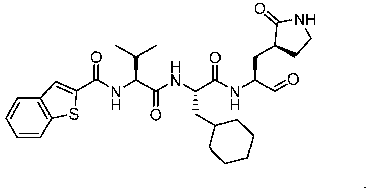 |
| 25 | 7-Bromo-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}quinoline-2-carboxamide | 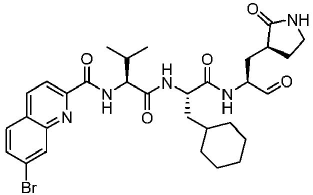 |
| 26 | 7-Methoxy-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}benzofuran-2-carboxamide | 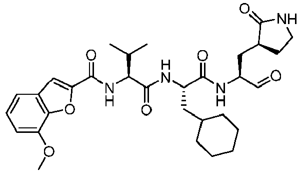 |
| 27 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}butan-2-yl}benzothiophene-2-carboxamide | 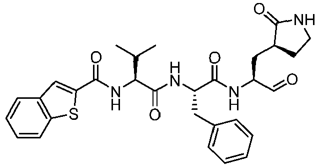 |
| 28 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-phenylpropan-2-yl}amino}pentan-2-yl}indole-2-carboxamide | 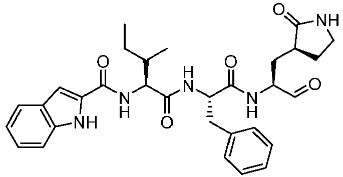 |
| 29 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}pentan-2-yl}indole-2-carboxamide | 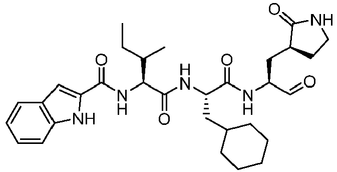 |
| 30 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-4fluorophenylpropan-2-yl}amino}butan-2-yl}indole-2-carboxamide | 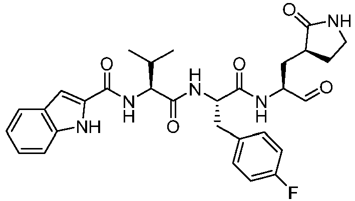 |
| 31 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclopentylpropan-2-yl}amino}butan-2-yl}indole-2-carboxamide | 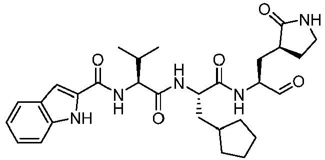 |
| 32 | 5-Methyl-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-4fluorophenylpropan-2-yl}amino}butan-2-yl}isoxazole-3-carboxamide | 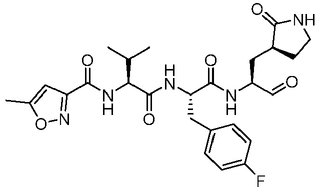 |
| 33 | N-{(S)-3,3-dimethyl-1-carbonyl-1-{{(S)-1-carbonyl-1-{{(S)-1-carbonyl-3-[(S)-2-carbonylpyrrolidin-3-yl]propan-2-yl}amino-3-phenylpropan-2-yl}amino}butan-2-yl}indole-2-carboxamide | 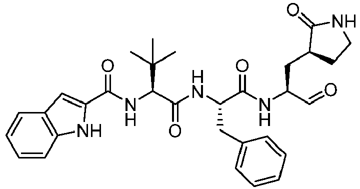 |
| 34 | N-{(S)-3,3-dimethyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}indole-2-carboxamide | 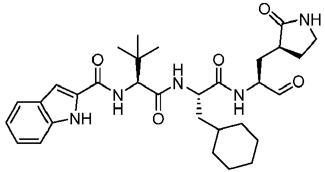 |
| 35 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-(3,4-difluorocyclohexyl)propan-2-yl}amino}butan-2-yl}indole-2-carboxamide | 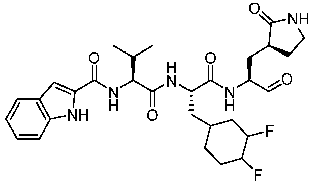 |
| 36 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclobutylpropan-2-yl}amino}butan-2-yl}indole-2-carboxamide | 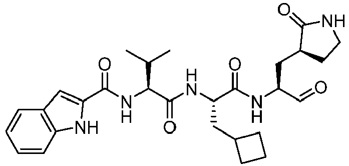 |
| 37 | N-{(S)-1-carbonyl-1-{{(S)-1-carbonyl-3-[(S)-2-carbonylpyrrolidin-3-yl]propan-2-yl}amino}-3-phenylpropan-2-yl}indole-2-carboxamide | 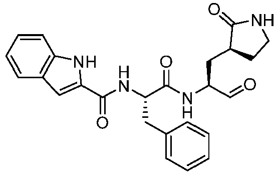 |
| 38 | 1-Methyl-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 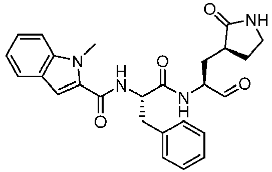 |
| 39 | 6-Trifluoromethyl-N-{(S)-1-carbonyl-1-{{(S)-1-carbonyl-3-[(S)-2-carbonylpyrrolidin-3-yl]propan-2-yl}amino}-3-phenylpropan-2-yl}-1*H-*indole- 2-carboxamide | 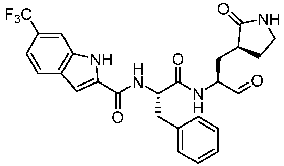 |
| 40 | 5-Methoxy-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 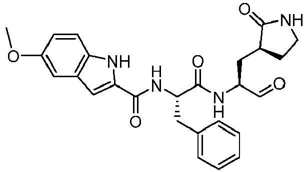 |
| 41 | 5-Chloro-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 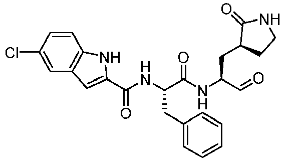 |
| 42 | 4-Fluoro-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 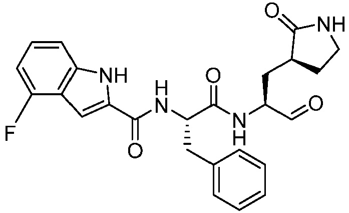 |
| 43 | 7-Fluoro-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 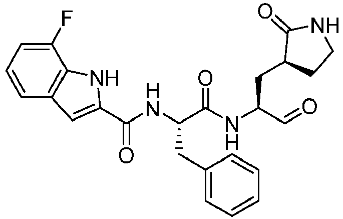 |
| 44 | 5,7-Difluoro-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 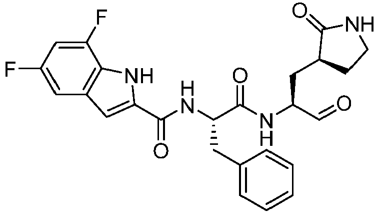 |
| 45 | 5-Fluoro-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 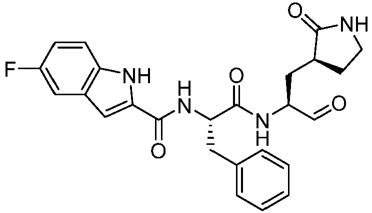 |
| 46 | 6-Fluoro-N-{(S)-1-carbonyl-1-{{(S)-1-carbonyl-3-[(S)-2-carbonylpyrrolidin-3-yl]propan-2-yl}amino}-3-phenylpropan-2-yl}-1*H*-indole-2-carboxamide | 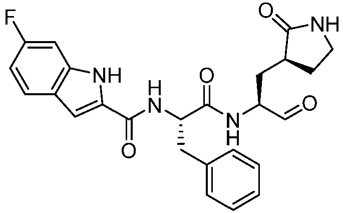 |
| 47 | 4,6-Dichloro-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 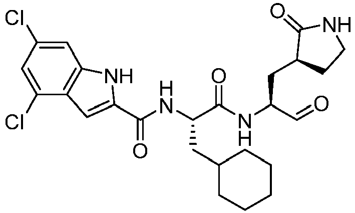 |
| 48 | N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H-*indole-2-carboxamide | 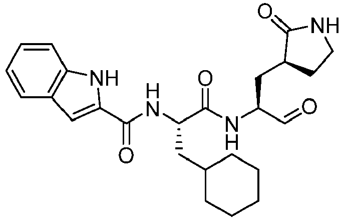 |
| 49 | 1-Methyl-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 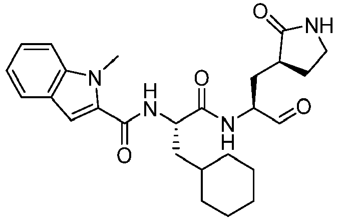 |
| 50 | 5-Methoxy-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 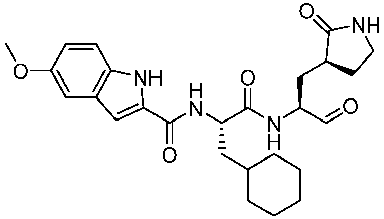 |
| 51 | 4-Fluoro-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 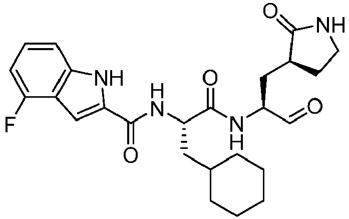 |
| 52 | 5-Fluoro-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 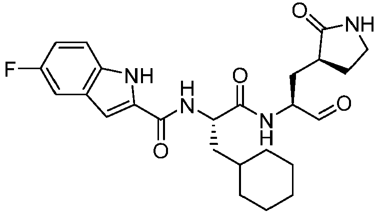 |
| 53 | 6-Fluoro-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 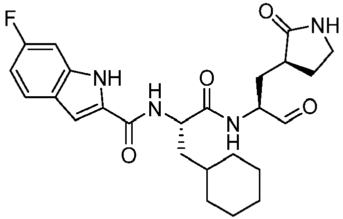 |
| 54 | 7-Fluoro-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 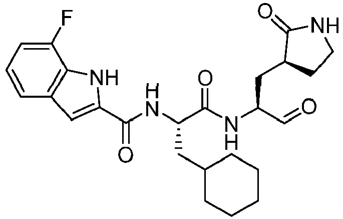 |
| 55 | N-{(S)-1-carbonyl-1-{{(S)-1-carbonyl-3-[(S)-2-carbonylpyrrolidin-3-yl]propan-2-yl}amino}-3-cyclohexylpropan-2-yl}-benzofuran-2-carboxamide | 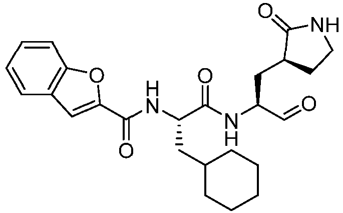 |
| 56 | N-{(S)-1-carbonyl-1-{{(S)-1-carbonyl-3-[(S)-2-carbonylpyrrolidin-3-yl]propan-2-yl}amino}-3-cyclohexylpropan-2-yl}-benzothiophene- 2-carboxamide | 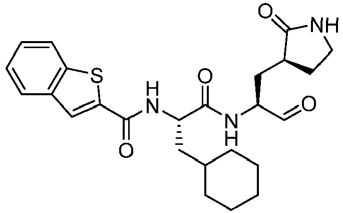 |
| 57 | N-{(S)-1-carbonyl-1-{{(S)-1-carbonyl-3-[(S)-2-carbonylpyrrolidin-3-yl]propan-2-yl}amino}-3-cyclohexylpropan-2-yl}-benzimidazole- 2-carboxamide | 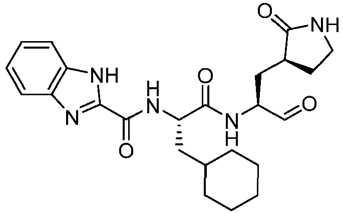 |
| 58 | N-{(S)-1-carbonyl-1-{{(S)-1-carbonyl-3-[(S)-2-carbonylpyrrolidin-3-yl]propan-2-yl}amino}-3-cyclohexylpropan-2-yl}-quinoline- 2-carboxamide | 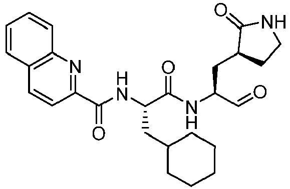 |
| 59 | N-{(S)-1-carbonyl-1-{{(S)-1-carbonyl-3-[(S)-2-carbonylpyrrolidin-3-yl]propan-2-yl}amino}-3-cyclohexylpropan-2-yl}-quinoxaline- 2-carboxamide | 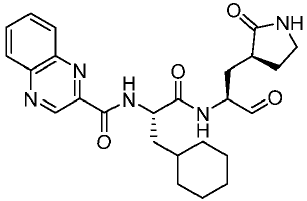 |
| 60 | N-{(S)-2-{{(S)-3-cyclohexyl-1-carbonyl-1-{{(S)-1-carbonyl-3-[(S)-2-carbonylpyrrolidin-3-yl]propan-2-yl}amino}-propan-2-yl}amino}-1-cyclopropylcarbonylethyl}-indole-2-carboxamide | 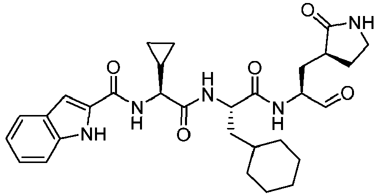 |
| 61 | N-{(S)-2-{{(S)-3-cyclohexyl-1-carbonyl-1-{{(S)-1-carbonyl-3-[(S)-2-carbonylpyrrolidin-3-yl]propan-2-yl}amino}-propan-2-yl}amino}-1-cyclopropylcarbonylethyl}-benzodioxol-5-carboxamide | 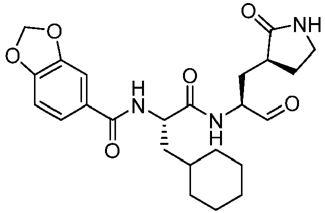 |
| 62 | 5-Methyl-N-{(S)-1-carbonyl-1-{{(S)-1-carbonyl-3-[(S)-2-carbonylpyrrolidin-3-yl]propan-2-yl}amino}-3-phenylylpropan-2-yl}-isoxazole-3-carboxamide | 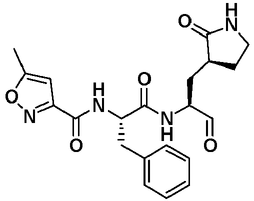 |
| 63 | 5-Fluoro-N-{(S)-1-carbonyl-1-{{(S)-1-carbonyl-3-[(S)-2-carbonylpyrrolidin-3-yl]propan-2-yl}amino}-3-phenylpropan-2-yl}nicotinamide | 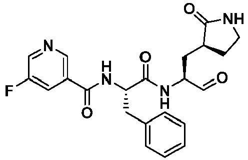 |
| 64 | N-{(S)-1-carbonyl-1-{{(S)-1-carbonyl-3-[(S)-2-carbonylpyrrolidin-3-yl]propan-2-yl}amino}-3-phenylylpropan-2-yl} - benzofuran - 5 -carboxamide | 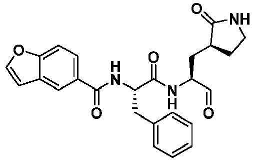 |
| 65 | N-{(S)-1-carbonyl-1-{{(S)-1-carbonyl-3-[(S)-2-carbonylpyrrolidin-3-yl]propan-2-yl}amino}-3-phenylpropan-2-yl}-2,3-dihydrobenzo[b][1,4]dioxane-6-carboxamide | 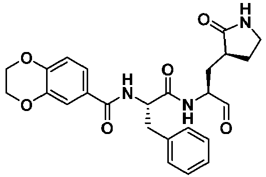 |
| 66 | N-{(S)-1-carbonyl-1-{{(S)-1-carbonyl-3-[(S)-2-carbonylpyrrolidin-3-yl]propan-2-yl}amino}-3-phenylpropan-2-yl}-2,3-dihydrobenzo[b][1,4]dioxane-5-carboxamide | 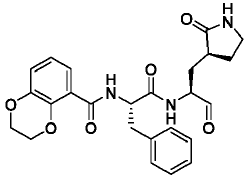 |
| 67 | N-{(S)-1-carbonyl-1-{{(S)-1-carbonyl-3-[(S)-2-carbonylpyrrolidin-3-yl]propan-2-yl}amino}-3-phenylpropan-2-yl}-1H-indole-5-carboxamide | 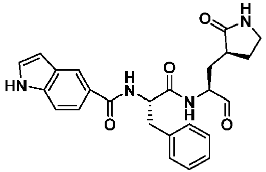 |
| 68 | 6-Bromo-N-{(S)-1-carbonyl-1-{{(S)-1-carbonyl-3-[(S)-2-carbonylpyrrolidin-3-yl]propan-2-yl}amino}-3-phenylpropan-2-yl}imidazo[1,2-a]pyridine-2-carboxamide | 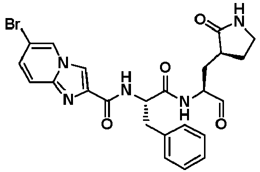 |
| 69 | N-{(S)-1-carbonyl-1-{{(S)-1-carbonyl-3-[(S)-2-carbonylpyrrolidin-3-yl]propan-2-yl}amino}-3-phenylpropan-2-yl}benzo[b]thiophene-2-carboxamide | 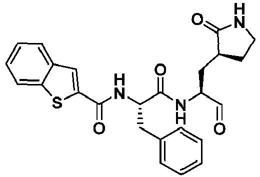 |
| 70 | N-{(S)-1-carbonyl-1-{{(S)-1-carbonyl-3-[(S)-2-carbonylpyrrolidin-3-yl]propan-2-yl}amino}-3-phenylpropan-2-yl}benzofuran-2-carboxamide | 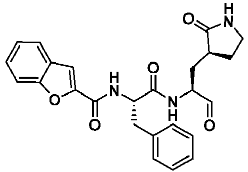 |
| 71 | N-{(S)-1-carbonyl-1-{{(S)-1-carbonyl-3-[(S)-2-carbonylpyrrolidin-3-yl]propan-2-yl}amino}-3-phenylpropan-2-yl}quinoxaline-2-carboxamide | 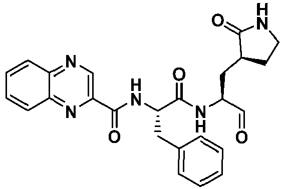 |
| 72 | 3-Methyl-N-{(S)-1-carbonyl-1-{{(S)-1-carbonyl-3-[(S)-2-carbonylpyrrolidin-3-yl]propan-2-yl}amino}-3-phenylpropan-2-yl}quinoxaline-2-carboxamide | 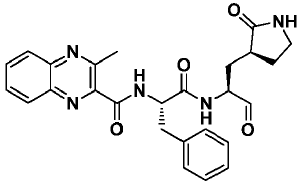 |
| 73 | N-{(S)-1-carbonyl-1-{{(S)-1-carbonyl-3-[(S)-2-carbonylpyrrolidin-3-yl]propan-2-yl}amino}-3-phenylpropan-2-yl} quinoline-2-carboxamide | 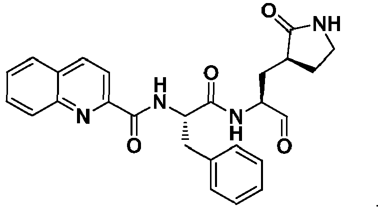 |
| 74 | N-{(S)-1-carbonyl-1-{{(S)-1-carbonyl-3-[(S)-2-carbonylpyrrolidin-3-yl]propan-2-yl}amino}-3-phenylpropan-2-yl}-4-trifluoromethylquinoline-2-carboxamide | 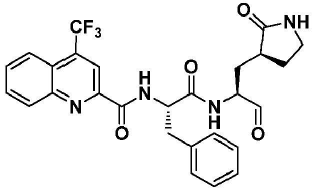 |
| 75 | 7-Bromo-N-{(S)-1-carbonyl-1-{{(S)-1-carbonyl-3-[(S)-2-carbonylpyrrolidin-3-yl]propan-2-yl}amino}-3-phenylpropan-2-yl} quinoline-2-carboxamide | 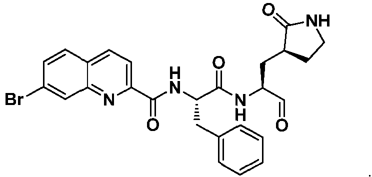 |
| 76 | 6-Chloro-N-{(S)-1-carbonyl-1-{{(S)-1-carbonyl-3-[(S)-2-carbonylpyrrolidin-3-yl]propan-2-yl}amino}-3-phenylpropan-2-yl}-2*H*-chromene-3-carboxamide | 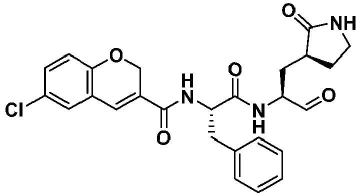 |
| 77 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}-2,3-dihydrobenzo[b] [1,4]dioxane-6-carboxamide | 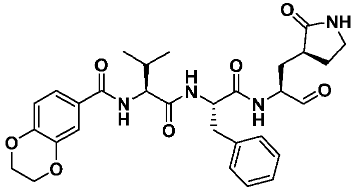 |
| 78 | 5-Methoxy-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}indole-2-carboxamide | 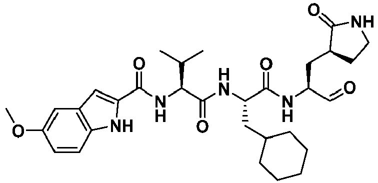 |
| 79 | 4,6-Dichloro-N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}indole-2-carboxamide | 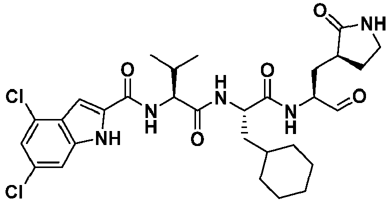 |
| 80 | N-{(S)-3-methyl-1-carbonyl-1-{{(S)-1-carbonyl-1-{{(S)-1-carbonyl-3-[(S)-2-carbonylpyrrolidin-3-yl]propan-2-yl}amino}-3-cyclohexylpropan-2-yl}amino}butan-2-yl}cinnamamide | 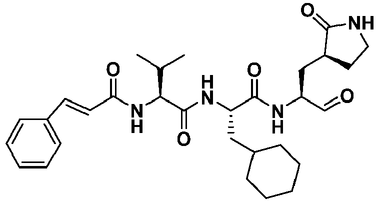 |
| 81 | N-{(S)-1-carbonyl-1-{{(S)-1-carbonyl-3-[(S)-2-carbonylpyrrolidin-3-yl]propan-2-yl}amino}-3-3fluorophenylpropan-2-yl}-1*H*-indole-2-carboxamide | 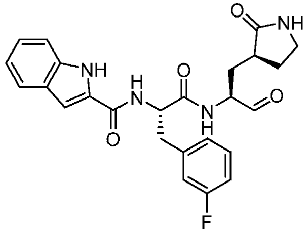 |
| 82 | N-{(S)-1-carbonyl-1-{{(S)-1-carbonyl-3-[(S)-2-carbonylpyrrolidin-3-yl]propan-2-yl}amino}-3-4fluorophenylpropan-2-yl}-1*H*-indole-2-carboxamide | 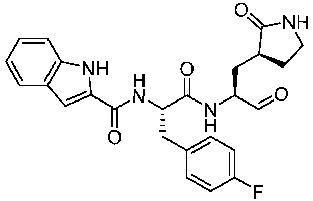 |
| 83 | 4,7-Difluoron-{(S)-1-carbonyl-1-{{(S)-1-carbonyl-3-[(S)-2-carbonylpyrrolidin-3-yl]propan-2-yl}amino}-3-3fluorophenylpropan-2-yl}-1*H*-indole-2-carboxamide | 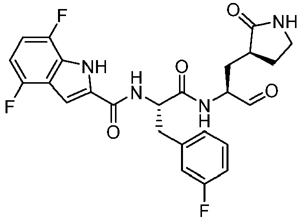 |
| 84 | 4-Methyl-N-{(S)-1-carbonyl-1-{{(S)-1-carbonyl-3-[(S)-2-carbonylpyrrolidin-3-yl]propan-2-yl}amino}-3-cyclohexylpropan-2-yl}-1*H*-indole-2-carboxamide | 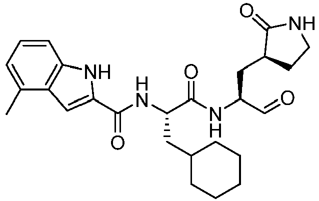 |
| 85 | N-{(S)-1-carbonyl-1-{{(S)-1-carbonyl-3-[(S)-2-carbonylpyrrolidin-3-yl]propan-2-yl}amino}-3-2fluorophenylpropan-2-yl}-1H-indole-2-carboxamide | 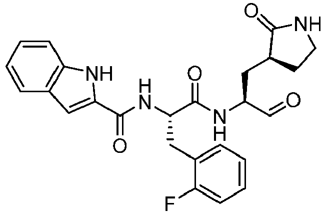 |
| 86 | N-{(S)-1-carbonyl-1-{{(S)-1-carbonyl-3-[(S)-2-carbonylpyrrolidin-3-yl]propan-2-yl}amino } -3-3fluorophenylpropan-2-yl} - benzimidazole- 2-carboxamide | 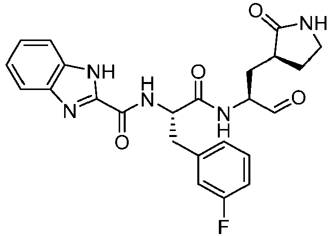 |
| 87 | N-{(S)-1-carbonyl-1-{{(S)-1-carbonyl-3-[(S)-2-carbonylpyrrolidin-3-yl]propan-2-yl}amino}-3-3fluorophenylpropan-2-yl}- benzofuran-2-carboxamide | 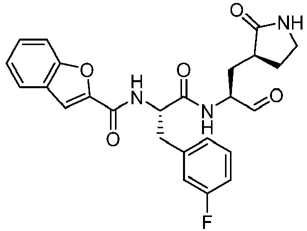 |
| 88 | N-{(S)-1-carbonyl-1-{{(S)-1-carbonyl-3-[(S)-2-carbonylpyrrolidin-3-yl]propan-2-yl}amino}-3-3fluorophenylpropan-2-yl}- quinoline-2-carboxamide | 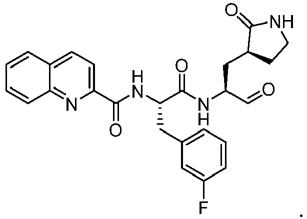 |

7. A pharmaceutical composition, wherein the pharmaceutical composition comprises (a) the aldehyde compounds according to General Formula I, or pharmaceutically acceptable salts, enantiomers, diastereomers or racemates thereof; and (b) a pharmaceutically acceptable carrier, 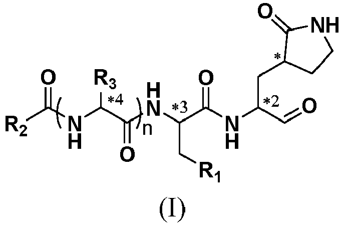 wherein,
the chiral carbon atoms C^{∗}, C^{∗2}, C^{∗3}, and C^{∗4} are each independently in S configuration, R configuration, or the combination thereof;
n=0 or 1;
R₁ is a group unsubstituted or substituted with 1-3 substituents, and the group is selected from the group consisting of C3-C7 cycloalkyl, trifluoromethyl, C2-C6 alkynyl, 4-7 membered heterocyclyl, C5-C7 aryl, 5-7 membered heteroaryl; each of the heterocyclyl and heteroaryl contain 1-3 heteroatoms selected from oxygen, sulfur and nitrogen; the substituents are each independently selected from the group consisting of halogen, C1-C4 straight or branched alkyl, C1-C4 straight or branched alkenyl, C2-C4 straight or branched alkynyl, C1-C4 straight or branched alkoxy, C1-C4 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring;
R₂ is a group unsubstituted or substituted with 1-3 substituents, and the group is selected from the group consisting of C3-C7 cycloalkyl, 5-12 membered heterocyclyl (preferably 5-7 membered heterocyclyl or 6-membered aryl fused 5-7 membered heterocyclyl), C6-C12 aryl, 5-12 membered heteroaryl, styryl, or -Cbz; wherein each of the heterocyclyl or heteroaryl contains 1-3 heteroatoms selected from oxygen, sulfur and nitrogen; the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring;
R₃ is a group unsubstituted or substituted with 1-3 substituents, and the group is selected from the group consisting of C1-C6 straight or branched alkyl, C1-C6 straight or branched alkoxy, C3-C7 cycloalkyl, C6-C12 aryl, 5-12 membered heteroaryl, wherein the heteroaryl contains 1-3 heteroatoms selected from oxygen, sulfur and nitrogen; wherein, the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring.

8. The use of the pharmaceutical composition of claim 7, wherein it is used for preparing drugs for the treatment and/or prevention, alleviation of related diseases caused by 2019 2019-nCov infection.

9. The use of claim 8, wherein the related diseases caused by 2019-nCov infection are selected from the groups consisting of respiratory tract infection, pneumonia and complications thereof, or the combinations thereof.

10. An aldehyde compound according to General Formula I, or pharmaceutically acceptable salts, enantiomers, diastereomers or racemates thereof; 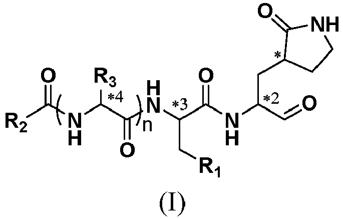 wherein,
the chiral carbon atoms C^{∗}, C^{∗2}, C^{∗3}, and C^{∗4} are each independently in S configuration, in R configuration, or the combinations thereof;
n=0 or 1;
R₁ is a group unsubstituted or substituted with 1-3 substituents, and the group is selected from the group consisting of C3-C7 cycloalkyl, trifluoromethyl, C2-C6 alkynyl, 4-7 membered heterocyclyl, C5-C7 aryl, 5-7 membered heteroaryl; each of the heterocyclyl and heteroaryl contains 1-3 heteroatoms selected from oxygen, sulfur and nitrogen; the substituents are each independently selected from the groups consisting of halogen, C1-C4 straight or branched alkyl, C1-C4 straight or branched alkenyl, C2-C4 straight or branched alkynyl, C1-C4 straight or branched alkoxy, C1-C4 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring;
R₂ is a group unsubstituted or substituted with 1-3 substituents, and the group is selected from the group consisting of C3-C7 cycloalkyl, 5-12 membered heterocyclyl (preferably 5-7 membered heterocyclyl or 6-membered aryl fused 5-7 membered heterocyclyl), C6-C12 aryl, 5-12 membered heteroaryl, styryl, or -Cbz; wherein each of the heterocyclyl or heteroaryl contains 1-3 heteroatoms selected from oxygen, sulfur and nitrogen; the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring;
R₃ is a group unsubstituted or substituted with 1-3 substituents, and the group is selected from the group consisting of C1-C6 straight or branched alkyl, C1-C6 straight or branched alkoxy, C3-C7 cycloalkyl, C6-C12 aryl, 5-12 membered heteroaryl, wherein the heteroaryl contains 1-3 heteroatoms selected from oxygen, sulfur and nitrogen; wherein, the substituents are each independently selected from halogen, C1-C6 straight or branched alkyl, C2-C6 straight or branched alkenyl, C2-C6 straight or branched alkynyl, C1-C6 straight or branched alkoxy, C1-C6 straight or branched alkylcarbonyloxy, cyano, nitro, hydroxyl, amino, hydroxymethyl, trifluoromethyl, carboxyl, thiol, C1-C4 acyl, amide, sulfonyl, aminosulfonyl, C1-C4 alkyl-substituted sulfonyl, or two adjacent substituents together with the carbon atoms to which they are connected form 5-7 membered ring.

11. The compound of claim 10, wherein R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl.

12. The compound of claim 10, wherein R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocycle and 5-12 membered heteroaromatic ring are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline.

13. The compound of claim 10, wherein the chiral carbon atoms C^{∗}, C^{∗2}, C^{∗3}, and C^{∗4} are in S configuration, and/or
R₁ is a group unsubstituted or substituted with 1-3 substituents, and the group is selected from the group consisting of trifluoromethyl, alkynyl, cyclopropanyl, cyclobutanyl, cyclopentyl, cyclohexyl, phenyl, thienyl, pyrazolyl, thiazolyl, pyridyl, furyl; and/or
R₂ is a group unsubstituted or substituted with 1-3 substituents, and the group is selected from the group consisting of phenyl, styryl, benzoheterocyclyl, 5-12 membered heteroaryl; preferably, the benzoheterocycle and 5-12 membered heteroaromatic ring are selected from benzodioxole, indole, isoxazole, 2-hydroproppyran, pyridine, pyrazole, dihydroimidazopyridine, imidazopyridine, benzothiophene, dihydrobenzodioxane, quinoxaline, benzofuran, indazole, benzimidazole, quinoline; and/or
R₃ is a group unsubstituted or substituted with 1-3 substituents, and the group is selected from the group consisting of C1-C6 straight or branched alkyl, C3-C7 cycloalkyl, phenyl.

14. The compound of claim 10, wherein the compounds in General Formula I are Compound 1-88 in Table 1.

15. A method for treating, preventing, and/or alleviating the related diseases caused by 2019-nCov infection, comprising the steps: administering a safe and effective amount of the aldehyde compounds according to Formula I, or pharmaceutically acceptable salts, enantiomers, diastereomers or racemates thereof to the subject in need thereof, wherein the aldehyde compounds according to Formula I are as described in claim 10.
